(19) Europäisches Patentamt
European Patent Office
Office européen des brevets

(11) EP 4 317 129 A1

(12) EUROPEAN PATENT APPLICATION
published in accordance with Art. 153(4) EPC

(43) Date of publication:
**07.02.2024 Bulletin 2024/06**

(21) Application number: **22775855.4**

(22) Date of filing: **25.03.2022**

(51) International Patent Classification (IPC):
***C07C 303/22*** (2006.01) ***C07C 309/80*** (2006.01)
***C07C 309/82*** (2006.01) ***C07C 313/02*** (2006.01)
***C07D 285/00*** (2006.01) ***C07D 285/01*** (2006.01)
***H01M 10/052*** (2010.01) ***H01M 10/0567*** (2010.01)
***H01M 10/0568*** (2010.01) ***H01M 10/0569*** (2010.01)

(52) Cooperative Patent Classification (CPC):
**C07C 303/22; C07C 309/80; C07C 309/82; C07C 313/02; C07D 285/00; C07D 285/01; H01M 10/052; H01M 10/0567; H01M 10/0568; H01M 10/0569**

(86) International application number:
**PCT/JP2022/014660**

(87) International publication number:
**WO 2022/203074 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2021 JP 2021053666**
**30.06.2021 JP 2021108912**
**30.06.2021 JP 2021108939**
**30.06.2021 JP 2021108940**
**30.08.2021 JP 2021140158**
**30.09.2021 JP 2021161369**
**30.09.2021 JP 2021161468**
**10.12.2021 JP 2021201100**

(71) Applicant: **Asahi Kasei Kabushiki Kaisha**
**Tokyo 1000006 (JP)**

(72) Inventors:
- **ITO, Makoto**
  **Tokyo 100-0006 (JP)**
- **MATSUOKA, Naoki**
  **Tokyo 100-0006 (JP)**
- **HORI, Kaishi**
  **Tokyo 100-0006 (JP)**
- **MAEDA, Kazuki**
  **Tokyo 100-0006 (JP)**
- **UEMATSU, Nobuyuki**
  **Tokyo 100-0006 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) FLUORINE-CONTAINING CYCLIC SULFONYLIMIDE SALT, AND METHOD FOR PRODUCING SAME, NON-AQUEOUS ELECTROLYTE, NON-AQUEOUS SECONDARY BATTERY

(57) Provided is a non-aqueous electrolyte containing a non-aqueous electro solvent and a lithium salt, wherein the non-aqueous solvent includes a carbonate solvent, the lithium salt contains a fluorine-containing cyclic sulfonylimide salt represented by general formula (1): (in the formula, $R^f$ each may be the same or different and each represent a fluorine atom or a C4 or lower perfluoro group), and a fluorine-containing sulfonamide compound represented by general formula (2): $H(CR_2)_m$-$SO_2NHM^2$ (in the formula, R each may be the same or different and each are a fluorine atom or a trifluoromethyl group, $M^2$ is Li, Na, K, H, or $NH_4$, and m is 1 or 2) is contained in an amount of 1,000 mass ppm or less relative to the total amount of non-aqueous electrolyte.

EP 4 317 129 A1

FIG. 1

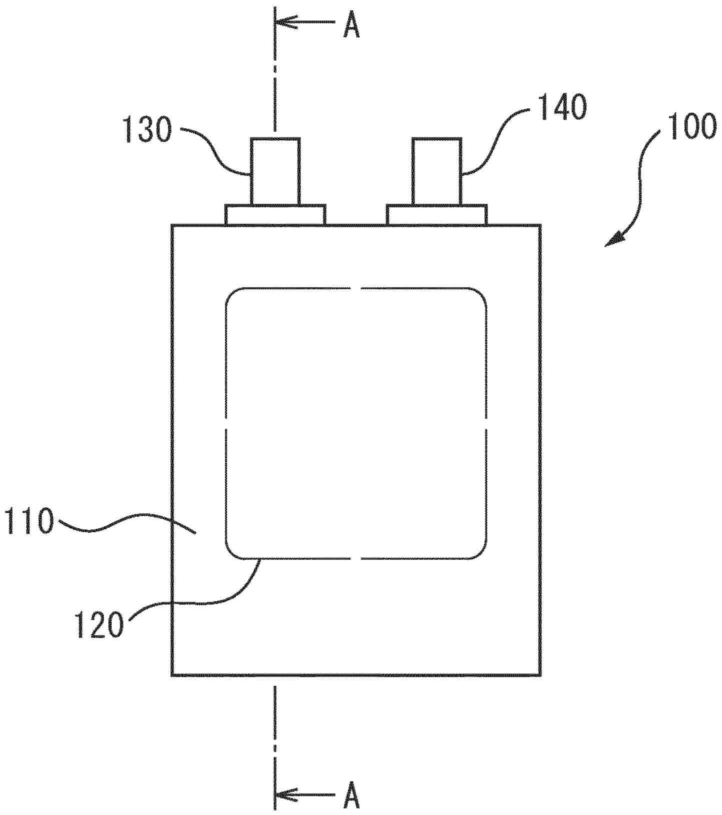

## Description

TECHNICAL FIELD

The present invention relates to a fluorine-containing cyclic sulfonylimide salt which is useful as antistatic agents, ion conductive materials such as organic electrolytes, and flame retardants, a method for producing the same, a non-aqueous electrolyte solution, and a non-aqueous secondary battery.

BACKGROUND ART

Non-aqueous secondary batteries including lithium ion batteries are characterized by their light weight, high energy and long life, and have widely been used as power sources for various portable electronic devices. In recent years, applications of non-aqueous electrolyte solutions have been widened to an industrial field typified by power tools such as electric tools, and in-vehicle use in electric cars and electric bicycles, and attention has also been focused on the field of a power storage field such as a residential power storage system.
As the non-aqueous electrolyte solution for lithium ion batteries, a combination of a high dielectric solvent such as a cyclic carbonate and a low viscosity solvent such as a chain carbonate is generally exemplified. In order to form a solid electrolyte interface (SEI) on a surface of a negative electrode to thereby suppress the reductive decomposition of the non-aqueous solvent, it is preferable to add an electrode protection additive such as vinylene carbonate.
In recent years, fluorine-containing cyclic sulfonylimide salts have attracted attention as materials for non-aqueous electrolyte solutions.
For example, PTL 1 discloses a non-aqueous electrolyte solution, which is free from corrosion of electrodes and maintains high ionic conductivity, by using a fluorine-containing cyclic sulfonylimide salt.
PTL 2 discloses a non-aqueous secondary battery in which a non-aqueous electrolyte solution containing a fluorine-containing cyclic sulfonylimide salt is used in combination with a positive electrode containing a reduced amount of cobalt.
Fluorine-containing sulfonylimide salts such as N,N-bis(trifluoromethanesulfonyl)imide and bis(fluorosulfonyl)imide, and derivatives thereof are known to be compounds which are useful as ion conductive materials and flame retardants (see, for example, NPL 1 below).
By the way, there is known, as a method for producing a compound (1A), a method in which $FO_2SCF_2CF_2SO_2F$ synthesized by an electrolytic coupling reaction of $FSO_2CF_2COOH$ is reacted with ammonia to obtain a fluorine-containing cyclic sulfonylimide ammonium salt (of a cyclization reaction), followed by reacting with a hydroxide or a carbonate of alkali metal (see PTL 3 below).

[CITATION LIST]

[PATENT LITERATURE]

[PTL 1] WO 2010/110388 A
[PTL 2] JP 2008-21517 A
[PTL 3] WO 2006/106960 A
[PTL 4] WO 2009/025246 A


[NON-PATENT LITERATURE]

[NPL 1] Research report of Asahi Glass Co., Ltd., Vol. 60(2010), pp. 13-21

SUMMARY

[TECHNICAL PROBLEM]

PTL 3 describes that a reaction crude product containing $FO_2SCF_2CF_2SO_2F$ synthesized by an electrolytic coupling reaction may be purified by a post-treatment such as distillation, but neither describes nor suggests a specific post-treatment to reduce the content of a specific by-product contained in the compound (1A) as a final product to thereby enhance the heat-resistant properties (low mass reduction rate at high temperature).
When a fluorine-containing cyclic sulfonylimide salt (hereinafter also referred to as compound (1A) herein)

represented by the general formula (1A):

[Chemical Formula 1]

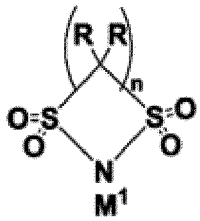

(1A)

wherein Rs may be the same or different and R each is a fluorine atom or a trifluoromethyl group, n is an integer of 1 to 4, and $M^1$ is Li, Na, K or $NH_4$ , is produced according to PTL 3, a fluorine-containing sulfonamide compound (hereinafter also referred to as compound (2)) represented by the following general formula (2):

$$H(CR_2)_m\text{-}SO_2NHM^2 \qquad (2)$$

wherein Rs may be the same or different and R each is a fluorine atom or a trifluoromethyl group, m is 1 or 2, and $M^2$ is Li, Na, K, H or $NH_4$ , is mixed in the final product as by-products (impurities), thus failing to obtain desired heat-resistant properties of the compound (1A).

PTL 4 describes, as a limited existing technique for crystallization of a fluorine-containing cyclic sulfonylimide salt, for example, Example of purification by crystallization of a lithium salt in a mixed solvent of 1,4-dioxane as a poor solvent and acetonitrile as a good solvent.

The techniques described in PTLs 1 to 3 had problems with sufficient suppression of metal corrosion of non-aqueous secondary battery members, or initial charging/discharging efficiency and cycle performance.

In view of the above circumstances, the problems to be solved by the present invention is to provide a fluorine-containing cyclic sulfonylimide salt and a non-aqueous electrolyte solution with excellent heat-resistant properties and reduced corrosiveness to metal.

[SOLUTION TO PROBLEM]

As a result of intensive studies and repeated experiments to solve the above problems, the present inventors have found unexpectedly that an alkali metal salt of a fluorine-containing cyclic sulfonimide with reduced specific impurities has excellent heat-resistant properties and reduces corrosiveness to metal, and thus the present invention has been completed.

Specifically, the present invention is as follows.

<1> A non-aqueous electrolyte solution comprising a non-aqueous solvent and a lithium salt, wherein

the non-aqueous solvent contains a carbonate solvent,
the lithium salt contains a fluorine-containing cyclic sulfonylimide salt represented by the following general formula (1):

[Chemical Formula 2]

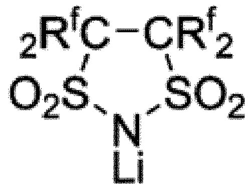

(1)

wherein $R^f$ s may be the same or different and $R^f$ each represents a fluorine atom or a perfluoro group having 4 or less carbon atoms, and contains a fluorine-containing sulfonamide compound represented by the following

general formula (2):

$$H(CR_2)_m\text{-}SO_2NHM^2 \qquad (2)$$

wherein Rs may be the same or different and R each is a fluorine atom or a trifluoromethyl group, $M^2$ is Li, Na, K, H or $NH_4$ , and m is 1 or 2, in an amount of 1,000 ppm by mass or less relative to the total amount of the non-aqueous electrolyte solution.

<2> The non-aqueous electrolyte solution according to item 1, wherein the fluorine-containing cyclic sulfonylimide salt represented by the general formula (1) contains any one of the following formulas (1-1) to (1-5).

[Chemical Formula 3]

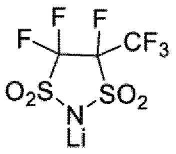 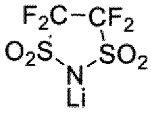 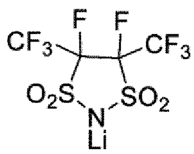 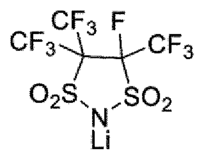 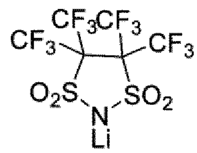

(1-1) (1-2) (1-3) (1-4) (1-5)

<3> The non-aqueous electrolyte solution according to item 1 or 2, wherein the fluorine-containing sulfonamide compound represented by the general formula (2) is contained in an amount of 0.1 ppm by mass or more relative to the total amount of the non-aqueous electrolyte solution.
<4> The non-aqueous electrolyte solution according to any one of items 1 to 3, wherein the non-aqueous solvent contains acetonitrile in an amount of 3% by volume or more and 97% by volume or less relative to the total amount of the non-aqueous solvent.
<5> The non-aqueous electrolyte solution according to item 4, wherein the content of the acetonitrile is 20% by volume or more and 95% by volume or less relative to the total amount of the non-aqueous solvent.
<6> The non-aqueous electrolyte solution according to any one of items 1 to 5, wherein the fluorine-containing sulfonamide compound represented by the general formula (2) is contained in an amount of 1 ppm by mass or more and 200 ppm by mass or less relative to the total amount of the non-aqueous electrolyte solution.
<7> The non-aqueous electrolyte solution according to any one of items 1 to 6, wherein the content of the fluorine-containing cyclic sulfonylimide salt represented by the general formula (1) is 0.8 mol or more and 1.5 mol or less relative to 1 L of the non-aqueous solvent.
<8> The non-aqueous electrolyte solution according to any one of items 1 to 7, wherein the fluorine-containing sulfonamide compound represented by the general formula (2) is contained in an amount of 80 ppm by mass or more and 120 ppm by mass or less relative to the total amount of the non-aqueous electrolyte solution.
<9> The non-aqueous electrolyte solution according to any one of items 1 to 8, wherein the lithium salt contains the fluorine-containing cyclic sulfonylimide salt represented by the general formula (1) and $LiPF_6$ ,

the non-aqueous solvent contains vinylene carbonate and/or fluoroethylene carbonate,
the content of the fluorine-containing cyclic sulfonylimide salt represented by the general formula (1) is 2.5 or more, in terms of a molar ratio, relative to the content of $LiPF_6$ , and
the content of $LiPF_6$ is 0.01 or more and 4 or less relative to the contents of vinylene carbonate and fluoroethylene carbonate, in terms of a molar ratio.

<10> The non-aqueous electrolyte solution according to any one of items 1 to 9, wherein the content of the fluorine-containing cyclic sulfonylimide salt represented by the general formula (1) is more than 10, in terms of a molar ratio, relative to the content of $LiPF_6$ .
<11> A non-aqueous secondary battery comprising the non-aqueous electrolyte solution according to any one of items 1 to 10.
<12> The non-aqueous secondary battery according to item 11, wherein the non-aqueous secondary battery comprises a battery exterior, and the battery exterior contains aluminum in at least a part of a liquid contact layer with the non-aqueous electrolyte solution on the positive electrode side.
<13> A method for producing the non-aqueous electrolyte solution according to any one of items 1 to 10, wherein the fluorine-containing cyclic sulfonylimide salt represented by the general formula (1) is produced by subjecting a

fluorosulfonyl group-containing carboxylic acid compound represented by the following general formula (3):

$$MO_2C\text{-}(CR_2)_m\text{-}SO_2F \qquad (3)$$

wherein Rs may be the same or different and R each is a fluorine atom or a trifluoromethyl group, m is 1 or 2, and M is H, Li, Na, K or $NH_4$, as a starting material, to an electrolytic coupling reaction step, a cyclization step and a cation exchange step, the method comprising:

a mixing step of mixing the fluorine-containing cyclic sulfonylimide salt represented by the general formula (1) with a non-aqueous solvent, and
a purification step of reducing a content of a fluorosulfonyl group-containing compound represented by the following general formula (5):

$$H\text{-}(CR_2)m\text{-}SO_2F \qquad (5)$$

wherein Rs may be the same or different and R each is a fluorine atom or a trifluoromethyl group, and m is 1 or 2, in the reaction solution, to 0.1% by mass or less after the electrolytic coupling reaction step.
<14> A method for producing the non-aqueous electrolyte solution according to any one of items 1 to 10, wherein the fluorine-containing cyclic sulfonylimide salt represented by the general formula (1) is produced by subjecting a fluorosulfonyl group-containing carboxylic acid compound represented by the following general formula (3):

$$MO_2C\text{-}(CR_2)_m\text{-}SO_2F \qquad (3)$$

wherein Rs may be the same or different and R each is a fluorine atom or a trifluoromethyl group, m is 1 or 2, and M is H, Li, Na, K or $NH_4$, as a starting material, to an electrolytic coupling reaction step, a cyclization step and a cation exchange step, the method comprising:

a crystallization step of dissolving the fluorine-containing cyclic sulfonylimide salt represented by the general formula (1) obtained after the cation exchange step in a monodentate chain ether solvent, followed by crystallization to obtain a purified fluorine-containing cyclic sulfonylimide salt, and
a mixing step of mixing the fluorine-containing cyclic sulfonylimide salt represented by the general formula (1) obtained in the crystallization step with a non-aqueous solvent.

<15> A fluorine-containing cyclic sulfonylimide salt composition comprising a fluorine-containing cyclic sulfonylimide salt represented by the following general formula (1A):

[Chemical Formula 4]

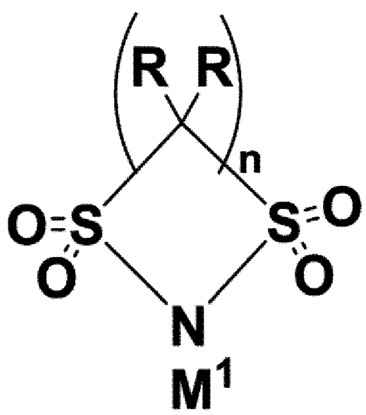

(1A)

wherein Rs may be the same or different and R each is a fluorine atom or a trifluoromethyl group, n is an integer of 1 to 4, and $M^1$ is Li, Na, K or $NH_4$ , wherein
the temperature at which a mass reduction rate reaches 2% by mass when heated at a temperature rise rate of 10°C/min from 100°C under nitrogen stream is 385°C or higher.
<16> The fluorine-containing cyclic sulfonylimide salt composition according to item 15, wherein a fluorine-containing

sulfonamide compound represented by the following general formula (2):

$$H(CR_2)_m\text{-}SO_2NHM^2 \qquad (2)$$

wherein Rs may be the same or different and R each is a fluorine atom or a trifluoromethyl group, $M^2$ is Li, Na, K, H or $NH_4$ , and m is 1 or 2, is contained in an amount of 5,000 ppm by mass or less.

<17> The fluorine-containing cyclic sulfonylimide salt composition according to item 16, wherein n is 2 in the general formula (1A).

<18> The fluorine-containing cyclic sulfonylimide salt composition according to item 16 or 17, wherein R is a fluorine atom in the general formulas (1A) and (2).

<19> The fluorine-containing cyclic sulfonylimide salt composition according to any one of items 16 to 18, wherein $M^1$ is Li in the general formula (1A), and $M^2$ is Li in the general formula (2).

<20> The fluorine-containing cyclic sulfonylimide salt composition according to any one of items 15 to 19, wherein the fluorine-containing cyclic sulfonylimide salt is represented by the following formula.

[Chemical Formula 5]

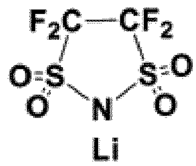

<21> A method for producing a fluorine-containing cyclic sulfonylimide salt represented by the following general formula (1A):

[Chemical Formula 6]

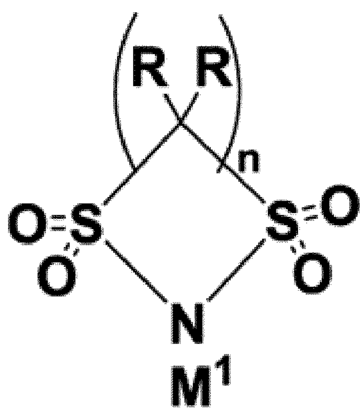

(1A)

wherein Rs may be the same or different and R each is a fluorine atom or a trifluoromethyl group, n is an integer of 1 to 4, and $M^1$ is Li, Na or K, wherein the fluorine-containing cyclic sulfonylimide salt is produced by subjecting a fluorosulfonyl group-containing carboxylic acid compound represented by the following general formula (3):

$$MO_2C\text{-}(CR_2)_m\text{-}SO_2F \qquad (3)$$

wherein Rs may be the same or different and R each is a fluorine atom or a trifluoromethyl group, m is 1 or 2, and M is H, Li, Na, K or $NH_4$, as a starting material, to an electrolytic coupling reaction step, a cyclization step and a cation exchange step, the method comprising a purification step of reducing the content of the fluorosulfonyl group-containing compound represented by the following general formula (5):

$$H\text{-}(CR_2)_m\text{-}SO_2F \qquad (5)$$

wherein Rs may be the same or different and R each is a fluorine atom or a trifluoromethyl group, and m is 1 or 2,

in the reaction solution, to 0.1% by mass or less after the electrolytic coupling reaction step.
<22> A method for producing a fluorine-containing cyclic sulfonylimide salt represented by the following general formula (1A):

[Chemical Formula 7]

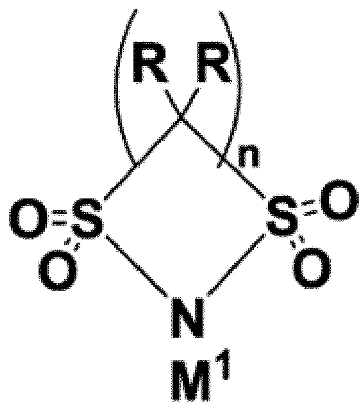

(1A)

wherein Rs may be the same or different and R each is a fluorine atom or a trifluoromethyl group, n is an integer of 1 to 4, and $M^1$ is Li, Na or K, wherein the fluorine-containing cyclic sulfonylimide salt is produced by subjecting a fluorosulfonyl group-containing carboxylic acid compound represented by the following general formula (3):

$MO_2C\text{-}(CR_2)_m\text{-}SO_2F$ (3)

wherein Rs may be the same or different and R each is a fluorine atom or a trifluoromethyl group, m is 1 or 2, and M is H, Li, Na, K or $NH_4$, as a starting material, to an electrolytic coupling reaction step, a cyclization step and a cation exchange step, the method comprising a crystallization step of dissolving the fluorine-containing cyclic sulfonylimide salt obtained after the cation exchange step in a monodentate chain ether solvent, followed by crystallization to obtain a purified fluorine-containing cyclic sulfonylimide salt.
<23> The method according to item 22, wherein the monodentate chain ether solvent used for crystallization is at least one solvent selected from the group consisting of ethyl ether, propyl methyl ether, propyl ethyl ether, propyl ether, isopropyl methyl ether, isopropyl ethyl ether, isopropyl ether, butyl methyl ether, butyl ethyl ether, isobutyl methyl ether, isobutyl ethyl ether, sec-butyl methyl ether, sec-butyl ethyl ether, tert-butyl methyl ether, tert-butyl ethyl ether, pentyl methyl ether, isopentyl methyl ether, sec-pentyl methyl ether, tert-pentyl methyl ether, neopentyl methyl ether and cyclopentyl methyl ether.
<24> The method according to item 22 or 23, wherein n is 2 in the general formula (1A).
<25> The method according to any one of items 22 to 24, wherein R is F in the general formula (1A).
<26> The method according to any one of items 22 to 25, wherein M is Li in the general formula (1A).
<27> The method according to any one of items 21 to 26, wherein, in the electrolytic coupling reaction step, the fluorosulfonyl group-containing carboxylic acid compound represented by the general formula (3) is subjected to an electrolytic coupling reaction in the presence of a mixed solvent of water and a nitrile group-containing solvent in which a ratio ($\beta/\alpha$) of the mass ($\alpha$) of water to the mass ($\beta$) of the nitrile group-containing solvent is 0.80 or less.
<28> The method according to item 27, wherein the ratio ($\beta/\alpha$) of the mass ($\alpha$) of water to the mass ($\beta$) of the nitrile group-containing solvent is 0.75 or less.
<29> The method according to any one of items 21 to 28, wherein, in the electrolytic coupling reaction step, the fluorosulfonyl group-containing carboxylic acid compound represented by the general formula (3) is subjected to an electrolytic coupling reaction in a reaction vessel in the presence of a mixed solvent of water and a nitrile group-containing solvent, and
wherein, during the reaction, the height (distance) TL from the bottom of the reaction vessel to the liquid surface of the reaction solution and the height (distance) TE from the bottom of the reaction vessel to the top of the electrode (highest level) satisfy the relationship TL > TE, and after the reaction, the height (distance) TS from the bottom of the reaction vessel to the interface between the organic phase and the aqueous phase and the height (distance) TE' from the bottom of the reaction vessel to the bottom of the electrode (lowest level) satisfy the relationship TE' > TS.
<30> The method according to item 29, wherein the TE' and TS satisfy the relationship: TE' $\times$ 0.6 > TS.
<31> The method according to any one of items 27 to 30, wherein a content of an alkali metal in the reaction solution

during the electrolytic coupling reaction is 2,000 ppm by mass or less.

<32> The method according to any one of items 27 to 31, wherein, in the electrolytic coupling reaction step, a molar ratio of the compound represented by the general formula (5) produced is set at 0.01 or less relative to 1 mol of the formation amount of a bis(fluorosulfonyl) compound (4) represented by the following general formula (4):

$$FO_2S\text{-}(CR_2)_m\text{-}SO_2F \qquad (4)$$

wherein Rs may be the same or different and R each is a fluorine atom or a trifluoromethyl group, and m is 1 or 2.

<33> The method according to any one of items 27 to 32, wherein, in the electrolytic coupling reaction step, the fluorosulfonyl group-containing carboxylic acid compound represented by the general formula (3) is subjected to an electrolytic coupling reaction in the presence of a mixed solvent of water and a nitrile group-containing solvent, and a ratio (6/y) of the mass (δ) of the mixed solvent to the mass (γ) of the fluorosulfonyl group-containing carboxylic acid compound is 1.5 or less.

<34> The method according to any one of items 27 to 33, wherein, in the electrolytic coupling reaction, a plate spacing between the anode and cathode is 0.001 mm to 9.9 mm.

<35> The method according to item 33 or 34, wherein, in the electrolytic coupling reaction, the yield of the compound (4) is set at 70% or more.

<36> The method according to any one of items 27 to 35, wherein the nitrile group-containing solvent is at least one selected from the group consisting of acetonitrile, propionitrile, butyronitrile and benzonitrile.

<37> The method according to item 36, wherein the nitrile group-containing solvent is acetonitrile.

<38> The method according to any one of items 27 to 37, wherein the electrolytic coupling reaction is carried out at a reaction temperature within a range of -35°C or higher and 10°C or lower.

[ADVANTAGEOUS EFFECTS OF INVENTION

The fluorine-containing cyclic sulfonylimide salt according to the present invention has heat-resistant properties and is usable in a wide temperature range, and has low metal corrosivity. According to the present invention, it is possible to provide a non-aqueous electrolyte solution which has low metal corrosivity, and excellent initial charging/discharging efficiency and cycle performance.

BRIEF DESCRIPTION OF DRAWINGS

FIG. 1 is a plan view schematically showing an example of a non-aqueous secondary battery of the present embodiment.

FIG. 2 is a cross-sectional view of the non-aqueous secondary battery of FIG. 1 taken along the line A-A.

FIG. 3 is a surface scanning electron microscope (SEM) photograph (at a magnification of 40 times) of a positive electrode aluminum (Al) current collector after a cycle test of a coin-type non-aqueous secondary battery fabricated with the non-aqueous electrolyte solution used in Example II-1.

FIG. 4 is a surface scanning electron microscope (SEM) photograph (at a magnification of 1,000 times) of a positive electrode Al current collector after a cycle test of a coin-type non-aqueous secondary battery fabricated with the non-aqueous electrolyte solution used in Example II-1.

FIG. 5 is a surface scanning electron microscope (SEM) photograph (at a magnification of 40 times) of a positive electrode Al current collector after a cycle test of a coin-type non-aqueous secondary battery fabricated with the non-aqueous electrolyte solution used in Comparative Example II-1.

FIG. 6 is a surface scanning electron microscope (SEM) photograph (at a magnification of 1,000 times) of a positive electrode Al current collector after a cycle test of a coin-type non-aqueous secondary battery fabricated with the non-aqueous electrolyte solution used in Comparative Example II-1.

FIG. 7 is a surface scanning electron microscope (SEM) photograph (at a magnification of 40 times) of a positive electrode Al current collector after a cycle test of a coin-type non-aqueous secondary battery fabricated with the non-aqueous electrolyte solution used in Comparative Example II-3.

FIG. 8 is a surface scanning electron microscope (SEM) photograph (at a magnification of 1,000 times) of a positive electrode Al current collector after a cycle test of a coin-type non-aqueous secondary battery fabricated with the non-aqueous electrolyte solution used in Comparative Example II-3.

FIG. 9 is a surface scanning electron microscope (SEM) photograph (at a magnification of 10,000 times) of a positive electrode Al current collector after a cycle test of a coin-type non-aqueous secondary battery fabricated with the non-aqueous electrolyte solution used in Comparative Example II-3.

FIG. 10 is a drawing showing an arrangement examples of a reaction solution, electrodes, etc. in a reaction vessel.

DESCRIPTION OF EMBODIMENTS

Embodiments for carrying out the present invention (hereinafter simply referred to as "present embodiment") will be described in detail below. The present invention is not limited to the following embodiments, and various modifications can be made without departing from the scope of the present invention. The numerical range described using "to" in the present description includes the numerical values described before and after the numerical range.

One embodiment of the present invention is directed to a fluorine-containing cyclic sulfonylimide salt composition comprising a fluorine-containing cyclic sulfonylimide salt represented by the following general formula (1A):

[Chemical Formula 8]

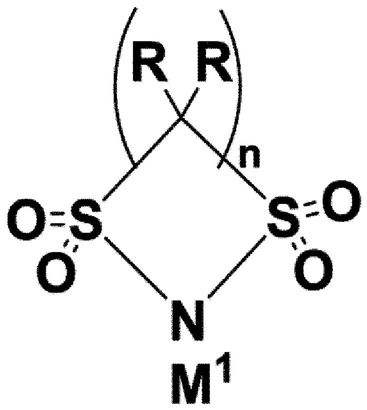

(1A)

wherein Rs may be the same or different and R each is a fluorine atom or a trifluoromethyl group, n is an integer of 1 to 4, and $M^1$ is Li, Na, K or $NH_4$ , wherein the temperature at which a mass reduction rate reaches 2% by mass when heated at a temperature rise rate of 10°C/min from 100°C under nitrogen stream is 385°C or higher.

Hereinafter, the compound (1A) will be described in detail.

<Composition Containing Fluorine-Containing Cyclic Sulfonylimide Salt (Compound (1A))>

In the general formula (1A), n is an integer of 1 to 4 from the viewpoint of ease of availability or production and excellent economic efficiency, and is preferably an integer of 1 to 3, more preferably 1 or 2, and most preferably 2 from the same viewpoint.

Rs may be the same or different, and R each is a fluorine atom or a trifluoromethyl group, and most preferably a fluorine atom, from the viewpoint of ease of availability or production and excellent economic efficiency.

$M^1$ is Li, Na, K or $NH_4$ (i.e., alkali metal ion, ammonium ion), preferably Li or Na, and most preferably Li, from the viewpoint of ease of availability or production and excellent economic efficiency.

Specific examples of the compound (1A) include compounds represented by the following structural formula:

[Chemical Formula 9]

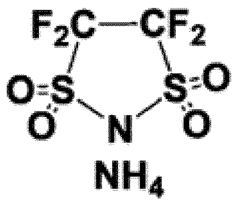

or

[Chemical Formula 10]

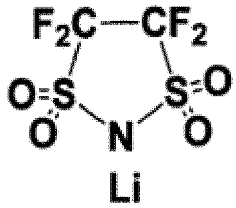

Of these, preferred is a compound represented by the following structural formula.

[Chemical Formula 11]

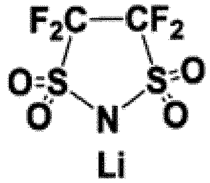

The composition including a compound (1A) preferably has a 2% mass reduction temperature of 385°C or higher when heated from 100°C at a temperature rise rate of 10°C/min under nitrogen stream, and more preferably 390°C or higher, and most preferably 395°C or higher, from the same viewpoint. The upper limit of the 2% mass reduction temperature is not particularly limited, but may be 1,000°C or lower or 500°C or lower. Here, the 2% mass reduction temperature is the temperature at which the mass reduction rate relative to the initial mass reaches 2% when thermogravimetric analysis is carried out under the above conditions. That is, the higher the 2% mass reduction temperature, the less decomposition products are generated, indicating higher heat-resistant properties.

The 2% mass reduction temperature is measured by heating from 25°C at a temperature rise rate of 10°C/min and maintaining at 100°C for 30 minutes to evaporate volatile components such as moisture, followed by heating from 100°C at a temperature rise rate of 10°C/min. The temperature at which the mass is reduced by 2% is determined based on the mass at the start of heating from 100°C

Examples of a device which can be used to measure the 2% mass reduction temperature include a simultaneous thermogravimetry/differential thermal analyzer (for example, "DTG-60A" manufactured by Shimadzu Corporation) or the like.

When the measurement sample contains moisture or residual solvent, it is difficult to obtain accurate measurement results. Therefore, the composition of the compound (1A) used for the above measurement preferably has a total content of moisture and residual solvent of 2,000 ppm by mass or less, and more preferably 1,500 ppm by mass or less, still more preferably 1,000 ppm by mass or less, and most preferably 600 ppm by mass or less, from the same viewpoint. The moisture content can be measured by an analytical instrument such as a Karl Fischer moisture meter, gas chromatography or liquid chromatography.

It is possible to obtain a fluorine-containing cyclic sulfonylimide salt composition in which the 2% mass reduction temperature is within the desired range, for example, by purifying the reaction solution obtained in an electrolytic coupling reaction step described below by distillation to remove the compound (5), or purifying the compound (1A) by crystallization. This composition is also obtained by performing both purification by distillation and crystallization. Further, the composition is obtained by adding the compound (2) after performing distillation or crystallization.

The content (purity) of the compound (1A) in the fluorine-containing cyclic sulfonylimide salt composition of the present embodiment is preferably 98.0% by mass or more, more preferably 99.0% by mass or more, and 99.5% by mass or more.

In the composition containing a fluorine-containing cyclic sulfonylimide salt (1A) of the present embodiment, the content of the fluorine-containing sulfonamide compound represented by the following general formula (2):

$$H(CR_2)_m\text{-}SO_2NHM^2 \qquad (2)$$

wherein Rs may be the same or different and R each is a fluorine atom or a trifluoromethyl group, m is 1 or 2, and $M^2$ is Li, Na, K, H or $NH_4$ , is preferably 10,000 ppm by mass (1% by mass) or less. When the content of the compound (2) is within the above range, the heat-resistant properties of the composition containing the compound (1A) are further enhanced, and the corrosiveness to metal is reduced, thus making it possible to suppress an increase in irreversible

capacity and deterioration of the cycle performance due to corrosion of a positive electrode Al current collector. From the same viewpoint, the content of the compound (2) is more preferably 5,000 ppm by mass or less, and most preferably 1,000 ppm by mass or less, relative to the total amount of the fluorine-containing cyclic sulfonylimide salt (1A). The lower limit of the content of the compound (2) is not particularly limited, but may be 0 ppm by mass or more, 0.001 ppm by mass or more, 10 ppm by mass or more, or 100 ppm by mass or more, relative to the total amount of the fluorine-containing cyclic sulfonylimide salt (1A).

The content of the compound (2) is calculated from an integrated value of a peak value of $^{19}$F-NMR. The content of the compound (2) is measured by the internal standard method (internal standard substance: benzotrifluoride). The content of the compound (2) is calculated by the following formula.

$$\text{Content of the compound (2) (\% by mass)} = (\text{Im} \times \text{Ci} \times \text{Cmw})/(\text{Ii} \times \text{Ia}) \times 100$$

wherein Im = number of mols of internal standard substance = mass of benzotrifluoride in measurement sample/molecular weight of benzotrifluoride
Ii = integrated value per 1F of internal standard substance = integrated value of benzotrifluoride/number of fluorine atoms of benzotrifluoride
Ci = integrated value per 1F of the compound (2) = integrated value of the compound (2)/number of fluorine atoms of the compound (2)
Cmw = molecular weight of the compound (2)
Ia = total mass of composition containing fluorine-containing cyclic sulfonylimide salt in measurement sample

When the content of the compound (2) in the non-aqueous electrolyte solution is 10 ppm by mass or less, and it is difficult to detect by the above internal standard method, regarding a sample obtained by redissolving a solid sample, which is obtained by removing a solvent in 100 mL of a non-aqueous electrolyte solution by distillation, in 0.5 mL of a solvent, the content of the compound (2) in the original non-aqueous electrolyte solution can be calculated by temporarily calculating the content of the compound (2) using the internal standard method and multiplying the value thus obtained by 200. Alternatively, the content of the compound (2) in the compound (1A) used as the starting material of the non-aqueous electrolyte solution is calculated using the internal standard method, and the compound (1A) in the non-aqueous electrolyte solution can be calculated by dividing by the dilution factor when diluted to the concentration of the compound (1A).

The structural formula of the compound (2) can be identified by one of general analytical techniques including mass spectrometry, nuclear magnetic resonance, infrared spectroscopy, elemental analysis, ion chromatography, ICP emission spectrometry, or combinations thereof.

It is possible to obtain a fluorine-containing cyclic sulfonylimide salt in which the content of the compound (2) is within the desired range, for example, by purifying the reaction solution obtained in an electrolytic coupling reaction step as described below due to distillation to remove the compound (5), or purifying the compound (1A) by crystallization. Purification by crystallization, which is highly effective in reducing the compound (2), is preferable, and the corrosiveness to metal is further reduced.

It is also possible to obtain a fluorine-containing cyclic sulfonylimide salt composition and a non-aqueous electrolyte solution in which the content of the compound (2) is in the desired range, by post-adding the compound (2) to the fluorine-containing cyclic sulfonylimide salt composition and the non-aqueous electrolyte solution.

In the general formula (2), the definition of R is the same as that of R in the general formula (1A). R is most preferably a fluorine atom. m is also most preferably 1. $M^2$ is Li, Na, K or $NH_4$ (i.e., alkali metal ion, ammonium ion), preferably Li or Na, and most preferably Li.

Specific examples of the compound (2) include compounds represented by the following structural formulas. Of these, $HCF_2$ $SO_2$ NHLi (compound (2-1)) and $HF_2CSO_2NHNH_4$ (compound (2-2)) are preferable, and $HCF_2$ $SO_2$ NHLi (compound (2-1)) is most preferable.

[Chemical Formula 12]

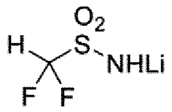 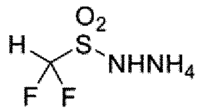 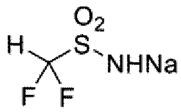 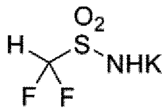

(2-1) (2-2)

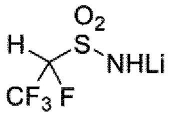 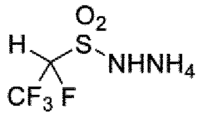 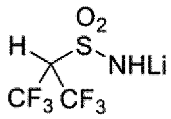 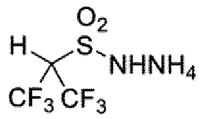

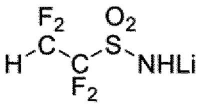 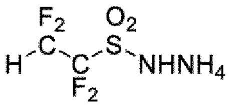 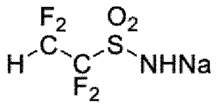 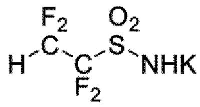

<Method for Producing Fluorine-Containing Cyclic Sulfonylimide Salt (Compound (1A))>

As described above, it is possible to adopt, as a method for producing a compound (1A), a conventionally known method, for example, the method described in PTL 3. The reaction scheme of the compound (1A) is shown below, taking the case where R is a fluorine atom and n is 2 as an example. As shown in the upper part of the following reaction scheme, such a method is a method in which a fluorosulfonyl group-containing carboxylic acid compound (hereinafter also referred to as compound (3)) represented by the following general formula (3):

$$HO_2C\text{-}(CR_2)_m\text{-}SO_2F \qquad (3)$$

wherein Rs may be the same or different and R each is a fluorine atom or a trifluoromethyl group, and m is 1 or 2, as a starting material, is subjected to an electrolytic coupling reaction step to obtain a compound (4), subjecting the resulting compound to a cyclization step to obtain a compound (1-N) and finally subjecting the resulting compound to a cation exchange step to produce a compound (1A). In the case of $M^1 = NH_4$ in the compound (1A), the cation exchange step can be omitted.

[Chemical Formula 13]

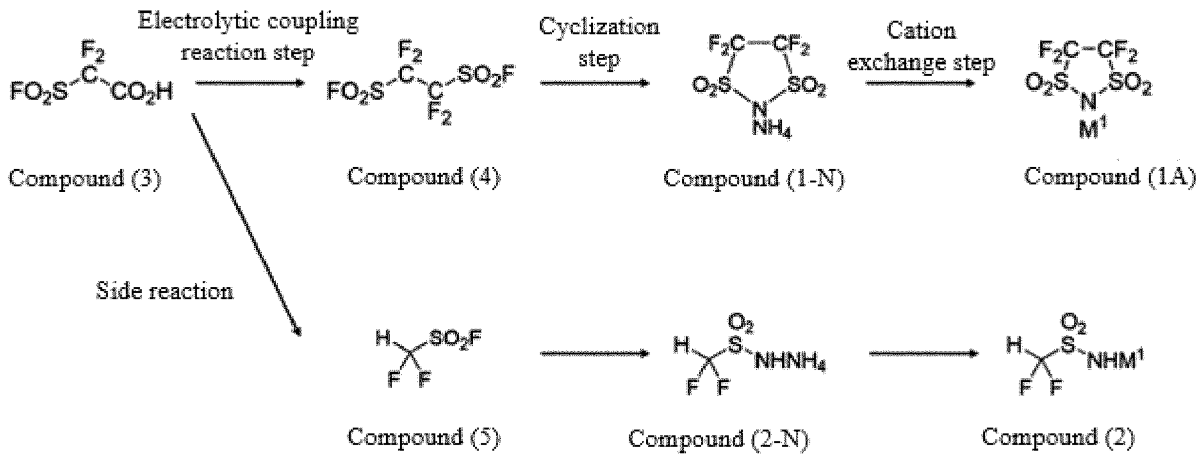


Compound (3) Compound (4) Compound (1-N) Compound (1A)

Compound (5) Compound (2-N) Compound (2)

However, according to the method of the prior art, the content of the compound (2) is reduced, thus failing to obtain a compound (1A) having the desired heat-resistant properties. As shown in the lower part of the reaction scheme, a compound (5) produced as a by-product in the electrolytic coupling reaction step affords a compound (2-N) through the cyclization step, and then the compound ( 2) is produced through the reaction in the cation exchange step.

As a result of intensive studies and repeated experiments, the inventors of the present application have found

that, as described above, the content of the compound (2) in the compound (1A) composition should be reduced to a predetermined value or less in order to enhance the heat-resistant properties of the compound (1A) composition as a final product and to reduce the corrosiveness to metal.

Further, in order to do so, as shown in the lower part of the above reaction scheme, the inventors have found that the content of the fluorosulfonyl group-containing compound (compound (5)) produced as a by-product in the electrolyte coupling reaction step should be reduced to 0.1% by mass or less in the reaction solution obtained in the step, and thus the present invention has been completed.

Namely, another embodiment of the present invention is directed to a method for producing a fluorine-containing cyclic sulfonylimide salt, wherein a fluorine-containing cyclic sulfonylimide salt represented by the following general formula (1A):

[Chemical Formula 14]

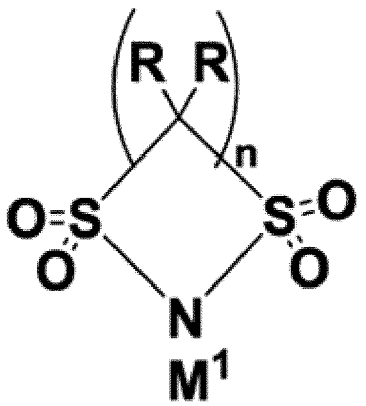

(1A)

wherein Rs may be the same or different and R each is a fluorine atom or a trifluoromethyl group, n is an integer of 1 to 4, and $M^1$ is Li, Na or K, is produced by subjecting a fluorosulfonyl group-containing carboxylic acid compound represented by the following general formula (3):

$HO_2C-(CR_2)_m-SO_2F$ (3)

wherein Rs may be the same or different and R each is a fluorine atom or a trifluoromethyl group, m is 1 or 2, as a starting material, to an electrolytic coupling reaction step, a cyclization step and a cation exchange step, the method comprising a purification step of reducing the content of the fluorosulfonyl group-containing compound represented by the following general formula (5):

$H-(CR_2)m-SO_2F$ (5)

wherein Rs may be the same or different and R each is a fluorine atom or a trifluoromethyl group, and m is 1 or 2, in the reaction solution, to 0.1% by mass or less after the electrolytic coupling reaction step. The compound (5) is a by-product of a bis(fluorosulfonyl) compound represented by the following general formula (4):

$FO_2S-(CR_2)_m-SO_2F$ (4)

wherein Rs may be the same or different and R each is a fluorine atom or a trifluoromethyl group, and m is 1 or 2, which is obtained by purifying the reaction solution obtained in the electrolytic coupling reaction step.

Further, in order to reduce the content of the compound (2) in the compound (1A) composition to a predetermined value or less, the inventors have found that the fluorine-containing cyclic sulfonylimide salt (1A) obtained after the cation exchange step should be purified by dissolving the fluorine-containing cyclic sulfonylimide salt obtained after the cation exchange step in a monodentate chain ether solvent, followed by crystallization, and thus the present invention has been completed.

Namely, another embodiment of the present invention is directed to a method for producing a fluorine-containing cyclic sulfonylimide salt represented by the general formula (1A) by subjecting a fluorosulfonyl group-containing carboxylic acid compound represented by the general formula (3), as a starting material, to an electrolytic coupling reaction step, a cyclization step and a cation exchange step, the method comprising a crystallization step of dissolving the fluorine-containing cyclic sulfonylimide salt obtained after the cation exchange step in a monodentate chain ether solvent, followed by crystallization to obtain a purified fluorine-containing cyclic sulfonylimide salt.

Hereinafter, such production method will be described in detail.

<Electrolytic Coupling Reaction Step>

A bis(fluorosulfonyl) compound (compound (4)) can be produced by subjecting a compound (3) to an electrolytic coupling reaction. Even if a commercially available compound (3) is used, it is possible to use a known method, for example, a method in which $SO_3$ is added to tetrafluoroethylene to obtain sultone, and a sultone ring-opened product is obtained in the presence of $NEt_3$, and then the sultone ring-opened product is hydrolyzed to obtain the compound (3) as a sultone hydrolysate.

In the general formula (3), the definition of R is the same as that of R in the general formula (1A). R is most preferably a fluorine atom. m is also most preferably 1.

Specific examples of the compound (3) include compounds represented by the following structural formulas. Of these, $FO_2SCF_2CO_2H$ (2,2-difluoro-2-(fluorosulfonyl)acetic acid, compound (3-1)) is preferable.

[Chemical Formula 15]

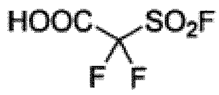

(3-1)

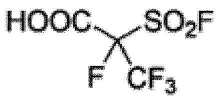

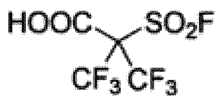

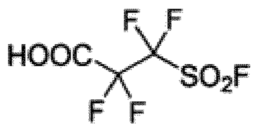

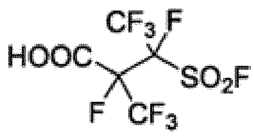

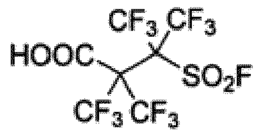

The reaction temperature of the electrolytic coupling reaction step is not particularly limited, as long as it is within a generally used range, but it is preferably -50°C or higher and 70°C or lower. When the reaction temperature is -50°C or higher, it is preferable because the electrical resistance decreases and heat generation can be suppressed, thereby facilitating temperature control. From the same viewpoint, the reaction temperature is more preferably - 40°C or higher, still more preferably -35°C or higher, and most preferably -30°C or higher. When the reaction temperature is 70°C or lower, it is preferable because decomposition of the compounds (3) and (4) can be suppressed and the yield of the compound (4) tends to increase. From the same viewpoint, the reaction temperature is more preferably 50°C or lower, still more preferably 40°C or lower, and most preferably 10°C or lower.

A solvent may be used in the electrolytic coupling reaction step.

The solvent is not particularly limited, as long as it is commonly used, and specific examples thereof include aromatic solvents such as benzene, toluene, chlorobenzene and 1,2-dichlorobenzene; ether group-containing solvents such as 1,2-dimethoxyethane, 1,2-diethoxyethane, diethylene glycol dimethyl ether, triethylene glycol dimethyl ether, tetraethylene glycol dimethyl ether, tetrahydrofuran, 2-methyltetrahydrofuran, 1,4-dioxane, 4-methyltetrahydropyran, cyclopentyl methyl ether and anisole; nitrile group-containing solvents such as acetonitrile, propionitrile, butyronitrile and benzonitrile; hydroxyl group-containing solvents such as water, methanol, ethanol, 1-propanol, 2-propanol, 1-butanol and 2-butanol; and carbonate group-containing solvents such as dimethyl carbonate, diethyl carbonate, ethyl methyl carbonate and propylene carbonate. Of these, because of a tendency of an increase in the yield of the compound (4), preferred are nitrile solvents such as acetonitrile, propionitrile, butyronitrile and benzonitrile; hydroxyl group-containing solvents such as water, methanol, ethanol, 1-propanol, 2-propanol, 1-butanol and 2-butanol; and carbonate group-containing solvents such as dimethyl carbonate, diethyl carbonate, ethyl methyl carbonate and propylene carbonate.

These solvents may be used alone, or a plurality thereof may be used in combination. Because of a tendency of an increase in the yield of the compound (4), a mixed solvent of water and a nitrile group-containing solvent, or a mixed solvent of water and a carbonate group-containing solvent is preferable, and a mixed solvent of water and a nitrile group-containing solvent is more preferable, and a mixed solvent of water and acetonitrile is most preferable. The amount of acetonitrile in the solvent is preferably 0.5 to 90% by weight.

In the present embodiment, it is preferable that the fluorosulfonyl group-containing carboxylic acid compound represented by the general formula (3) is subjected to an electrolytic coupling reaction in the presence of a mixed solvent

of water and a nitrile group-containing solvent in which a ratio (β/α) of the mass (α) of water to the mass (β) of the nitrile group-containing solvent is 0.80 or less, thus producing a bis(fluorosulfonyl) compound represented by the general formula (4). When β/α is 0.80 or less, production of the compound (5) can be suppressed. From the same viewpoint, β/α is more preferably 0.75 or less, still more preferably 0.5 or less, yet more preferably 0.2 or less, and further preferably 0.1 or less. The lower limit of β/α is not particularly limited, but may be 0.01 or more, 0.03 or more, 0.05 or more, or 0.18 or more.

PTL 3 discloses a method for producing a bis(fluorosulfonyl) compound ($FO_2 SCF_2 CF_2 SO_2 F$) in one step by an electrolytic coupling reaction using $FO_2SCF_2CO_2H$ as a starting material. However, when the bis(fluorosulfonyl) compound is produced according to PTL 3, a fluorosulfonyl group-containing compound represented by the general formula (5) is mixed as a by-product. As described above, the bis(fluorosulfonyl) compound is used as catalysts and electrolytes, and in these fields, of a trace amount of impurities to be mixed sometimes significantly cause deterioration of performances such as catalyst activity and electrical conductivity. Therefore, when the bis(fluorosulfonyl) compound is produced according to PTL 3, there is a need to separate and remove the fluorosulfonyl group-containing compound by a purification operation such as distillation, and there has been required a method for producing a bis(fluorosulfonyl) compound having a small content of a fluorosulfonyl group-containing compound.

The present inventors have unexpectedly found that a compound (4) having a small content of a fluorosulfonyl group-containing compound (compound (5)) as a by-product can be obtained by setting the ratio (β/α) of the mass of water (α) to the mass of the nitrile group-containing solvent at a specific value or less, and thus the present invention has been completed.

In the present embodiment, preferred is a method for producing a bis(fluorosulfonyl) compound represented by the general formula (4) (compound 4) by subjecting a fluorosulfonyl group-containing carboxylic acid compound represented by the general formula (3) to an electrolytic coupling reaction in a reaction vessel in the presence of a mixed solvent of water and a nitrile group-containing solvent, wherein, during the reaction, the height (distance) TL from the bottom of the reaction vessel to the liquid surface of the reaction solution and the height (distance) TE from the bottom of the reaction vessel to the top of the electrode (highest level) satisfy the relationship TL > TE, and after the reaction, the height (distance) TS from the bottom of the reaction vessel to the interface between the organic phase and the aqueous phase and the height (distance) TE' from the bottom of the reaction vessel to the bottom of the electrode (lowest level) satisfy the relationship TE' > TS.

As shown in FIG. 10, in the present embodiment, it is preferable that, during the reaction, the height (distance) TL from the bottom of the reaction vessel to the liquid surface of the reaction solution and the height (distance) TE from the bottom of the reaction vessel to the top of the electrode (highest level) satisfy the relationship TL > TE, and after the reaction, the height (distance) TS from the bottom of the reaction vessel to the interface between the organic phase and the aqueous phase and the height (distance) TE' from the bottom of the reaction vessel to the bottom of the electrode (lowest level) satisfy the relationship TE' > TS. When TL and TE, and TE' and TS satisfy the above relationships, respectively, a rapid increase in voltage during the electrolytic coupling reaction is suppressed, and the production method tends to be safe.

From the same viewpoint, the relationship between TE' and TS more preferably satisfies the relationship $TE' \times 0.6 > TS$, and most preferably further satisfies the relationship $TE' \times 0.8 > TS$.

PTL 3 discloses a method for producing a bis(fluorosulfonyl) compound ($FO_2 SCF_2 CF_2 SO_2 F$) in one step by an electrolytic coupling reaction using $FO_2 SCF_2 CO_2 H$ as a starting material. However, when the bis(fluorosulfonyl) compound is produced according to PTL 3, hunting such as a sudden increase in voltage during the electrolytic coupling reaction is observed, and an improvement in stability of the electrolytic coupling reaction is required.

The present inventors have found that, during the reaction, the height (distance) TL from the bottom of the reaction vessel to the liquid surface of the reaction solution and the height (distance) TE from the bottom of the reaction vessel to the top of the electrode (highest level) satisfy the relationship TL > TE, and after the reaction, the height (distance) TS from the bottom of the reaction vessel to the interface between the organic phase and the aqueous phase and the height (distance) TE' from the bottom of the reaction vessel to the bottom of the electrode (lowest level) satisfy the relationship TE' > TS, whereby, the occurrence of hunting can be suppressed and the reactor is excellent in operation stability, and thus the present invention has been completed.

In the present embodiment, in the electrolytic coupling reaction step in which a fluorosulfonyl group-containing carboxylic acid compound represented by the general formula (3) is subjected to an electrolytic coupling reaction in the presence of a mixed solvent of water and a nitrile group-containing solvent, the ratio (6/y) of the mass (γ) of the compound (3) to the mass (δ) of the mixed solvent is preferably 1.5 or less. When 6/y is 1.5 or less, it is preferable because the yield of the compound (2) tends to be higher. From the same viewpoint, 6/y is preferably 1.35 or less, and most preferably 1.00 or less. The lower limit of 6/y is not particularly limited, but may be 0.01 or more, 0.05 or more, or 0.1 or more.

The electrode used in the electrolytic coupling reaction step is not particularly limited, as long as it is an electrode which is commonly used, and is preferably a platinum electrode from the viewpoint of suppressing the decomposition of the solvent.

In present embodiment, in the electrolytic coupling reaction, the electrode plate spacing between an anode and a cathode is preferably 0.001 mm to 9.9 mm. When the electrode plate spacing is 0.001 mm or more, it is preferable because the electrolyte solution easily flows between the electrode plates, which tends to increase the yield of the compound (4). When the electrode plate spacing is 9.9 mm or less, it is preferable because the voltage is reduced and the side reaction is suppressed, which tends to increase the yield of the compound (4). From the same viewpoint, the electrode plate spacing between the anode and the cathode is preferably 0.01 mm to 5 mm, and most preferably 0.1 mm to 2.9 mm.

PTL 3 discloses a method for producing a bis(fluorosulfonyl) compound ($FO_2SCF_2CF_2SO_2F$) in one step by an electrolytic coupling reaction using $FO_2$ $SCF_2$ $CO_2$ H as a starting material. However, PTL 3 specifically describes neither the mixing ratio of the starting material to the solvent during the electrolytic coupling reaction nor the electrode plate spacing between the anode and the cathode. In fact, even when $FO_2$ $SCF_2$ $CO_2$ H is used as a starting material, the yield of the bis(fluorosulfonyl) compound varies greatly depending on the mixing ratio of the starting material to the solvent. Depending on the electrode plate spacing between the anode and cathode, the target bis(fluorosulfonyl) compound may not be obtained at all.

The present inventors have unexpectedly found that a bis(fluorosulfonyl) compound can be stably obtained in a high yield by a combination of setting the ratio (6/y) of the mass (δ) of the mixed solvent to the mass (γ) of the fluorosulfonyl group-containing carboxylic acid compound at a specific value or less, and setting the electrode plate spacing between the anode and cathode within a certain range, and thus the present invention has been completed.

In the present embodiment, the content of alkali metal in the reaction solution in the electrolytic coupling reaction is preferably 2,000 ppm by mass or less. When the alkali metal content is within the above range, the decomposition of the compound (3) is suppressed, and the yield of the compound (4) tends to increase. The content of the alkali metal in the reaction solution is more preferably 1,000 ppm by mass or less, and still more preferably 500 ppm by mass or less.

In the electrolytic coupling reaction step, it is possible to use, as a method of separating each from the reaction product containing the compound (3), the compound (4) and the solvent after the electrolytic coupling reaction of the compound (3), a method of removing by separation without particular limitation, as long as it is commonly used. Examples thereof include separation and removal of volatile components by distillation, separation of liquid components by separation operation, separation and removal of insoluble solids by filtration and the like.

These methods may be used alone, or a plurality of methods may be used in combination.

In the electrolytic coupling reaction step, $H-(CR_2)_m-SO_2F$ wherein Rs may be the same or different and each of Rs is a fluorine atom or a trifluoromethyl group, and m is 1 or 2 (compound (5)) may be sometimes produced, together with the target compound (4), as by-products. The compound (5) changes to a compound (2) through a cyclization step and a cation exchange step following the electrolytic coupling reaction step, thus causing deterioration of the heat-resistant properties of the compound (1A). Further, both the compounds (1A) and (2) are solids, and it is difficult to remove the compound (2) from a mixture of the compounds (1A) and (2) by separation. Therefore, after the electrolytic coupling reaction step, the content of the compound (5), which causes deterioration of the heat-resistant properties of the compound (1A), is preferably reduced to 0.1% by mass or less in the reaction solution obtained in the step.

The detailed reason why the compound represented by the general formula (5) is by-produced during the electrolytic coupling reaction of the compound (3) is not clear, but is considered as follows: as shown in the reaction scheme below, the carboxyl group of the compound (3) and the nitrile group of the solvent interact with each other to promote the decarboxylation reaction of the compound (3), followed by reaction with water of the solvent to form a compound (5).

[Chemical Formula 16]

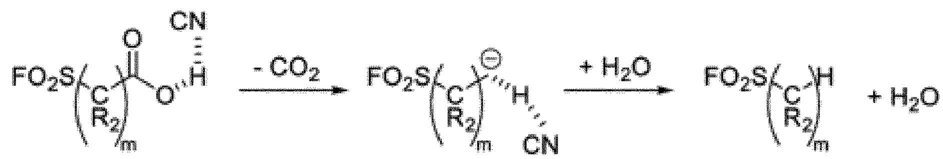


It is possible to use, as the method of separating each of the compounds (4) and (5) from the mixture, a method of removing by separation without particular limitation, as long as it is commonly used. Examples thereof include a separation method by distillation.

In general formula (5), the definition of R is the same as that of R in the general formula (1A). R is most preferably a fluorine atom. m is also most preferably 1.

Specific examples of the compound (5) include compounds represented by the following structural formulas. Of these, $HCF_2SO_2F$ (1,1-difluoromethanesulfonyl fluoride, compound (5-1)) is preferable.

[Chemical Formula 17]

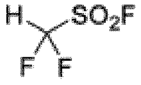

(5-1)

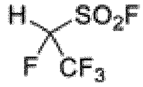

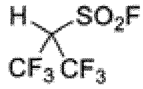

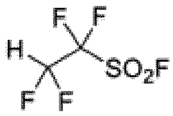

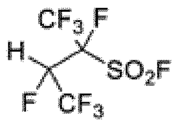

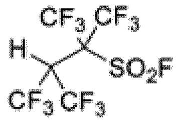

The current density during electrolytic coupling is preferably 0.001 A/$cm^2$ to 2.0 A/$cm^2$ . When the current density is within the above range, it is preferable because the side reaction is suppressed and the yield of the compound (4) tends to be higher. From the same viewpoint, the current density is more preferably 0.005 A/$cm^2$ to 1.5 A/$cm^2$ , and most preferably 0.01 A/$cm^2$ to 1.0 A/$cm^2$ .

An electrolysis vessel used in the present embodiment is one commonly used in an organic electrolytic reaction, and may be of either a batch type or a continuous type in which the electrolytic reaction is carried out while supplying the compound (3) or the like.

The reaction time of the electrolytic coupling reaction step is not particularly limited, as long as it is within the range which is commonly used, but is preferably 0.5 to 100 hours, and more preferably 1 to 50 hours.

The pressure of the electrolytic coupling reaction step depends on the reaction temperature, but is not particularly limited, as long as it is within the range which is commonly used range, but is preferably 10 kPa to 5,000 kPa.

The reaction atmosphere is not particularly limited, as long as it is an atmosphere which is commonly used, but an air atmosphere, a nitrogen atmosphere, an argon atmosphere or the like is commonly used. Of these, a nitrogen atmosphere and an argon atmosphere are preferable because the compound (4) tends to be produced more safely. A nitrogen atmosphere is more preferable because and the production method tends to be more economical.

The reaction atmosphere may be used alone, or a plurality of reaction atmospheres may be used in combination.

A supporting electrolyte may be added during the electrolytic coupling reaction. The supporting electrolyte is not particularly limited, as long as it is a supporting electrolyte which is commonly used, and specific examples thereof include metal hydroxides such as lithium hydroxide, sodium hydroxide and potassium hydroxide; sulfates such as sodium sulfate and potassium sulfate; acid compounds such as nitric acid, sulfuric acid and hydrochloric acid; perchlorates such as sodium perchlorate and ammonium perchlorate; and quaternary ammonium salts such as tetramethylammonium bromide and tetrabutylammonium paratoluenesulfonate.

In the production method of the present embodiment, the molar ratio of the compound (5) produced is preferably set at 0.01 or less relative to 1 mol of the compound (4) formed in the electrolytic coupling reaction. The molar ratio of the compound (5) produced is more preferably set at 0.008 or less, still more preferably 0.005 or less, and yet more preferably 0.003 or less. By performing the electrolytic coupling reaction within a predetermined range of $\beta/\alpha$, as described above, the production molar ratio can be set within the above range. The production ratio is obtained by measuring the reaction solution after electrolytic coupling with $^{19}$F-NMR. Specific conditions are described in Examples.

The yield of the compound (4) in the electrolytic coupling reaction step is preferably 60% or more, more preferably 70% or more, still more preferably 80% or more, and particularly preferably 90% or more. By performing the electrolytic coupling reaction step in the above yield of the compound (4), the production cost of the compound (4) can be reduced and the production amount per unit time can be improved.

<Cyclization Step>

By reacting the compound (4) with ammonia, it is possible to produce a fluorine-containing cyclic sulfonylimide ammonium salt (compound (1-N)) represented by the following general formula (1-N):

[Chemical Formula 18]

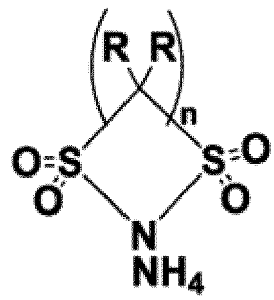

(1-N)

wherein Rs may be the same or different and R each is a fluorine atom or a trifluoromethyl group, and n is an integer of 1 to 4.

In general formula (4), the definition of R is the same as that of R in the general formula (1A). R is most preferably a fluorine atom. m is most preferably 1.

Specific examples of the compound (4) include compounds represented by the following structural formulas. Of these, $FO_2SCF_2CF_2SO_2F$ (1,1,2,2-tetrafluoro-1,2-ethanedisulfonyldifluoride, compound (4-1)) is preferable.

[Chemical Formula 19]

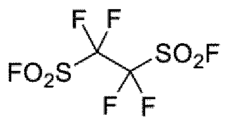

(4-1)

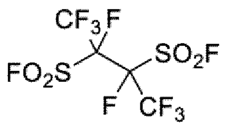

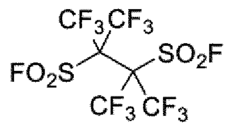

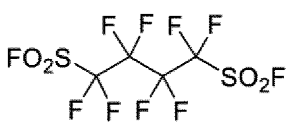

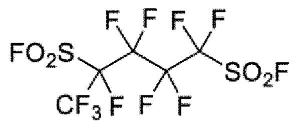

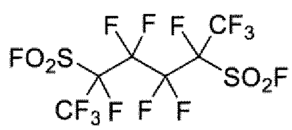

The molar ratio ($\mu/\lambda$) of the molar number ($\mu$) of ammonia to the molar number ($\lambda$) of the compound (4) is not particularly limited, as long as it is within the range which is commonly used, but is preferably 2 to 50. When $\mu/\lambda$ is 2 or more, it is preferable because the yield of the compound (1-N) tends to be higher. When $\delta/\gamma$ is 50 or less, it is preferable because waste can be reduced, and the production method tends to be more economical. From the same viewpoint, $\mu/\lambda$ is more preferably 5 to 40, and most preferably 10 to 30.

A solvent may be used in the cyclization step.

The solvent is not particularly limited, as long as it is inert during the reaction and is commonly used. Specific examples thereof include aromatic solvents such as benzene, toluene, chlorobenzene and 1,2-dichlorobenzene; ether group-containing solvents such as 1,2-dimethoxyethane, 1,2-diethoxyethane, diethylene glycol dimethyl ether, triethylene glycol dimethyl ether, tetraethylene glycol dimethyl ether, tetrahydrofuran, 2-methyltetrahydrofuran, 1,4-dioxane, 4-methyltetrahydropyran, cyclopentyl methyl ether and anisole; nitrile group-containing solvents such as acetonitrile, propionitrile and benzonitrile; and hydroxyl group-containing solvents such as methanol, ethanol, 1-propanol, 2-propanol, 1-butanol and 2-butanol. Of these, since the yield of the compound (1-N) tends to be higher, more preferred are ether group-containing solvents such as 1,2-dimethoxyethane, 1,2-diethoxyethane, diethylene glycol dimethyl ether, triethylene glycol dimethyl ether, tetraethylene glycol dimethyl ether, tetrahydrofuran, 2-methyltetrahydrofuran, 1,4-dioxane, 4-methyltetrahydropyran, cyclopentyl methyl ether and anisole. From the same viewpoint, 1,2-dimethoxyethane, tetrahydrofuran, 1,4-dioxane and 4-methyltetrahydropyran are more preferable, and 1,2-dimethoxyethane, tetrahydrofuran and 4-methyltetrahydropyran are still more preferable.

These organic solvents may be used alone, or a plurality of solvents may be used in combination.

The ratio ($\zeta/\varepsilon$) of the mass ($\varepsilon$) of the compound (4) to the mass ($\zeta$) of the solvent is not particularly limited, as long as it is within a range which is commonly used, but is preferably 0.01 to 100. When $\zeta/\varepsilon$ is within the above range, the yield of the compound (1-N) tends to be higher. From the same viewpoint, $\zeta/\varepsilon$ is more preferably 0.05 to 50, and more preferably 0.1 to 10.

The reaction temperature of the cyclization step is not particularly limited, as long as it is within the range which

is commonly used, but it is preferably -90°C to 20°C. When the reaction temperature is -90°C or higher, it is preferable because the reactivity of the compound (4) is enhanced and the yield of the compound (1-N) tends to be higher. When the reaction temperature is 20°C or lower, it is preferable because a by-product can be suppressed and the yield of the compound (1-N) tends to be higher. From the same viewpoint, the reaction temperature is more preferably -80°C to 10°C, and most preferably -80°C to 0°C.

The reaction time of the cyclization step is not particularly limited, as long as it is within the range which is commonly used, but is preferably 1 to 100 hours, and more preferably 5 to 50 hours.

The pressure of the cyclization step depends on the temperature at which the reaction is carried out, but is not particularly limited, as long as it is within the range which is commonly used, but is preferably 10 kPa to 5,000 kPa.

The reaction atmosphere is not particularly limited, as long as it is an atmosphere which is commonly used, but an air atmosphere, a nitrogen atmosphere, an argon atmosphere or the like is commonly used. Of these, a nitrogen atmosphere and an argon atmosphere are preferable because the compound (1-N) tends to be produced more safely. A nitrogen atmosphere is more preferable because and the production method tends to be more economical.

The reaction atmosphere may be used alone, or a plurality of reaction atmospheres may be used in combination.

In the cyclization step, it is possible to use, as a method of separating each from the reaction product containing the compound (1-N) and the solvent after the reaction between the compound (4) and ammonia, a method of removing by separation without particular limitation, as long as it is commonly used. Examples thereof include separation and removal of volatile components by distillation, separation of liquid components by separation operation, separation and removal of insoluble solids by filtration and the like.

These methods may be used alone, or a plurality of methods may be used in combination.

<Cation Exchange Step>

It is possible to produce a compound (1A) by reacting a compound (1-N) with an alkali metal salt (hereinafter also referred to as compound (6)) represented by the following general formula (6):

$$M^1_pX \qquad (6)$$

wherein $M^1$ is Li, Na or K, X is OH or $CO_3$, and p is 1 when X is OH, or 2 when X is $CO_3$ .

In general formula (1-N) below, the definition of R is the same as that of R in the general formula (1A). R is most preferably a fluorine atom. n is preferably 1 or 2, and most preferably 2. m is most preferably 1. n in the formula (1-N) is an integer of 1 to 4.

Specific examples of the compound (1-N) include ammonium salts represented by the following general formulas (1-N-1) to (1-N-5).

[Chemical Formula 20]

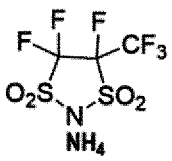
(1-N-1)

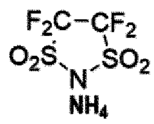
(1-N-2)

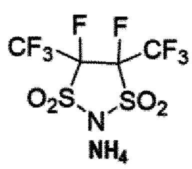
(1-N-3)

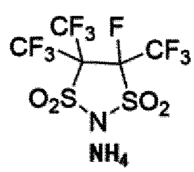
(1-N-4)

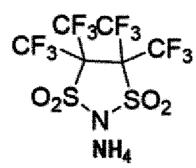
(1-N-5)

Of these, an ammonium salt represented by the (1-N-2) is preferable.

[Chemical Formula 21]

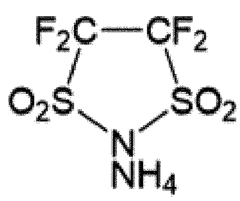

(1-N-2)

The molar ratio (θ/η) of the molar number (θ) of the compound (6) to the molar number (η) of the compound (1-N) is not particularly limited, as long as it is within the range which is commonly used, but is preferably 0.2 to 10. When θ/η is 0.2 or more, it is preferable because the yield of the compound (1A) tends to be higher. When θ/η is 10 or less, it is preferable because waste can be reduced, and the manufacturing method tends to be more economical. From the same viewpoint, θ/η is more preferably 0.5 to 5, and most preferably 0.8 to 3.

A solvent may be used in the cation exchange step.

The solvent is not particularly limited, as long as it is inert during the reaction and is commonly used. Specific examples thereof include aromatic solvents such as benzene, toluene, chlorobenzene and 1,2-dichlorobenzene; ether group-containing solvents such as 1,2-dimethoxyethane, 1,2-diethoxyethane, diethylene glycol dimethyl ether, triethylene glycol dimethyl ether, tetraethylene glycol dimethyl ether, tetrahydrofuran, 2-methyltetrahydrofuran, 1,4-dioxane, 4-methyltetrahydropyran, cyclopentyl methyl ether and anisole; nitrile group-containing solvents such as acetonitrile, propionitrile and benzonitrile; and hydroxyl group-containing solvents such as methanol, ethanol, 1-propanol, 2-propanol, 1-butanol and 2-butanol. Of these, since the yield of the compound (1A) tends to be higher, more preferred are ether group-containing solvents such as 1,2-dimethoxyethane, 1,2-diethoxyethane, diethylene glycol dimethyl ether, triethylene glycol dimethyl ether, tetraethylene glycol dimethyl ether, tetrahydrofuran, 2-methyltetrahydrofuran, 1,4-dioxane, 4-methyltetrahydropyran, cyclopentylmethyl ether and anisole. From the same viewpoint, 1,2-dimethoxyethane, tetrahydrofuran, 1,4-dioxane and 4-methyltetrahydropyran are more preferable, and 1,2-dimethoxyethane, tetrahydrofuran and 4-methyltetrahydropyran are still more preferable.

These organic solvents may be used alone, or a plurality of solvents may be used in combination.

The ratio (κ/ι) of the mass (κ) of the solvent to the mass (t) of the compound (1-N) is not particularly limited, as long as it is within the range which is commonly used, and is preferably 0.01 to 100. When κ/ι is within the above range, the yield of the compound (1A) tends to be higher. From the same viewpoint, it is more preferably 0.05 to 50, and more preferably 0.1 to 10.

The reaction temperature of the cation exchange step is not particularly limited, as long as it is within the range which is commonly used, but is preferably 0°C to 150°C. When the reaction temperature is 0°C or higher, it is preferable because the reactivity of the compound (1-N) is enhanced, and the yield of the compound (1A) tends to be higher. When the reaction temperature is 150°C or lower, it is preferable because a by-product can be suppressed and the yield of the compound (1A) tends to be higher. From the same viewpoint, the reaction temperature is more preferably 20°C to 140°C, and most preferably 40°C to 130°C

The reaction time of the cation exchange step is not particularly limited, as long as it is within the range which is commonly used, but is preferably 1 to 100 hours, and more preferably 5 to 50 hours.

The pressure of the cation exchange step depends on the temperature at which the reaction is carried out, but is not particularly limited, as long as it is within the range which is commonly used, but is preferably 10 kPa to 5,000 kPa.

The reaction atmosphere is not particularly limited, as long as it is an atmosphere which is commonly used, but an air atmosphere, a nitrogen atmosphere, an argon atmosphere or the like is commonly used. Of these, a nitrogen atmosphere and an argon atmosphere are preferable because the compound (1A) tends to be produced more safely. A nitrogen atmosphere is more preferable because and the production method tends to be more economical.

A single reaction atmosphere may be used, or a plurality of reaction atmospheres may be used in combination.

<Crystallization Step of Fluorine-Containing Cyclic Sulfonylimide Salt (Compound (1A))>

A fluorine-containing cyclic sulfonylimide salt commonly has extremely high solubility in water and various coordinating organic solvents, which favors its use in a wide range of applications such as antistatic agents, ion conductive materials such as organic electrolytes, and flame retardants. Meanwhile, this high solubility is directly linked to low crystallinity and an increase in amount of the residual solvent during drying. Crystallization is one of the most common and simple production methods for purifying powdery substances such as fluorine-containing cyclic sulfonylimide salts, and the low crystallinity and increase in amount of the residual solvent become problematic, and thus there is a limitation on the existing technologies for purification by crystallization of the fluorine-containing cyclic sulfonylimide salt.

PTL 4 describes Examples of purification by crystallization of a lithium salt in a mixed solvent of 1,4-dioxane as a poor solvent and acetonitrile as a good solvent. However, according to the technique of PTL 1 described above, about 5% by mass of 1,4-dioxane remains in the crystals after drying, and there is a problem that it takes a long time and a lot of money to remove the residual solvent. It is considered that this problem is essentially due to the use of 1,4-dioxane, a cyclic ether with two oxygen atoms, leading to strong coordination of the solvent to lithium in the crystalline state. PTL 4 describes a high purification method by suspension filtration and washing of the same fluorine-containing cyclic sulfonyl imide ammonium salt with 1,4-dioxane, but does not describe the amount of the residual solvent after drying, and it is unclear whether this will contribute to solution of the problem related to the amount of residual solvent.

In fact, the present inventors, in the course of the study to invent a crystallization method that is simple and can achieve high purity, tried to crystallize the fluorine-containing cyclic sulfonylimide salt by dissolving it in a solvent

such as dimethyl carbonate, acetonitrile, tetrahydrofuran or acetone, and removing the solvent, followed by concentration. However, it was confirmed that the entire mixture became gelatinous during concentration and did not crystallize and dry up even if the concentration was continued, and that no crystals were formed even if the concentrated solution was cooled, thus failing to achieve purification. Thus, high affinity with the solvent capable of dissolving the fluorine-containing cyclic sulfonylimide salt was a barrier to crystallization of the fluorine-containing cyclic sulfonylimide salt.

As a result of intensive studies, the present inventors have unexpectedly found that, in crystallization, a fluorine-containing cyclic sulfonylimide salt with extremely high purity and low amount of the residual solvent can be easily obtained by using a monodentate chain ether solvent, and thus the present invention has been completed.

The crystallization step may be carried out by crystallization by cooling. In view of high hygroscopicity of the fluorine-containing cyclic sulfonylimide salt, the crystallization step is preferably carried out in a dry atmosphere because the obtained crystals are easy to handle. Although the drying atmosphere is not particularly limited, drying can be carried out in an atmosphere of gas such as dry air, dry nitrogen or dry argon, in an air stream, or in a vacuum or under reduced pressure.

Hereinafter, the term "poor solvent" means a solvent suitable for forming crystals when a solution of the fluorine-containing cyclic sulfonylimide salt is cooled, and does not necessarily have significantly low solubility in which the fluorine-containing cyclic sulfonylimide salt is dissolved.

The solvent used for crystallization in the production method of the present embodiment is a monodentate chain ether solvent from the viewpoint of satisfying both relatively high solubility of the fluorine-containing cyclic sulfonylimide salt and low coordination with metal. Although it is not clear the reason why a highly pure fluorine-containing cyclic sulfonylimide salt with reduced amounts of residual fluorine impurities and residual solvent can be easily produced when a monodentate chain ether solvent is used for crystallization, the present inventors presumed that monodentate chain ether solvent tend to have lower coordinating properties to metal, dielectric constant and dipole moments compared with other polar solvents and cyclic ethers, which favors the removal of the solvent from the crystals. In the production method of the present embodiment, by using a monodentate chain ether solvent for crystallization of the fluorine-containing cyclic sulfonylimide salt, it is possible to easily obtain a highly purity fluorine-containing cyclic sulfonylimide salt with reduced amounts of residual fluorine impurities and residual solvent.

The term "monodentate" solvent means a solvent that having one moiety with coordinating ability to metal. Examples of the moiety with coordinating ability include an ether bond.

In the crystallization step, it is preferable to use a single solvent from the viewpoint of production costs and, in the present embodiment. In the present embodiment, since the monodentate coordinating chain ether solvent itself functions effectively as a poor solvent in crystallization, it is entirely possible to obtain high purity imide salts using only a single solvent. However, in the present embodiment, a plurality of solvents can be mixed, and crystallization can be promoted by adding a polar solvent as a good solvent or by further adding a poor solvent. However, when a plurality of solvents are mixed, the amount of the residual solvent after drying tends to increase, which may be disadvantageous in terms of heat history and production costs. When a polar solvent, which is a good solvent, is added, the resulting crystals may adhere to the inside of a crystallizer, raising concerns such as a decrease in recovery rate and an increase in cost due to the introduction of special crushing equipment.

From the viewpoint of reducing the amount of residual solvent after drying, the monodentate chain ether solvent is preferably a solvent has a low boiling point, i.e. a solvent having 6 or less carbon atoms, and examples thereof include ethyl ether, propyl methyl ether, propyl ethyl ether, propyl ether, isopropyl methyl ether, isopropyl ethyl ether, isopropyl ether, butyl methyl ether, butyl ethyl ether, isobutyl methyl ether, isobutyl ethyl ether, sec-butyl methyl ether, sec-butyl ethyl ether, tert-butyl methyl ether, tert-butyl ethyl ether, pentyl methyl ether, isopentyl methyl ether, sec-pentyl methyl ether, tert-pentyl methyl ether, neopentyl methyl ether, cyclopentyl methyl ether and the like. From the viewpoint of the solubility, more preferred are propyl methyl ether, propyl ethyl ether, isopropyl methyl ether, isopropyl ethyl ether, butyl methyl ether, butyl ethyl ether, isobutyl methyl ether, isobutyl ethyl ether, sec-butyl methyl ether, sec-butyl ethyl ether, tert-butyl methyl ether, tert-butyl ethyl ether, pentyl methyl ether, isopentyl methyl ether, sec-pentyl methyl ether, tert-pentyl methyl ether, neopentyl methyl ether and cyclopentyl methyl ether, and still more preferred are butyl methyl ether, butyl ethyl ether, tert-butyl methyl ether, tert-butyl ethyl ether, tert-pentyl methyl ether and cyclopentyl methyl ether.

When the polar solvent is added as a good solvent, the good solvent used is not particularly limited, and from the viewpoint of its particularly preferably dissolution of the fluorine-containing cyclic sulfonylimide salt and relatively low boiling point, examples thereof include acetone, tetrahydrofuran, dimethyl carbonate, diethyl carbonate, ethylene carbonate, propylene carbonate, acetonitrile, methyl acetate, ethyl acetate, butyl acetate and the like.

When the poor solvent is added especially to promote crystallization, the poor solvent used is not particularly limited, but should not dissolve the fluorine-containing cyclic sulfonylimide salt and miscible with the monodentate chain ether solvent, and have a low boiling point, and examples thereof include pentane, hexane, cyclohexane, heptane, chloroform, dichloromethane, 1,2-dichloroethane and the like.

The crystallization step mainly consists of a step of dissolving a crude fluorine-containing cyclic sulfonylimide salt with a monodentate chain ether solvent and a crystallization step, and it is possible to carry out a step of removing

insoluble matters in the mixture immediately before the crystallization step. This removal step can be carried out by any of known methods such as centrifugation, gravity filtration, vacuum filtration and pressure filtration. When performing filtration, any known filtering material may be used, as long as it can withstand the use of an organic solvent, and a filter aid may be added. The pore size of the filtering material is preferably 10 μm or less, and more preferably 1 μm or less.

In the step of dissolving the crude fluorine-containing cyclic sulfonylimide salt, the monodentate chain ether solvent is added in the amount of 100 to 1000% by weight, more preferably 100 to 500% by weight, and still more preferably 100 to 300% by weight, relative to the imide salt-containing solid before crystallization. In this step, dissolution may be promoted by stirring with a magnetic stirrer or stirring blades, or shaking, ultrasonic vibration or the like. The temperature during dissolution is not particularly limited, but the dissolution can be carried out at a temperature from 0°C to the boiling point of the solvent plus 50°C. When heating above the boiling point of the solvent, a known reflux apparatus can be used.

The crystallization step can be carried out, for example, by cooling the solution of the crude fluorine-containing cyclic sulfonylimide salt obtained in the dissolution step to a desired temperature. When cooling, it is preferable to cool gently to a desired temperature from the viewpoint of highly purifying the resulting crystals. The cooling temperature is preferably - 100°C to 30°C, more preferably -80°C to 20°C, and still more preferably -50°C to 20°C. During crystallization, seed crystals may be added, as is widely practiced as a known technique, and concentration may be performed by distilling off the solvent before and during cooling. Crystallization can be promoted by adding the poor solvent while performing cooling.

The purified fluorine-containing cyclic sulfonylimide salt can be collected by any known methods such as centrifugation, gravity filtration, vacuum filtration, pressure filtration or the like. Any known filtering material may be used for filtration, as long as it can withstand the use of the organic solvent. The pore size of the filtering material is preferably 10 μm or less, and more preferably 1 μm or less. For high purification, when crystals are collected, the crystals are preferably washed with a monodentate chain ether solvent, an added poor solvent, or a separately prepared fluorine-containing cyclic sulfonylimide salt solvent solution. The liquid used for washing may be separately heated or cooled to a desired temperature before use.

Although it is disadvantageous in terms of production costs, the crystallization step can be repeated plural times as necessary in order to obtain high purity crystals.

From the viewpoint of improving the efficiency of the production step, it is preferable to recover all the liquid portion and washing liquid removed during the collection of the crystals and reuse them in the dissolution step or the crystallization step.

The purified fluorine-containing cyclic sulfonylimide salt obtained in the crystallization step is a high purity imide salt in which impurities, especially fluorine atom-containing impurities mixed during production, are reduced. The purity of the purified fluorine-containing cyclic sulfonylimide salt is preferably 98% by mass or more, and more preferably 99% by mass or more.

It is possible to use, as an indicator of the purity of the compound (1A), the ratio T1/T2 of the sum T1 of the integrated values of the peaks of the fluorine-containing cyclic sulfonylimide (1A) in $^{19}$F-NMR to the sum T2 of the integrated values of the peaks of impurities characteristic of appearing as single or plural doublet peaks within a region of -115 ppm to -125 ppm. According to the study of the inventors, unless special purification is carried out when the compound (1A) is produced in the above step, the ratio T1/T2 of the sum T1 of the integrated values of the peaks of the fluorine-containing cyclic sulfonylimide (1A) in $^{19}$F-NMR to the sum T2 of the integrated values of the peaks of impurities characteristic of appearing as single or plural doublet peaks within a region of -115 ppm to -125 ppm tends to be less than 500, and thus the compound (1A) turns brown in color.

As used herein, examples of impurities characterized by the term "single or plural doublet peaks within a region of -115 ppm to -125 ppm" include, but are not particularly limited to, compounds having a difluoromethyl group, such as H-$CF_2$ -X-Y (wherein X is a linking group having neither hydrogen nor fluorine, Y is a functional group which is not particularly limited).

In the purified fluorine-containing cyclic sulfonylimide salt, the ratio T1/T2 of the sum T1 of the integrated values of the peaks of the fluorine-containing cyclic sulfonylimide (1A) in $^{19}$F-NMR to the sum T2 of the integrated values of the peaks of impurities characteristic of appearing as single or plural doublet peaks within a region of -115 ppm to -125 ppm is preferably 500 or more, more preferably 800 or more, still more preferably 1,000 or more, and yet more preferably 2,000 or more. Most preferably, the impurity peak is not observed at all, but such a case is inefficient from the viewpoint of production costs. When T1/T2 is the above value or more, the resulting fluorine-containing cyclic sulfonylimide salt tends to be highly purified and completely free from coloration. As a result, for example, it is possible to suppress poor appearance when mixed with a resin as an antistatic agent, deterioration of the quality during long-term storage, thus enabling use in a wider applications.

<Drying Step of Fluorine-Containing Cyclic Sulfonylimide Salt (Compound (1A))>

Next, it is preferable to carry out a drying step in order to remove excess solvent in the fluorine-containing cyclic sulfonylimide salt after collection. In the drying step, heat vacuum drying can be carried out as is commonly carried out. The heating temperature is preferably 40°C, and more preferably 60°C. As long as the decomposition of the fluorine-containing cyclic sulfonylimide salt does not proceed, heating may be carried out at a temperature of 100°C or higher, and the upper limit is preferably 200°C taking the thermal stability of the fluorine-containing cyclic sulfonylimide salt into consideration. The pressure during depressurization is preferably 100 hPa or less, more preferably 50 hPa or less, still more preferably 10 hPa or less, and most preferably 1 hPa or less.

When drying, the residual solvent can be sufficiently removed by drying the crystals collected in the above step as they are. For the purpose of more rapid drying, the crystals may be crushed and stirred, which is expected to further reduce thermal history, time and costs. According to the production method of the present embodiment, the crystals thus obtained contains a small amount of the residual solvent, and since adhesion of crystals and adhesion to the container can be suppressed, no special production device is required for the crushing and pulverizing step of the crystals, and it is possible to use stirring blades or mills which are widely used in common.

When drying, the residual solvent can be sufficiently removed even if the crystals collected in the above step are dried as they are. A washing operation with a fluorine-containing solvent may be added to reduce the amount of the residual solvent and to obtain a higher purity fluorine-containing cyclic sulfonylimide salt. The washing operation with a fluorine-containing solvent is presumed to weaken the affinity between the residual solvent and lithium in the fluorine-containing cyclic sulfonylimide salt, and can reduce the amount of the residual solvent in the fluorine-containing cyclic sulfonylimide salt. As a specific washing operation, a sufficient amount of a fluorine-containing solvent is added to the fluorine-containing cyclic sulfonylimide salt after collection, and the mixture is stirred for a certain period of time or left to stand under no-stirring conditions, and after filtration or depressurization operation, the solvent is distilled off and the above drying step is carried out. The washing operation described above may be repeated plural times. Examples of the fluorine-containing solvent include HCFC (hydrochlorofluorocarbon), HFC (hydrofluorocarbon), CFC (chlorofluorocarbon), PFC (perfluorocarbon), PFPE (perfluoropolyether), HFE (hydrofluoroether) and HFO (hydrofluoroolefin). Of these, 1,1,1,2,2,3,4,5,5,5-decafluoropentane, 1,1,1,2,2,3,4,4,5,5,5-undecafluoropentane, 1,1,1,2,2,3,3,4,5,5,5-undecafluoropentane, and HFC (hydrofluorocarbon) represented by perfluoropentane are preferable, and HFC (hydrofluorocarbon) represented by 1,1,1,2,2,3,4,5,5,5-decafluoropentane is most preferable.

The amount of the residual solvent in the fluorine-containing cyclic sulfonylimide salt is preferably 5.0% by weight or less, preferably 100 ppm by weight or more, more preferably 100 ppm by weight or more and 2.0% by weight or less, still more preferably 100 ppm by weight or more and 1.0% by weight or less, and yet more preferably 100 ppm by weight or more and 0.1% by weight or less, based on the results of $^1$H-NMR measurement with the addition of an internal standard substance such as benzotrifluoride. When the amount of the residual solvent is 5.0% by weight or less, it is preferable because the handleability of the fluorine-containing cyclic sulfonylimide salt as a powder tends to be less likely to deteriorate. When the amount of the residual solvent is 100 ppm by weight or more, it is preferable from the viewpoint of production costs because the time for the drying step tends to be shortened and further heating tends to be unnecessary.

<1. Non-Aqueous Electrolyte Solution>

By using, in the non-aqueous electrolyte solution, a fluorine-containing cyclic sulfonylimide salt represented by the following general formula (1):

[Chemical Formula 22]

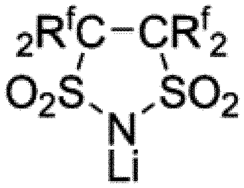

(1)

wherein $R^f$ s may be the same or different and $R^f$ each represents a fluorine atom or a perfluoro group having 4 or less carbon atoms, the initial charging/discharging efficiency and cycle performance are improved. Although the detailed mechanism is unknown, it is presumed that cyclic anions produced by the dissociation of the fluorine-containing cyclic sulfonylimide salt is reductively decomposed at a negative electrode and deposited on the negative electrode to thereby

act as SEI. It is also presumed that the fluorine-containing cyclic sulfonylimide salt has low corrosiveness to metal and suppresses the elution of Al ions from a positive electrode current collector.

By using the fluorine-containing cyclic sulfonylimide salt represented by the formula (1) in a non-aqueous electrolyte solution in which a non-aqueous solvent contains acetonitrile, the initial charging/discharging efficiency and cycle performance are improved because of low corrosiveness to metal. The mechanism is presumed as follows.

The fluorine-containing cyclic sulfonylimide salt represented by the formula (1) dissociates into cations and cyclic anions in the non-aqueous electrolyte solution containing acetonitrile. The cyclic anions electrically interacts with acetonitrile and weakens the metal coordination ability of the nitrile group of acetonitrile. As a result, acetonitrile can suppress the metal elution caused by forming a complex with transition metal in the positive electrode active material at high temperature, and the movement of the metal complex to the negative electrode can be suppressed. Therefore, deterioration of the negative electrode SEI due to reduction deposition of the metal complex on the negative electrode is suppressed, thus leading to an improvement in initial charging/discharging efficiency and cycle performance.

The decomposition product produced by the reductive decomposition of the cyclic anions at the negative electrode deposits on the negative electrode and acts as SEI, and improves the durability of the negative electrode SEI against the reductive deposition of the metal complex at the negative electrode, thus enabling suppression of the reductive decomposition of the solvent at the negative electrode.

In the formula (1), n is an integer of 1 to 4. Specific examples of the fluorine-containing cyclic sulfonylimide salt in which n is 1 to 4 include lithium salts represented by the following compounds (1-1) to (1-5). Of these, the compound (1-2) in which n is 2 is preferable from the viewpoint of easily obtaining an electrolyte solution with high ionic conductivity.

[Chemical Formula 23]

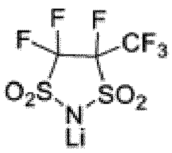

(1-1)

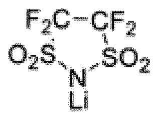

(1-2)

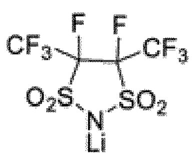

(1-3)

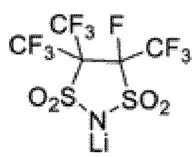

(1-4)

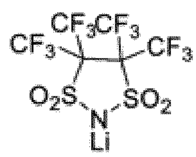

(1-5)

The content of the fluorine-containing cyclic sulfonylimide salt represented by the formula (1) is preferably 0.1 mol or more, more preferably 0.5 mol or more, and still more preferably 0.8 mol or more, as the amount per 1 L of the non-aqueous solvent. When the content is within the above range, the ionic conductivity tends to increase, thus making it possible to exhibit high output characteristics. The content of the fluorine-containing cyclic sulfonylimide salt represented by the formula (1) is preferably less than 3 mol, more preferably 2.5 mol or less, still more preferably 1.8 mol or less, yet more preferably 1.5 mol or less, and further preferably 1.3 mol or less, as the amount per 1 L of the non-aqueous solvent. When the content of the fluorine-containing cyclic sulfonylimide salt represented by the formula (1) is within the above range, there is a tendency that the ionic conductivity of the non-aqueous electrolyte solution increases, thus making it possible to exhibit high output characteristics of the battery and suppressing a decrease in ionic conductivity that accompanies an increase in viscosity of the non-electrolyte solution at low temperature. There is also a tendency that high-temperature cycle characteristics and other battery characteristics can be further improved while maintaining excellent performance of the non-aqueous electrolyte solution.

To improve the volumetric energy density of the non-aqueous secondary battery, when the basis weight of a positive electrode active material layer contained in the positive electrode is adjusted within a range of 24 to 200 $mg/cm^2$, the content of the fluorine-containing cyclic sulfonylimide salt represented by the formula (1) is preferably 1 mol or more, more preferably 1.2 mols or more, still more preferably 1.5 mols or more, and particularly preferably 2 mol or more. When the content of the fluorine-containing cyclic sulfonylimide salt represented by the formula (1) is within the above range, rapid ion conduction is possible even in the deep part of the positive electrode away from the positive electrode-electrolyte solution interface, and thus there is a tendency that the volumetric energy density can be improved while maintaining a balance with the output performance of the battery.

Examples of the method for measuring the content of the fluorine-containing cyclic sulfonylimide salt from the electrolyte solution include a method of combining the measurement of the content of cyclic anions by $^{19}F$-NMR using perfluorobenzene or the like as an internal standard, and the measurement of the content of lithium ions by ion chromatography.

In the non-aqueous electrolyte, since excessive $LiPF_6$ causes deterioration of the battery performance at high temperature, while the fluorine-containing cyclic sulfonylimide salt suppresses deterioration of the battery performance at high temperature by a mechanism presumed to contribute to negative electrode SEI, the molar ratio of the content of

the fluorine-containing cyclic sulfonylimide salt represented by the formula (1) to the content of $LiPF_6$ is preferably 2.5 or more, more preferably more than 10, still more preferably 15 or more, yet preferably 20 or more, and particularly preferably 25 or more. When the molar ratio of the content of the fluorine-containing cyclic sulfonylimide salt to the content of $LiPF_6$ is within the above range, it is possible to effectively suppress deterioration of the battery performance at high temperature such as 50°C or higher, thus obtaining excellent high temperature durability.

The non-aqueous electrolyte solution according to one embodiment of the present invention comprises a non-aqueous solvent and a lithium salt, wherein

the non-aqueous solvent contains a carbonate solvent,
the lithium salt contains a fluorine-containing cyclic sulfonylimide salt represented by the above general formula (1), and contains a fluorine-containing sulfonamide compound represented by the following general formula (2):

$$H(CR_2)_m\text{-}SO_2NHM^2 \qquad (2)$$

wherein Rs may be the same or different and R each is a fluorine atom or a trifluoromethyl group, $M^2$ is Li, Na, K, H or $NH_4$, and m is 1 or 2, in an amount of 1,000 ppm by mass or less relative to the total amount of the non-aqueous electrolyte solution.

The non-aqueous electrolyte solution refers to an electrolyte solution containing 1% by weight or less of water relative to the total amount of the non-aqueous electrolyte solution. The non-aqueous electrolyte solution according to the present embodiment preferably contains as little water as possible, but may contain a very small amount of water, as long as it does not interfere with the solution of the problems of the present invention. The content of such water is 300 ppm by weight or less, and preferably 200 ppm by weight or less, based on the total amount of the non-aqueous electrolyte solution. If the non-aqueous electrolyte has a configuration for achieving the solution of problems of the present invention, the constituent materials in a known non-aqueous electrolyte solution used in lithium ion batteries can be appropriately selected and applied, for other structural elements.

The non-aqueous electrolyte solution of the present embodiment can be produced by mixing a non-aqueous solvent, a lithium salt and a compound (2) and, as necessary, various additives shown below (herein sometimes simply referred to as "additives") by any means. The various additives are a general term of electrode protection additives and other optional additives, and contents thereof are as shown below.

Unless otherwise specified, regarding the content of each compound in the non-aqueous solvent, the mixing ratio is defined by % by volume relative to the total amount of each components constituting the non-aqueous solvent for <2-1. Non-Aqueous Solvent> and <2-3. Additives for Electrode Protection>, the mixing ratio is defined by the number of mols per 1 L of the non-aqueous solvent for <2-2. Lithium Salt>, and the mixing ratio is defined by parts by weight based on 100 parts by weight of the lithium salt and the non-aqueous solvent as a whole for <2-4. Other Optional Additives>.

In the present embodiment, when the electrolyte solution contains a compound other than the compounds specifically shown in each item of <2-1> to <2-4> below, in case where the compound is liquid at room temperature (25°C), it is handled according to the non-aqueous solvent, and the mixing ratio is represented by % by volume relative to the total amount of each component (including the compound) constituting the non-aqueous solvent. Meanwhile, in case where the compound is solid at room temperature (25°C), the mixing ratio is represented by parts by weight based on 100 parts by weight of the lithium salt and the non-aqueous solvent.

<2-1. Non-Aqueous Solvent>

The non-aqueous solvent of the present embodiment includes a carbonate solvent. Examples of the carbonate solvent include cyclic carbonate, fluoroethylene carbonate, chain carbonate, and a compound in which H atoms of the carbonate solvent are partially or entirely substituted with a halogen atom.

Examples of the cyclic carbonate include ethylene carbonate, propylene carbonate, 1,2-butylene carbonate, trans-2,3-butylene carbonate, cis-2,3-butylene carbonate, 1,2-pentylene carbonate, trans-2,3-pentylene carbonate, cis-2,3-pentylene carbonate, vinylene carbonate, 4,5-dimethylvinylene carbonate and vinyl ethylene carbonate;

examples of the fluoroethylene carbonate include 4-fluoro-1,3-dioxolan-2-one, 4,4-difluoro-1,3-dioxolan-2-one, cis-4,5-difluoro-1,3-dioxolan-2-one, trans-4,5-difluoro-1,3-dioxolan-2-one, 4,4,5-trifluoro-1,3-dioxolan-2-one, 4,4,5,5-tetrafluoro-1,3-dioxolan-2-one and 4,4,5-trifluoro-5-methyl-1,3-dioxolan-2-one; and
examples of the chain carbonate include ethyl methyl carbonate, dimethyl carbonate, diethyl carbonate, methyl propyl carbonate, methyl isopropyl carbonate, dipropyl carbonate, methyl butyl carbonate, dibutyl carbonate, ethyl propyl carbonate.

Examples of the fluorinated product of the chain carbonate include methyltrifluoroethyl carbonate, trifluorodimethyl carbonate, trifluorodiethyl carbonate, trifluoroethylmethyl carbonate, methyl 2,2-difluoroethyl carbonate, methyl 2,2,2-trifluoroethyl carbonate, methyl 2,2,3,3-tetrafluoropropyl carbonate and the like. The above fluorinated linear carbonate can be represented by the following general formula:

$$R_{c\,c}\text{-O-C(O)O-}R_{d\,d}$$

wherein $R_{c\,c}$ and $R_{d\,d}$ is at least one selected from the group consisting of $CH_3$, $CH_2\,CH_3$ , $CH_2\,CH_2\,CH_3$ , $CH(CH_3)_2$ and $CH_2\,R^f{}_{e\,e}$ , $R^f{}_{e\,e}$ is an alkyl group having 1 to 3 carbon atoms in which hydrogen atoms are substituted with at least one fluorine atom, and $R_{c\,c}$ and/or $R_{d\,d}$ contain(s) at least one fluorine atom.

The carbonate solvent other than acetonitrile in the present embodiment can be used alone or in combination of two or more thereof.

From the viewpoint of improving the stability of the non-aqueous electrolyte solution, it is preferable to use acetonitrile in combination with at least one of cyclic carbonate and chain carbonate as the non-aqueous solvent in the present embodiment. From this viewpoint, the non-aqueous solvent in the present embodiment is more preferably used in combination with acetonitrile and cyclic carbonate, and more preferably acetonitrile and both cyclic carbonate and chain carbonate.

When cyclic carbonate is used in combination with acetonitrile, it is particularly preferable that such cyclic carbonate includes ethylene carbonate, vinylene carbonate and/or fluoroethylene carbonate.

The term "non-aqueous solvent" as used in the present embodiment refers to an element in which the lithium salt and various additives are removed from the non-aqueous electrolyte solution. When the non-aqueous electrolyte contains electrode protection additives, "non-aqueous solvent" refers to an element in which the lithium salt and additives other than the electrode protection additives are removed from the non-aqueous electrolyte solution. Examples of the non-aqueous solvents include alcohols such as methanol and ethanol; and aprotic solvents. Of these, the non-aqueous solvent is preferably an aprotic solvent. The non-aqueous solvent may contain solvents other than the aprotic solvent, as long as it does not interfere with the solution of the problems of the present invention.

The non-aqueous solvent for the non-aqueous electrolyte solution of the present embodiment may contain acetonitrile as the aprotic solvent. Since the non-aqueous solvent contains acetonitrile, the ionic conductivity of the non-aqueous electrolyte solution is improved, thus making it possible to enhance the diffusivity of lithium ions in the battery. Therefore, when the non-aqueous electrolyte solution contains acetonitrile, in a positive electrode in which the positive electrode active material layer is thickened to increase the filling amount of the positive electrode active material, it becomes possible for lithium ions to satisfactorily reach the region in the vicinity of the current collector where lithium ions hardly reach during high-load discharging. Therefore, it becomes possible to draw out a sufficient capacity even during high-load discharging, thus making it possible to obtain a non-aqueous secondary battery having excellent load characteristics.

When the non-aqueous solvent contains acetonitrile, it is possible to enhance quick charging characteristics of the non-aqueous secondary battery. In constant current (CC)-constant voltage (CV) charging of a non-aqueous secondary battery, the capacity per unit time during the CC charging period is larger than the charge capacity per unit time during the CV charging period. When acetonitrile is used as the non-aqueous solvent of the non-aqueous electrolyte solution, the area capable of CC charging can be increased (CC charging time can be extended) and the charging current can also be increased. Therefore, it is possible to significantly reduce the time required to fully charge the battery from the start of charging the non-aqueous secondary battery.

Acetonitrile easily undergoes electrochemical reductive decomposition. Therefore, when acetonitrile is used, it is preferable to use acetonitrile as a non-aqueous solvent in combination with other solvents (for example, aprotic solvents other than acetonitrile) and/or to add an electrode protection additive for forming a protective film on the electrode.

The content of acetonitrile is preferably 3% by volume or more and 97% by volume or less relative to the total amount of the non-aqueous solvent. The content of acetonitrile is more preferably 5% by volume or more, or 10% by volume or more, or 20% by volume or more, or 30% by volume or more, or 40% by volume or more, or 50% by volume or more, and still more preferably 60% by volume or more, relative to the total amount of the non-aqueous solvent. This value is yet more preferably 97% by volume or less, and further preferably 95% by volume or less. When the content of acetonitrile is 3% by volume or more relative to the total amount of the non-aqueous solvent, the ionic conductivity tends to increase, thus making it possible to exhibit high output characteristics, and also the dissolution of the lithium salt can be promoted. Since the below-described additives suppress an increase in internal resistance of the battery, when the content of acetonitrile in the non-aqueous solvent is within the above range, there is a tendency that high-temperature cycle characteristics and other battery characteristics can be further improved while maintaining excellent performance of the acetonitrile.

Examples of the aprotic solvent other than acetonitrile include lactone, organic compound containing a sulfur atom, cyclic ether, mononitrile other than acetonitrile, alkoxy group-substituted nitrile, dinitrile, cyclic nitrile, short-chain

fatty acid ester, chain ether, fluorinated ether, ketone, and a compound in which H atoms of the aprotic solvent are partially or entirely substituted with a halogen atom.

Examples of the lactone include γ-butyrolactone, α-methyl-γ-butyrolactone, γ-valerolactone, γ-caprolactone, δ-valerolactone, δ-caprolactone and ε-caprolactone;

Examples of the organic compound containing a sulfur atom include ethylene sulfite, propylene sulfite, butylene sulfite, pentene sulfite, sulfolane, 3-sulfolene, 3-methyl sulfolane, 1,3-propane sultone, 1,4-butane sultone, 1-propene 1,3-sultone, dimethyl sulfoxide, tetramethylene sulfoxide and ethylene glycol sulfite;
Examples of the cyclic ether include tetrahydrofuran, 2-methyltetrahydrofuran, 1,4-dioxane and 1,3-dioxane;
Examples of the mononitrile other than acetonitrile include propionitrile, butyronitrile, valeronitrile, benzonitrile and acrylonitrile;
Examples of the alkoxy group-substituted nitrile include methoxyacetonitrile and 3-methoxypropionitrile;
Examples of the dinitrile include malononitrile, succinonitrile, glutaronitrile, adiponitrile, 1,4-dicyanoheptane, 1,5-dicyanopentane, 1,6-dicyanohexane, 1,7-dicyanoheptane, 2,6-dicyanoheptane, 1,8-dicyanooctane, 2,7-dicyanooctane, 1,9-dicyanononane, 2,8-dicyanononane, 1,10-dicyanodecane, 1,6-dicyanodecane and 2,4-dimethylglutaronitrile;
Examples of the cyclic nitrile include benzonitrile;
Examples of the short-chain fatty acid ester include methyl acetate, methyl propionate, methyl isobutyrate, methyl butyrate, methyl isovalerate, methyl valerate, methyl pivalate, methyl hydroangelate, methyl caproate, ethyl acetate, ethyl propionate, ethyl isobutyrate, ethyl butyrate, ethyl isovalerate, ethyl valerate, ethyl pivalate, ethyl hydroangelate, ethyl caproate, propyl acetate, propyl propionate, propyl isobutyrate, propyl butyrate, propyl isovalerate, propyl valerate, propyl pivalate, propyl hydroangelate, propyl caproate, isopropyl acetate, isopropyl propionate, isopropyl isobutyrate, isopropyl butyrate, isopropyl isovalerate, isopropyl valerate, isopropyl pivalate, isopropyl hydroangelate, isopropyl caproate, butyl acetate, butyl propionate, butyl isobutyrate, butyl butyrate, butyl isovalerate, butyl valerate, butyl pivalate, butyl hydroangelate, butyl caproate, isobutyl acetate, isobutyl propionate, isobutyl isobutyrate, isobutyl butyrate, isobutyl isovalerate, isobutyl valerate, isobutyl pivalate, isobutyl hydroangelate, isobutyl caproate, tert-butyl acetate, tert-butyl propionate, tert-butyl isobutyrate, tert-butyl butyrate, tert-butyl isovalerate, tert-butyl valerate, tert-butyl pivalate, tert-butyl hydroangelate and tert-butyl caproate;
Examples of the chain ether include dimethoxyethane, diethyl ether, 1,3-dioxolane, diglime, triglime and tetraglime;
Examples of the fluorinated ether include $Rf_{a\,a}$ -$OR_{b\,b}$, wherein $Rf_{a\,a}$ is an alkyl group containing a fluorine atom, and $R_{b\,b}$ is an organic group optionally containing a fluorine atom;
Examples of the ketone include acetone, methyl ethyl ketone and methyl isobutyl ketone; and
Examples of the compound in which H atoms of the aprotic solvent are partially or entirely substituted with a halogen atom include a compound in which a halogen atom is fluorine.

Examples of the fluorinated product of the short-chain fatty acid ester include fluorinated short-chain fatty acid ester typified by 2,2-difluoroethyl acetate, 2,2,2-trifluoroethyl acetate and 2,2,3,3-tetrafluoropropyl acetate. The fluorinated short-chain fatty acid ester can be represented by the following general formula:

$$R_{f\,f}\text{-}C(O)O\text{-}R_{g\,g}$$

wherein $R_{f\,f}$ is at least one selected from the group consisting of $CH_3$ , $CH_2CH_3$ ,$CH_2CH_2CH_3$ , $CH(CH_3)_2$ , $CF_3CF_2H$, $CFH_2$, $CF_2R^f_{h\,h}$ , $CFHR^f_{h\,h}$ and $CH_2R^f_{i\,i}$, $R_{g\,g}$ is at least one selected from the group consisting of $CH_3$ , $CH_2CH_3$ , $CH_2CH_2CH_3$ , $CH(CH_3)_2$ and $CH_2R^f_{i\,i}$, $R^f_{h\,h}$ is an alkyl group having 1 to 3 carbon atoms in which hydrogen atoms may be substituted with at least one fluorine atom, $R^f_{i\,i}$ is an alkyl group having 1 to 3 carbon atoms in which hydrogen atoms are substituted with at least one fluorine atom, $R_{f\,f}$ and/or $R_{g\,g}$ contain(s) at least one fluorine atom, and when $R_{f\,f}$ is $CF_2H$, $R_{g\,g}$ is not $CH_3$.

The aprotic solvent other than acetonitrile in the present embodiment can be used alone, or two or more thereof may be used in combination.

<2-2. Lithium Salt>

(Fluorine-Containing Cyclic Sulfonylimide Salt (1))

The lithium salt is a fluorine-containing cyclic sulfonylimide salt represented by the general formula (1).

The content of the fluorine-containing cyclic sulfonylimide salt represented by the formula (1) is preferably 0.8 mol or more and 1.5 mols or less relative to 1 L of the non-aqueous solvent.

The fluorine-containing cyclic sulfonylimide salt represented by the formula (1) may be the same as described

in the above item <Composition Containing Fluorine-Containing Cyclic Sulfonylimide Salt (Compound (1A))>.

The fluorine-containing cyclic sulfonylimide salt represented by the formula (1) has a 5-membered ring structure. In a 6-membered ring fluorine-containing cyclic sulfonylimide salt represented by the following general formula (1B):

[Chemical Formula 24]

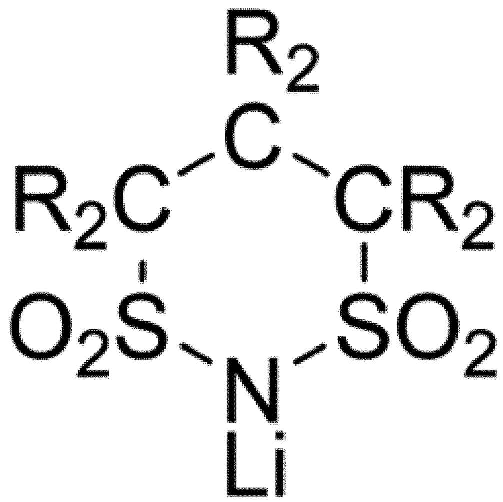

(1B)

wherein Rs may be the same or different and each of Rs represents a fluorine atom or a perfluoro group having 4 or less carbon atoms, because of weak bond between the carbon atom in the $CR_2$ group, which is not adjacent to the sulfonyl group, and the R group, the high-temperature durability of the battery deteriorates by reduction at the negative electrode during charging. A 6- or higher-membered ring fluorine-containing cyclic sulfonylimide salt having has a higher molecular weight of the lithium salt compared with a 5-membered ring fluorine-containing cyclic sulfonylimide salt. Therefore, the weight of the lithium salt required to prepare a non-aqueous electrolyte containing lithium ions at a certain molar concentration increases, and the viscosity of the non-aqueous electrolyte solution tends to increase, leading to deterioration of the output characteristics.

(Method for Producing Fluorine-Containing Cyclic Sulfonylimide Salt (Compound (1))

The method for producing a fluorine-containing cyclic sulfonylimide salt (compound (1)) represented by the formula (1) may be the same as described in the above item <Method for Producing Fluorine-Containing Cyclic Sulfonylimide Salt (Compound (1A)>.

The lithium salt in the present embodiment may further include, in addition to the fluorine-containing cyclic sulfonylimide salt, one or more selected from a lithium-containing imide salt, a fluorine-containing inorganic lithium salt, an organolithium salt and other lithium salts.

The method for measuring the content of the lithium salt in the present embodiment is the same method as the above-described method for measuring a fluorine-containing cyclic sulfonylimide salt.

($LiPF_6$)

The non-aqueous electrolyte solution of the present embodiment may further include a $LiPF_6$ as the lithium salt. The content of $LiPF_6$ in the non-aqueous electrolyte solution of the present embodiment is preferably less than 0.5 mol, more preferably less than 0.1 mol, and still more preferably less than 0.01 mol, as the amount per 1 L of the non-aqueous solvent. When the content of $LiPF_6$ is within the above range, it is possible to suppress the generation of an acid component due to the thermal decomposition reaction of $LiPF_6$ , and to minimize an increase in resistance of the negative electrode due to excessive deposition of an inorganic component in the negative electrode SEI.

The content of $LiPF_6$ is 4 or less, preferably 1 or less, and more preferably 0.2 or less, in terms of a molar ratio, relative to the contents of vinylene carbonate and fluoroethylene carbonate. The content of $LiPF_6$ is 0.01 or more, preferably 0.05 or more, and still more preferably 0.1 or more, in terms of a molar ratio, relative to the contents of vinylene carbonate and fluoroethylene carbonate. By adjusting the content of $LiPF_6$ within the above range, a negative electrode SEI having an excellent balance between the organic component derived from vinylene carbonate and fluoroethylene

carbonate and inorganic component derived from $LiPF_6$ is formed, thus making it possible to suppress an increase in the resistance of the negative electrode in a high temperature test at 50°C or higher.

When the ratio of the organic component derived from vinylene carbonate and fluoroethylene carbonate in the negative electrode SEI is significantly high compared to the ratio of the inorganic component derived from $LiPF_6$, the internal resistance of the non-aqueous secondary battery tends to increase in the high temperature durability test. Although the details are unknown, it is presumed that the negative electrode SEI mainly composed of the organic component derived from vinylene carbonate and fluoroethylene carbonate has low strength, and the reductive decomposition of the solvent in the negative electrode cannot be sufficiently suppressed and the decomposition products accumulate on the negative electrode, leading to an increase in internal resistance. At this time, since the reductive decomposition of the solvent occurs without consuming lithium ions, the reversible capacity of the battery is kept high, but the output performance tends to significantly deteriorate because of high internal resistance of the battery.

Also, when the ratio of the organic component derived from vinylene carbonate and fluoroethylene carbonate in the negative electrode SEI is extremely low compared to the ratio of the inorganic component derived from $LiPF_6$ , the internal resistance of the non-aqueous secondary battery increases in the high temperature durability test. Although the details are unknown, it is presumed that the negative electrode SEI, which is mainly composed of an inorganic component derived from $LiPF_6$ , has high insulation properties and inhibits lithium ion conduction at the negative electrode-electrolyte solution interface.

(Lithium-Containing Imide Salt)

Specifically, the imide salt preferably contains at least one of $LiN(SO_2F)_2$ and

$LiN(SO_2CF_3)_2$.

When the non-aqueous solvent contains acetonitrile, the saturation concentration of the lithium-containing imide salt with respect to acetonitrile is higher than that of $LiPF_6$ , so that the lithium salt preferably contains the lithium-containing imide salt at a molar concentration satisfying: $LiPF_6 \leq$ lithium-containing imide salt because the association and precipitation of the lithium salt and the acetonitrile can be suppressed at low temperature. From the viewpoint of securing the amount of ions supplied to the non-aqueous electrolyte solution according to the present embodiment, the content of the lithium-containing imide salt is preferably 0.5 mol or more and 3.0 mol or less as the amount per 1 L of the non-aqueous solvent.

According to the acetonitrile-containing non-aqueous electrolyte solution containing at least one of $LiN(SO_2F)_2$ and $LiN(SO_2CF_3)_2$, a reduction in ionic conductivity in a low temperature range such as -10°C or -30°C can be effectively suppressed, and excellent low-temperature characteristics can be obtained. In the present embodiment, by adjusting the content of the lithium-containing imide salt, it is possible to more effectively suppress an increase in resistance during high-temperature heating.

(Fluorine-Containing Inorganic Lithium Salt)

The lithium salt used in the non-aqueous electrolyte solution of the present embodiment may contain a fluorine-containing inorganic lithium salt other than $LiPF_6$ , and may contain, for example, fluorine-containing inorganic lithium salts such as $LiBF_4$, $LiAsF_6$, $Li_2SiF_6$, $LiSbF_6$ and $Li_2 B_{12} F_b H_{12-b}$, wherein b is an integer of 0 to 3. Here, "inorganic lithium salt" refers to a lithium salt which does not contain a carbon atom in anions, but contains a fluorine atom in anions and is soluble in acetonitrile. "Fluorine-containing inorganic lithium salt" refers to a lithium salt which does not contain a carbon atom in anions, but contains a fluorine atom in anions and is soluble in acetonitrile. The fluorine-containing inorganic lithium salt is excellent in that it forms a passive film on a surface of an aluminum foil, which is a positive electrode current collector, and suppresses corrosion of the positive electrode current collector. These fluorine-containing inorganic lithium salts are used alone or in combination of two or more thereof. The fluorine-containing inorganic lithium salt is preferably a compound which is a double salt of LiF and Lewis acid is preferable, and of these, a fluorine-containing inorganic lithium salt containing a phosphorus atom is more preferable because it facilitates the release of free fluorine atoms. When a fluorine-containing inorganic lithium salt containing a boron atom is used as the fluorine-containing inorganic lithium salt, it is preferable because it is easy to capture an excess free acid component which may cause deterioration of the battery, and from such a point of view, $LiBF_4$ is preferable.

The content of the fluorine-containing inorganic lithium salt in the lithium salt used in the non-aqueous electrolyte solution according to the present embodiment is preferably 0.01 mol or more, more preferably 0.1 mol or more, and still more preferably 0.25 mol or more, relative to 1 L of the non-aqueous solvent. When the content of the fluorine-containing inorganic lithium salt is within the above range, the ionic conductivity tends to increase and high output characteristics tend to be exhibited. The content is preferably less than 2.8 mol, more preferably less than 1.5 mol, and still more

preferably less than 1 mol, relative to 1 L of the non-aqueous solvent. When the content of the fluorine-containing inorganic lithium salt is within the above range, the ionic conductivity tends to increase and high output characteristics can be exhibited, and deterioration of the ionic conductivity due to an increase in viscosity at low temperature tends to be suppressed. Moreover, the high-temperature cycle characteristics and other battery characteristics tend to be further improved while maintaining excellent performance of the non-aqueous electrolyte solution.

(Organolithium Salt)

The lithium salt in the present embodiment may contain an organolithium salt. The "organolithium salt" refers to a lithium salt other than an imide salt, which contains a carbon atom as anions and is soluble in acetonitrile.

Examples of the organolithium salt include an organolithium salt having an oxalic acid group. Specific examples of the organolithium salt having an oxalate group include organolithium salts represented by $LiB(C_2O_4)_2$, $LiBF_2(C_2O_4)$, $LiPF_4(C_2O_4)$ and $LiPF_2(C_2O_4)_2$, respectively. Of these, at least one lithium salt selected from the lithium salts represented by $LiB(C_2O_4)_2$ and $LiBF_2(C_2O_4)$ is preferable. It is more preferable to use one or more of these salts together with a fluorine-containing inorganic lithium salt. The organolithium salt having an oxalic acid group may be added to the non-aqueous electrolyte solution or contained in a negative electrode (negative electrode active material layer).

The amount of the organolithium salt added to the non-aqueous electrolyte solution in the present embodiment is preferably 0.005 mol or more, more preferably 0.01 mol or more, still more preferably 0.02 mol or more, and particularly preferably 0.05 mol or more, as the amount per 1 L of the non-aqueous solvent, from the viewpoint of ensuring better effects due to its use. However, if the amount of the organolithium salt having an oxalic acid group in the non-aqueous electrolyte solution is too large, it may precipitate. Therefore, the amount of the organolithium salt having an oxalic acid group added to the non-aqueous electrolyte solution is preferably less than 1.0 mol, more preferably less than 0.5 mol, and still more preferably less than 0.2 mol, as the amount per 1 L of the non-aqueous solvent.

The organolithium salt having an oxalic acid group is known to be hardly insoluble in organic solvents having low polarity, especially chain carbonates. The content of the organolithium salt in the non-aqueous electrolyte solution according to the present embodiment may be, for example, 0.01 mol or more and 0.5 mol or less as the amount per 1 L of the non-aqueous solvent.

The organolithium salt having an oxalic acid group sometimes contain a trace amount of lithium oxalate, and sometimes reacts with a trace amount of moisture contained in other starting materials when mixed as a non-aqueous electrolyte solution, thus generating a new white precipitate of lithium oxalate. Therefore, the content of lithium oxalate in the non-aqueous electrolyte solution according to the present embodiment is preferably suppressed within a range of 500 ppm or less.

(Other Lithium Salts)

The lithium salt in the present embodiment may contain other lithium salts, in addition to the above lithium salts.

Specific examples of the other lithium salts include:

inorganic lithium salts containing no fluorine atom in anions, such as $LiClO_4$, $LiAlO_4$, $LiAlCl_4$, $LiB_{10}Cl_{10}$ and chloroborane Li;

organolithium salts such as $LiCF_3SO_3$, $LiCF_3CO_2$, $Li_2C_2F_4(SO_3)_2$, $LiC(CF_3SO_2)_3$, $LiC_nF_{(2n+1)}SO_3$, wherein $n \geq 2$, lower aliphatic carboxylic acid Li, tetraphenylboric acid Li and $LiB(C_3O_4H_2)_2$;

organolithium salts represented by $LiPF_n(C_pF_{2p+1})_{6-n}$, wherein n is an integer of 1 to 5, and p is an integer of 1 to 8, such as $LiPF_5(CF_3)$;

organolithium salts represented by $LiBF_q(C_sF_{2s+1})_{4-}$ q, wherein q is an integer of 1 to 3, and s is an integer of 1 to 8, such as $LiBF_3(CF_3)$;

lithium salts bonded to polyvalent anions;

organolithium salts represented by:

the following formula (XXa):

$$LiC(SO_2R_{jj})(SO_2R_{kk})(SO_2R_{ll}) \quad \text{(XXa)}$$

wherein $R_{jj}$, $R_{kk}$, and $R_{ll}$ may be the same or different and represent a perfluoroalkyl group having 1 to 8 carbon atoms,

the following formula (XXb):

$$LiN(SO_2\,OR_{m\,m})(SO_2\,OR_{n\,n}) \qquad (XXb)$$

wherein $R_{m\,m}$ and $R_{n\,n}$ may be the same or different and represent a perfluoroalkyl group having 1 to 8 carbon atoms, and
the following formula (XXc):

$$LiN(SO_2\,R_{o\,o})(SO_2\,OR_{p\,p}) \qquad (XXc)$$

wherein $R_{o\,o}$ and $R_{p\,p}$ may be the same or different and represent a perfluoroalkyl group having 1 to 8 carbon atoms, etc., and one or more of these salts can be used together with the fluorine-containing inorganic lithium salt.

The amount of the other lithium salt added to the non-aqueous electrolyte solution may be appropriately set within a range of 0.01 mol or more and 0.5 mol or less as the amount per 1 L of the non-aqueous solvent.

<2-3. Electrode Protection Additives>

The non-aqueous electrolyte solution according to the present embodiment may contain an additive for protecting the electrode (electrode protection additive). The electrode protection additive may substantially overlap with a substance (i.e., the non-aqueous solvent described above) which serves as a solvent for dissolving the lithium salt. The electrode protection additive is preferably a substance which contributes to an improvement in performance of the non-aqueous electrolyte solution and the non-aqueous secondary battery, but also contains a substance which is not directly involved in the electrochemical reaction.

Specific examples of the electrode protection additive include:

fluoroethylene carbonates typified by 4-fluoro-1,3-dioxolan-2-one, 4,4-difluoro-1,3-dioxolan-2-one, cis-4,5-difluoro-1,3-dioxolan-2-one, trans-4,5-difluoro-1,3-dioxolan-2-one, 4,4,5-trifluoro-1,3-dioxolan-2-one, 4,4,5,5-tetrafluoro-1,3-dioxolan-2-one and 4,4,5-trifluoro-5-methyl-1,3-dioxolan-2-one;
unsaturated bond-containing cyclic carbonates typified by vinylene carbonate, 4,5-dimethylvinylene carbonate and vinylethylene carbonate;
lactones typified by γ-butyrolactone, γ-valerolactone, γ-caprolactone, δ-valerolactone, δ-caprolactone and ε-caprolactone;
cyclic ethers typified by 1,4-dioxane; and
cyclic sulfur compounds typified by ethylene sulfite, propylene sulfite, butylene sulfite, pentene sulfite, sulfolane, 3-sulfolene, 3-methyl sulfolane, 1,3-propane sultone, 1,4-butane sultone, 1-propene 1,3-sultone and tetramethylene sulfoxide. These electrode protection additives are used alone, or in combination of two or more thereof.

The content of the electrode protection additive in the non-aqueous electrolyte solution is preferably 0.1 to 30% by volume, more preferably 0.3 to 15% by volume, still more preferably 0.4 to 8% by volume, and particularly preferably 0.5 to 4% by volume, as the amount per total amount of the non-aqueous solvent.

In the present embodiment, the larger the content of the electrode protection additive, the more deterioration of the non-aqueous electrolyte solution can be suppressed. However, the smaller the content of the electrode protection additive, the higher output characteristics of the non-aqueous secondary battery in a low temperature environment is improved. Therefore, by adjusting the content of the electrode protection additive within the above range, it tends to be possible to exhibit excellent performance based on high ionic conductivity of the electrolyte solution without impairing the basic function as a non-aqueous secondary battery. By preparing a non-aqueous electrolyte solution with such a composition, it tends to be possible to further improve the cycle performance of the non-aqueous secondary battery, high output performance in a low temperature environment, and other battery characteristics.

Acetonitrile easily undergoes electrochemical reductive decomposition. Therefore, the non-aqueous solvent containing acetonitrile preferably contains, as the electrode protection additive for forming a protective film on the negative electrode, one or more cyclic aprotic polar solvents, and more preferably one or more unsaturated bond-containing cyclic carbonates.

The unsaturated bond-containing cyclic carbonate is preferably vinylene carbonate, and the content of vinylene carbonate is preferably 0.1% by volume or more and 4% by volume or less, more preferably 0.2% by volume or more and less than 3% by volume, and still more preferably 0.5% by volume or more and less than 2.5% by volume. As a result, the low-temperature durability can be more effectively improved, thus making it possible to provide a secondary battery having excellent low-temperature performance.

The vinylene carbonate as the electrode protection additive suppresses the reductive decomposition reaction of acetonitrile on a surface of the negative electrode, and meanwhile, excessive film formation causes deterioration of

low-temperature performance. Therefore, by adjusting the amount of vinylene carbonate added within the above range, the interface (film) resistance can be suppressed to a low level, thus making it possible to suppress cycle deterioration at low temperature.

(Acid Anhydride)

The non-aqueous secondary battery according to the present embodiment is stabilized by partially decomposing the non-aqueous electrolyte solution at the time of initial charging to form an SEI on a surface of a negative electrode. To enhance this SEI more effectively, an acid anhydride can be added to the non-aqueous electrolyte solution, battery member or non-aqueous secondary battery. When acetonitrile is contained as a non-aqueous solvent, the strength of the SEI tends to decrease as the temperature rises, but the addition of the acid anhydride promotes the enhancement of the SEI. Therefore, use of the acid anhydride enables effective suppression of an increase in internal resistance over time due to thermal history.

Specific examples of the acid anhydride include chain acid anhydrides typified by acetic anhydride, propionic anhydride and benzoic anhydride; cyclic acid anhydrides typified by malonic anhydride, succinic anhydride, glutaric anhydride, maleic anhydride, phthalic anhydride, 1,2-cyclohexanedicarboxylic anhydride, 2,3-naphthalenedicarboxylic anhydride or naphthalene-1,4,5,8-tetracarboxylic dianhydride; and mixed acid anhydrides having a structure in which different types of acids, such as two different types of carboxylic acids, or carboxylic acid and sulfonic acid undergo dehydration condensation. These acid anhydrides are used alone, or in combination of two or more thereof.

Since it is preferable for the non-aqueous secondary battery according to the present embodiment to enhance an SEI before the reductive decomposition of the non-aqueous solvent, it is preferable to contain, as the acid anhydride, at least one cyclic acid anhydride which acts early at the time of initial charging. Only one type or plural types of these cyclic acid anhydrides may be contained. Alternatively, a cyclic acid anhydride other than these cyclic acid anhydrides may be contained. The cyclic acid anhydride preferably contains at least one of succinic anhydride, maleic anhydride and phthalic anhydride.

According to a non-aqueous electrolyte solution containing at least one of succinic anhydride, maleic anhydride and phthalic anhydride, it is possible to form a strong SEI on a negative electrode, thus suppressing more effectively an increase in resistance during high-temperature heating. In particular, it is preferable to contain succinic anhydride. Thus, it is possible to form a strong SEI on the negative electrode more effectively while suppressing the side reaction.

When the non-aqueous electrolyte solution according to the present embodiment contains an acid anhydride, the content thereof may be preferably within a range of 0.01 part by weight or more and 10 parts by weight or less, more preferably 0.05 part by weight or more and 1 part by weight or less, and still more preferably 0.1 part by weight or more and 0.5 part by weight or less, as the amount per 100 parts by weight of the non-aqueous electrolyte solution.

The acid anhydride is preferably contained in the non-aqueous electrolyte solution. Meanwhile, as long as the acid anhydride can act in a non-aqueous secondary battery, at least one battery member selected from the group consisting of a positive electrode, a negative electrode and a separator may contain the acid anhydride. As a method of containing the acid anhydride in the battery member, for example, the acid anhydride may be contained in the battery member at the time of fabricating the battery member, or the battery member may be impregnated with the acid anhydride by a post-treatment typified by coating, dipping or spray drying on the battery member.

<2-4. Other Optional Additives>

In the present embodiment, for the purpose of improving charging/discharging cycle characteristics, high-temperature storage and safety (for example, prevention of overcharging) of the non-aqueous secondary battery, it is also possible for the non-aqueous electrolyte solution to appropriately contain optional additives (additives other than the acid anhydride and the electrode protection additive).

Examples of the optional additive include a sulfonic acid ester, diphenyl disulfide, cyclohexylbenzene, biphenyl, fluorobenzene, tert-butylbenzene, a phosphoric acid ester [ethyldiethylphosphonoacetate (EDPA); $(C_2H_5O)_2(P{=}O)\text{-}CH_2(C{=}O)OC_2H_5$, tris(trifluoroethyl) phosphate (TFEP); $(CF_3CH_2O)_3P{=}O$, triphenyl phosphate (TPP); $(C_6H_5O)_3P{=}O$, triallyl phosphate; $(CH_2{=}CHCH_2O)_3P{=}O$, triamyl phosphate, trioctyl phosphate, tris(2-butoxyethyl) phosphate, tris(2-ethylhexyl) phosphate, etc.], a nitrogen-containing cyclic compound with no steric hindrance around unshared electron pair [pyridine, 1-methyl-1H-benzotriazole, 1-methylpyrazole, etc.]. In particular, the phosphoric acid ester has the effect of suppressing the side reaction during storage and the effect of improving the impregnability of the non-aqueous electrolyte solution into the separator, and is effective as the optional additive.

When the non-aqueous electrolyte solution according to the present embodiment contains other optional additives, the content thereof is preferably within a range of 0.01% by weight or more and 10% by weight or less, more preferably 0.02% by weight or more and 5% by weight or less, and still more preferably 0.05 to 3% by weight. By adjusting the content of other optional additives within the above range, it tends to be possible to add more satisfactory battery

characteristics without impairing the basic function of the non-aqueous secondary battery.

<2-5. Compound (2)>

The compound (2) is a fluorine-containing sulfonamide compound represented by the general formula (2).

The compound (2) is corrosive to metal, and when using a non-aqueous electrolyte solution containing a large amount of a fluorine-containing sulfonamide compound, a metal foil used as the positive electrode current collector corrodes, and the irreversible capacity during charging/discharging is reduced and the cycle performance deteriorates. In particular, when an Al foil or stainless steel foil is used as the metal foil, it corrodes significantly.

Specifically, for example, when an Al current collector is used as the positive electrode, it is presumed that the Al corrosion reaction (reaction scheme a) at the positive electrode and the Al reductive deposition reaction (reaction scheme b) at the negative electrode are promoted during charging, thus promoting the irreversible reaction in which Al moves from the positive electrode to the negative electrode during charging, leading to an increase in irreversible capacity of the battery. In the long term, it is considered that a large amount of Al deposition at the negative electrode promotes uneven SEI formation or uneven deposition of metallic lithium at the negative electrode, leading to a decrease in battery capacity or micro short circuits and short circuits in the battery.

Reaction schme a: $Al + 3(H(CR_2)_m\text{-}SO_2NHM) \rightarrow [Al(H(CR_2)_m\text{-}SO_2NHM)_3]^{3+} + 3e^-$

Reaction schme b: $[Al(H(CR_2)_m\text{-}SO_2NHM)_3]^{3+} + 3e^- \rightarrow Al + 3(H(CR_2)_m\text{-}SO_2NHM)$

By reducing the content of the compound (2), it is possible to suppress the Al corrosion reaction at the positive electrode during charging (reaction scheme a) and the reductive deposition reaction of Al at the negative electrode (reaction scheme b), leading to reduction in irreversible capacity during charging/discharging and an improvement in cycle performance. These phenomena are newly discovered by the study of the present inventors, and are not described at all in PTLs 1 to 4.

The content of the fluorine-containing sulfonamide compound (2) contained in the non-aqueous electrolyte solution is 1,000 ppm by weight or less, preferably 500 ppm by weight or less, more preferably 300 ppm by weight, still more preferably 200 ppm by weight or less, yet more preferably 150 ppm by weight or less, and further preferably 120 ppm by weight or less, relative to the total amount of the non-aqueous electrolyte solution. By adjusting the content of the fluorine-containing sulfonamide compound within the above range, it is possible to suppress an increase in irreversible capacity and deterioration of the cycle performance during charging/discharging due to the corrosion of the positive electrode Al current collector.

The content of the fluorine-containing sulfonamide compound contained in the non-aqueous electrolyte solution is preferably 0.001 ppm by weight or more, more preferably 0.01 ppm by weight or more, still more preferably 0.1 ppm by weight or more, yet more preferably 1 ppm by weight or more, and further preferably 80 ppm by weight or more, relative to the total amount of the non-aqueous electrolyte solution. By adjusting the content of the fluorine-containing sulfonamide compound within the above range, the decomposition product of the fluorine-containing sulfonamide compound reduced at the negative electrode functions as a negative electrode SEI, thus enabling an improvement in long-term durability of the battery.

<3. Non-Aqueous Secondary Battery>

The non-aqueous electrolyte solution of the present embodiment can be used in a non-aqueous secondary battery.

The non-aqueous secondary battery according to the present embodiment is not particularly limited, but is configured with a positive electrode, a negative electrode, a separator and a non-aqueous electrolyte solution housed in a suitable battery exterior.

Specifically, the non-aqueous secondary battery according to the present embodiment may be a non-aqueous secondary battery 100 shown in FIG. 1 and FIG. 2. Here, FIG. 1 is a plan view schematically illustrating a non-aqueous secondary battery, and FIG. 2 is a cross-sectional view taken along lines A-A of FIG. 1.

The non-aqueous secondary battery 100 shown in FIG. 1 and FIG. 2 is composed of a pouch-type cell. The non-aqueous secondary battery 100 houses a laminated electrode body formed by stacking a positive electrode 150 and a negative electrode 160 via a separator 170 in a space 120 of a battery exterior 110, and a non-aqueous electrolyte solution (not shown). The battery exterior 110 is made of, for example, an aluminum laminated film, and is sealed by heat-sealing upper and lower films at the outer periphery of the space formed by the two aluminum laminated films. The laminated body in which the positive electrode 150, the separator 170 and the negative electrode 160 are stacked in this order is impregnated with the non-aqueous electrolyte solution. However, in FIG. 2, in order to avoid complicating

the drawing, the respective layers constituting the battery exterior 110 and the respective layers of the positive electrode 150 and the negative electrode 160 are not shown separately.

The aluminum laminate film constituting the battery exterior 110 is preferably an aluminum laminate film in which both sides of the aluminum foil are coated with a polyolefin-based resin.

The positive electrode 150 is connected to a positive electrode lead body 130 in the non-aqueous secondary battery 100. Although not shown, the negative electrode 160 is also connected to a negative electrode lead body 140 in the non-aqueous secondary battery 100. One end of each of the positive electrode lead body 130 and the negative electrode lead body 140 is pulled out to the outside of the battery exterior body 110 so that they can be connected to an external device or the like, and their ionomer portions are heat-sealed together with one side of the battery exterior body 110.

In the non-aqueous secondary battery 100 shown in FIG. 1 and FIG. 2, the positive electrode 150 and the negative electrode 160 each have one laminated electrode body, but the number of laminated positive electrodes 150 and 160 can be appropriately increased by the capacity design. In the case of a laminated electrode body having a plurality of positive electrodes 150 and negative electrodes 160, tabs of the same electrode may be joined by welding or the like, and then joined to one lead body by welding or the like and taken out of the battery. As the tab of the same pole, a mode composed of the exposed portion of a current collector, a mode configured by welding a metal piece to the exposed portion of a current collector, and the like are possible.

The positive electrode 150 is composed of a positive electrode current collector and a positive electrode active material layer. The negative electrode 160 is composed of a negative electrode current collector and a negative electrode active material layer.

The positive electrode active material layer contains a positive electrode active material, and the negative electrode active material layer contains a negative electrode active material.

The positive electrode 150 and the negative electrode 160 are disposed so that the positive electrode active material layer and the negative electrode active material layer face each other via the separator 170.

Hereinafter, each element constituting the non-aqueous secondary battery according to the present embodiment will be described in order.

<4. Positive Electrode>

The positive electrode 150 is composed of a positive electrode active material layer made from a positive electrode mixture and a positive electrode current collector. The positive electrode mixture contains a positive electrode active material, and further contain a conductive aid and/or a binder as necessary.

The positive electrode active material layer contains a material capable of occluding and releasing lithium ions as the positive electrode active material. Such material is capable of obtaining high voltage and high energy density.

The positive electrode active material includes, for example, a positive electrode active material containing at least one transition metal element selected from the group consisting of Ni, Mn and Co, and is suitably at least one Li-containing metal oxide selected from lithium (Li)-containing metal oxides represented by the following general formula ($a^t$):

$$Li_pNi_qCo_rMn_sM_tO_u \qquad (a^t)$$

wherein M is at least one metal selected from the group consisting of Al, Sn, In, Fe, V, Cu, Mg, Ti, Zn, Mo, Zr, Sr and Ba, p, q, r, s, t and u are within the following ranges: $0 < p < 1.3$, $0 < q < 1.2$, $0 < r < 1.2$, $0 \leq s < 0.5$, $0 \leq t < 0.3$, $0.7 \leq q+r+s+t \leq 1.2$, and $1.8 < u < 2.2$, and p is the value determined by the charging/discharging state of the battery.

Specific examples of the positive electrode active material include lithium cobalt oxide typified by $LiCoO_2$; lithium manganese oxide typified by $LiMnO_2$, $LiMn_2O_4$ and $Li_2Mn_2O_4$; lithium nickel oxide typified by $LiNiO_2$; and lithium-containing composite metal oxide represented by $Li_zMO_2$, typified by $LiNi_{1/3}Co_{1/3}Mn_{1/3}O_2$, $LiNi_{0.5}Co_{0.2}Mn_{0.3}O_2$ and $LiNi_{0.8}Co_{0.2}O_2$ (wherein M represents two or more metal elements selected from the group consisting of Ni, Mn, Al and Mg, and z represents a number of more than 0.9 and less than 1.2).

In particular, when a Ni content ratio q of the Li-containing metal oxide represented by the general formula ($a^t$) satisfies: $0.5 < q < 1.2$, it is preferable because both a reduction in amount of Co, which is rare metal, and higher energy density is achieved. Examples of the positive electrode active material include lithium-containing composite metal oxide typified by $LiNi_{0.6}Co_{0.2}Mn_{0.2}O_2$, $LiNi_{0.75}Co_{0.15}Mn_{0.15}O_2$, $LiNi_{0.8}Co_{0.1}Mn_{0.1}O_2$, $LiNi_{0.85}Co_{0.075}Mn_{0.075}O_2$, $LiNi_{0.8}Co_{0.15}Al_{0.05}O_2$, $LiNi_{0.81}Co_{0.1}Al_{0.09}O_2$, $LiNi_{0.85}Co_{0.1}Al_{0.05}O_2$ and the like.

As the Ni content ratio of the Li-containing metal oxide increases, deterioration tends to progress at a low voltage. The layered rock salt type positive electrode active material represented by the general formula ($a^t$) essentially has the active point which causes oxidative deterioration of the electrolyte solution. This active point sometimes unintentionally consumes the electrode protection additive.

These additive decomposition products taken in and deposited on the positive electrode side not only increase

the internal resistance of the non-aqueous secondary battery, but also accelerate deterioration of the lithium salt. In particular, when $LiPF_6$ is contained as a lithium salt, it is considered that HF is generated by deterioration, leading to acceleration of the elution of the transition metal. In a non-aqueous electrolyte solution containing acetonitrile as a non-aqueous solvent, a complex of the metal cation and acetonitrile is formed, thus accelerating deterioration of the battery.

Furthermore, deterioration of the electrode protection additive or deterioration of the lithium salt makes the original purpose of protecting the surface of the negative electrode insufficient. In particular, in a non-aqueous electrolyte solution containing acetonitrile as a non-aqueous solvent, if the negative electrode surface is not sufficiently protected, the reductive decomposition of acetonitrile proceeds and the battery performance rapidly deteriorates, causing a fatal problem.

In order to deactivate the active point which essentially causes oxidative deterioration of the non-aqueous electrolyte solution, it is important to control the Jahn-Teller strain or to coexist with a component which acts as a neutralizer. Therefore, the positive electrode active material preferably contains at least one metal selected from the group consisting of Al, Sn, In, Fe, V, Cu, Mg, Ti, Zn, Mo, Zr, Sr and Ba.

For the same reason, it is preferable that the surface of the positive electrode active material is coated with a compound containing at least one metal element selected from the group consisting of Zr, Ti, Al and Nb. It is more preferable that the surface of the positive electrode active material is coated with an oxide containing at least one metal element selected from the group consisting of Zr, Ti, Al and Nb. It is particularly preferable that the surface of the positive electrode active material is coated with at least one oxide selected from the group consisting of $ZrO_2$, $TiO_2$, $Al_2 O_3$ , NbOs and $LiNbO_2$ because it does not inhibit the permeation of lithium ions.

In the non-aqueous secondary battery according to the present embodiment, it is preferable to use a lithium phosphorus metal oxide having an olivine crystal structure containing iron (Fe) atoms, and it is more preferable to a lithium phosphorus metal oxide having an olivine structure represented by the following formula (Xba):

$$Li_w M^{II}PO_4 \qquad (Xba)$$

wherein $M^{II}$ represents one or more transition metal elements containing at least one transition metal element including Fe, and w is the value determined by the charging/discharging state of the battery and represents the number of 0.05 to 1.10. For the purpose of stabilizing the structure, these lithium-containing compounds may be those in which transition metal elements are partially substituted with Al, Mg or other transition metal elements, those in which these metal elements are included in grain boundaries, those in which oxygen atoms are partially substituted with a fluorine atom or the like, those in which a surface of the positive electrode active material is partially coated with other positive electrode active materials, and the like.

Examples of the positive electrode active material include phosphate metal oxides containing lithium and a transition metal element, and silicate metal oxides containing lithium and a transition metal element. From the viewpoint of obtaining a higher voltage, the lithium-containing metal oxide is particularly preferably phosphate metal oxide containing lithium and at least one transition selected from the group consisting of Co, Ni, Mn, Fe, Cu, Zn, Cr, V and Ti, and is more preferably phosphate metal oxide containing Li and Fe from the viewpoint of the lithium phosphorus metal oxide represented by the above formula (Xba).

It is possible to use, as the lithium phosphorus metal oxide different from the lithium phosphorus metal oxide represented by the above formula (Xba), a compound represented by the following formula (Xa):

$$Li_vM^ID_2 \qquad (Xa)$$

wherein D represents a chalcogen element and $M^I$ represents one or more transition metal elements containing at least one transition metal element, and the value of v is the value determined by the charging/discharging state of the battery, and represents the number of 0.05 to 1.10.

As the positive electrode active material in the present embodiment, only the lithium-containing metal oxide as described above may be used, or other positive electrode active materials may be used together with the lithium-containing metal oxide. Examples of other positive electrode active materials include metal oxides or metal chalcogenides having a tunnel structure and a layered structure; sulfur; conductive polymers, and the like. Examples of the metal oxides or metal chalcogenide having a tunnel structure and a layered structure include oxides of metals other than lithium, typified by $MnO_2$, $FeO_2$, $FeS_2$, $V_2 O_5$, $V_6 O_{13}$, $TiO_2$, $TiS_2$, $MoS_2$ and $NbSe_2$ ; sulfides; selenides, and the like. Examples of the conductive polymer include conductive polymers typified by polyaniline, polythiophene, polyacetylene and polypyrrole.

The above-described other positive electrode active materials may be used alone, or in combination of two or more thereof. Since lithium ions can be occluded and released in a reversible and stable manner, and high energy density can be achieved, it is preferable that the positive electrode active material layer contains at least one transition metal element selected from Ni, Mn and Co.

When a lithium-containing metal compound and other positive electrode active materials are used in combination as the positive electrode active material, a ratio of both used is preferably 80% by weight or more, and more preferably 85% by weight, as the ratio of the lithium-containing metal compound used to the entire positive electrode active material used.

The positive electrode active material layer is formed by coating a positive electrode mixture-containing slurry, which is prepared by dispersing a positive electrode mixture obtained by mixing a positive electrode active material, and a conductive aid and a binder as necessary, in a solvent, onto a positive electrode current collector, followed by drying (removing the solvent) and pressing as necessary. It is possible to use, as such a solvent, a known solvent. Examples thereof include N-methyl-2-pyrrolidone, dimethylformamide, dimethylacetamide, water and the like.

Examples of the conductive aid include graphite; carbon black typified by acetylene black and Ketjen black; and carbon fibers. The content of the conductive aid is preferably set at 1 part by mass to 20 parts by mass, more preferably 2 parts by mass to 15 parts by mass, as the amount per 100 parts by mass of the positive electrode active material.

If the content of the conductive aid is too large, the volumetric energy density decreases, but if it is too small, electron conduction paths are insufficiently formed. In particular, since the positive electrode active material layer has lower electron conductivity compared to the negative electrode active material layer, if the amount of the conductive aid is insufficient, it becomes impossible to take out a predetermined battery capacity when the non-aqueous secondary battery is discharged or charged at a high current value. Therefore, in applications that require high output and/or rapid charging, it is preferable to increase the content of the conductive aid as long as the reaction based on electron transfer is not limited.

Examples of the binder include polyvinylidene fluoride (PVDF), polytetrafluoroethylene (PTFE), polyacrylic acid, carboxymethylcellulose, styrene-butadiene rubber and fluororubber. The content of the binder is preferably set at 6 parts by weight or less, and more preferably 0.5 to 4 parts by weight, relative to 100 parts by weight of the positive electrode active material.

The positive electrode current collector is composed of, for example, a metal foil such as an aluminum foil, a nickel foil or a stainless steel foil. Further, the positive electrode current collector may be carbon-coated on a surface thereof or may be processed into a mesh shape. The thickness of the positive electrode current collector is preferably 5 to 40 μm, more preferably 7 to 35 μm, and still more preferably 9 to 30 μm.

The basis weight per side of the positive electrode excluding the positive electrode current collector is preferably 15 mg/cm$^2$ or more, more preferably 17.5 mg/cm$^2$ or more, and still more preferably 24 mg/cm$^2$ or more, from the viewpoint of improving the volumetric energy density of the non-aqueous secondary battery. The basis weight per one side of the positive electrode excluding the positive electrode current collector is preferably 200 mg/cm$^2$ or less, more preferably 100 mg/cm$^2$ or less, and still more preferably 60 mg/cm$^2$ or less. By limiting the basis weight per side of the positive electrode excluding the positive electrode current collector within the above range, a non-aqueous secondary battery capable of realizing high output performance can be provided even when an electrode active material layer with a high volumetric energy density is designed.

Here, when the electrode active material layer is formed on one side of the current collector, the basis weight indicates the weight of the electrode active material contained per 1 cm$^2$ of the electrode area, and when the electrode active material layer is formed on both sides of the current collector, the basis weight indicates the mass of the electrode active material contained per 1 cm$^2$ of electrode area on each side. When a large amount of the electrode active material is applied to the electrode current collector, the amount of electrode active material per unit volume of the battery becomes relatively larger than that of other battery materials that are not related to battery capacity, such as current collector foils and separators, so that the battery has a higher capacity.

The basis weight when the electrode active material layer is formed on one side of the current collector can be calculated by the following formula (12).

$$\text{Basis weight [mg/cm}^2\text{]} = (\text{electrode weight [mg]} - \text{electrode current collector weight [mg]})/\text{electrode area [cm}^2\text{]} \qquad (12)$$

<5. Negative Electrode>

The negative electrode 160 is composed of a negative electrode active material layer made from a negative electrode mixture, and a negative electrode current collector. The negative electrode 160 can act as a negative electrode of a non-aqueous secondary battery.

The negative electrode active material layer contains a negative electrode active material, and may further contain a conductive aid and/or a binder as necessary.

It is possible to use, as the negative electrode active material, include amorphous carbon (hard carbon, soft carbon), graphite (artificial graphite, natural graphite), thermally decomposed carbon, coke, glassy carbon, calcined product of organic polymer compound, mesocarbon microbeads, carbon materials typified by carbon fiber, activated carbon, graphite, carbon colloid and carbon black, metallic lithium, metal oxides, metal nitrides, lithium alloys, tin alloys, silicon alloys, intermetallic compounds, organic compounds, inorganic compounds, metal complexes and organic polymer compounds. The negative electrode active materials may be used alone, or in combination of two or more thereof.

It is preferable to use, as the negative electrode active material of the present embodiment, a compound containing graphite or one or more elements selected from the group consisting of Ti, V, Sn, Cr, Mn, Fe, Co, Ni, Zn, Al, Si and B.

It is preferable that the negative electrode active material layer contains, as the negative electrode active material, a material capable of occluding lithium ions at a lower potential than 0.4V vs. $Li/Li^+$ from the viewpoint of increasing the battery voltage.

The negative electrode active material layer is formed by applying a negative electrode mixture-containing slurry, which is prepared by dispersing a negative electrode mixture of a negative electrode active material and, as necessary, a conductive aid and a binder in a solvent, to a negative electrode current collector and drying (removing a solvent), followed by pressing as necessary. Known solvents can be used as such solvents, and examples thereof include N-methyl-2-pyrrolidone, dimethylformamide, dimethylacetamide, water and the like.

Examples of the conductive aid include carbon black typified by acetylene black and Ketjen black, and carbon fibers. The content of the conductive aid is preferably set at 20 parts by weight or less, and more preferably 0.1 to 10 parts by weight, relative to 100 parts by weight of the negative electrode active material.

Examples of the binder include carboxymethyl cellulose, PVDF, PTFE, polyacrylic acid and fluororubber. A diene-based rubber such as a styrene-butadiene rubber can also be exemplified. The content of the binder is preferably set at 10 parts by weight or less, and more preferably 0.5 to 6 parts by weight, relative to 100 parts by weight of the negative electrode active material.

The negative electrode current collector is made of, for example, a metal foil such as a copper foil, a nickel foil or a stainless steel foil. Further, the negative electrode current collector may be carbon-coated on a surface thereof or may be processed into a mesh shape. The thickness of the negative electrode current collector is preferably 5 to 40 μm, more preferably 6 to 35 μm, and still more preferably 7 to 30 μm.

<6. Separator>

The non-aqueous secondary battery 100 in the present embodiment preferably includes a separator 170 between a positive electrode 150 and a negative electrode 160 from the viewpoint of preventing short circuits between the positive electrode 150 and the negative electrode 160 and imparting the safety such as shutdown. As the separator 170, an insulating thin film having high ion permeability and excellent mechanical strength is preferable. Examples of the separator 170 include a woven fabric, a nonwoven fabric, a synthetic resin microporous membrane and the like, and of these, the synthetic resin microporous membrane is preferable.

It is possible to preferably use, as the synthetic resin microporous membrane, for example, a microporous membrane containing polyethylene or polypropylene as a main component, or a polyolefin-based microporous membrane such as a microporous membrane containing both of these polyolefins. Examples of the nonwoven fabric include porous membranes made of heat-resistant resins such as glass, ceramic, polyolefin, polyester, polyamide, liquid crystal polyester and aramid.

The separator 170 may have a configuration in which one type of a microporous membrane is laminated in a single layer or a plurality of layers, or a configuration in which two or more types of microporous membranes are laminated. The separator 170 may have a configuration in which a mixed resin material prepared by melt-kneading two or more types of resin materials is used to form a single layer or a plurality of layers.

For the purpose of imparting the functionality, inorganic particles may be present on the surface layer or inside the separator, and other organic layers may be further coated or laminated. Moreover, the separator may include a crosslinked structure. These techniques may be combined as necessary to enhance the safety performance of the non-aqueous secondary battery.

By using such a separator 170, it is possible to realize satisfactory input/output characteristics and low self-discharge characteristics, which are particularly required for lithium ion batteries for high power applications. The thickness of the separator is preferably 1 μm or more from the viewpoint of the separator strength, preferably 500 μm or less, more preferably 5 μm or more and 30 μm or less, and still more preferably 10 μm or more and 25 μm or less, from the viewpoint of the permeability, The thickness of the separator is more preferably 15 μm or more and 20 μm or less when emphasis is placed on the short-circuit resistance, but when emphasis is placed on the high energy density, it is more preferably 10 μm or more and less than 15 μm. The porosity of the separator is preferably 30% or more and 90% or less, more preferably 35% or more and 80% or less, and still more preferably 40% or more and 70% or less, from the

viewpoint of following the rapid movement of lithium ions at high output. When priority is given to improving the output performance while ensuring the safety, the porosity is particularly preferably 50% or more and 70% or less is, and when emphasizing both short-circuit resistance and output performance, the porosity is particularly preferably 40% or more and less than 50%. The air permeability of the separator is preferably 1 second/100 $cm^3$ or more and 400 seconds/100 $cm^3$ or less, more preferably 100 seconds/100 $cm^3$ or more and 350 seconds/100 $cm^3$ or less, from the viewpoint of a balance between the separator thickness and porosity. When emphasizing both short-circuit resistance and output performance, the air permeability is particularly preferably 150 seconds/100 $cm^3$ or more and 350 seconds/100 $cm^3$ or less, and when priority is given to improving the output performance while ensuring the safety, the air permeability is particularly preferably 100 seconds/100 $cm^3$ or more and less than 150 seconds/100 $cm^3$ . Meanwhile, when a non-aqueous electrolyte solution with low ionic conductivity is combined with a separator within the above range, the migration rate of lithium ions is not controlled by the structure of the separator, but controlled by high ionic conductivity of the electrolyte solution, thus failing to obtain the respected input/output characteristics. Therefore, the ionic conductivity of the non-aqueous electrolyte solution at 25°C is preferably 10 mS/cm or more, more preferably 15 mS/cm or more, and still more preferably 20 mS/cm or more. However, the thickness, air permeability and porosity of the separator, and the ionic conductivity of the non-aqueous electrolyte solution are not limited to the above examples.

<7. Battery Exterior>

It is possible to employ, as a configuration of a battery exterior 110 of the non-aqueous secondary battery 100 in the present embodiment, for example, a battery exterior of a battery can (not shown) or a laminated film exterior. It is possible to use, as the battery can, for example, a rectangular, prismatic, cylindrical, elliptical, flat, coin-shaped or button-shaped metal can made of steel, stainless steel (SUS), aluminum or a clad material. It is possible to use, as the laminated film exterior, for example, a laminated film having a three-layer structure of hot-melt resin/metal film/resin.

The laminated film exterior can be used as an exterior in a state where two sheets are stacked with the hot melt resin side facing inward, or bent so that the heat-melting resin side faces inward, and then the end is sealed by heat sealing. When the laminated film exterior is used, a positive electrode lead body 130 (or a lead tab connected to a positive electrode terminal and a positive electrode terminal) may be connected to a positive electrode current collector, and a negative electrode lead body 140 (or a negative electrode terminal and a negative electrode terminal) may be connected to a negative electrode current collector. In this case, the laminated film outer body may be sealed in a state where the ends of the positive electrode lead body 130 and the negative electrode lead body 140 (or lead tabs connected to the positive electrode terminal and the negative electrode terminal respectively) are pulled out to the outside of the battery exterior.

The battery exterior of the non-aqueous secondary battery in the present embodiment preferably contains aluminum in at least a part of the liquid contact layer with the non-aqueous electrolyte solution on the positive electrode side. By using such a battery exterior, corrosion deterioration of the battery exterior can be suppressed to the maximum extent, thus enabling an improvement in cycle characteristics of the battery. The liquid contact layer may be the same material as that of a main body, i.e., a liquid contact layer of the main body, or may be a layer obtained by coating the main body with another material.

Aluminum contained in the liquid contact layer may be present in a part of the liquid contact layer with the non-aqueous electrolyte solution, or may be present in the entire liquid contact layer. It is more preferable that the liquid contact layer is entirely made of aluminum in order to minimize corrosion deterioration of the battery exterior and to maximize an improvement in the cycle characteristics of the battery.

Moreover, the battery exterior of the non-aqueous secondary battery in the present embodiment may be one in which the main body is made of metal other than aluminum, and at least a part of the liquid contact layer with the non-aqueous electrolyte solution contains aluminum. The liquid contact layer containing aluminum can be formed by plating, clad or the like. More preferably, it is composed of an aluminum clad. A clad is a material in which two or more types of different metals are laminated each other, and it is commonly assumed that the interfaces of dissimilar metals are diffusion-bonded (has an alloy layer). The clad can be fabricated by pressing or explosive welding.

In the case of a coin-type non-aqueous secondary battery, the battery exterior preferably contains aluminum in at least a part of the liquid contact layer at the positive electrode side, and more preferably aluminum is cladded on at least a part of the liquid contact layer at the positive electrode side.

When the body metal layer of the battery exterior is made of metal other than aluminum, the body metal is not particularly limited, but is preferably austenitic stainless steels such as SUS304, SUS316 and SUS316L; austenitic-ferritic stainless steels such as SUS329J1 and SUS329J3L; austenitic-ferritic stainless steels such as SUS405 and SUS430; and martensitic stainless steels such as SUS403 and SUS410, from the viewpoint of preventing corrosion on the outside of a container.

<8. Method for Fabricating Battery>

The non-aqueous secondary battery 100 in the present embodiment is fabricated by a known method using the non-aqueous electrolyte solution described above, a positive electrode 150 having a positive electrode active material layer on one or both sides of a current collector, a negative electrode 160 having a negative electrode active material layer on one or both sides of a current collector, and a battery exterior 110, and a separator 170 as necessary.

First, a laminated body composed of a positive electrode 150, a negative electrode 160 and a separator 170 as necessary is formed. For example, it is possible to employ: a mode in which a long positive electrode 150 and negative electrode 160 are wound in a laminated state where a long separator is interposed into the space between the positive electrode 150 and the negative electrode 160 to form a laminated body having a wound structure; a mode in which a positive electrode sheet and a negative electrode sheet obtained by cutting the positive electrode 150 and the negative electrode 160 into a plurality of sheets having the same area and shape are alternately stacked via a separator sheet to form a laminated body; and a mode in which a long separator is folded into a spiral, and a cathode sheet and an anode sheet are alternately inserted into the space between the spiral separators to form a laminated body having a laminated structure.

Next, the above-described laminated body is housed in the battery exterior 110 (battery case) and the electrolyte solution according to the present embodiment is injected into the battery case, and then the laminated body is immersed in the electrolyte solution, followed by sealing, thus enabling the fabrication of the non-aqueous secondary battery in the embodiment. Alternatively, a non-aqueous secondary battery 100 can also be fabricated by impregnating a substrate made of a polymer material with the electrolyte solution to fabricate an electrolyte membrane in a gel state in advance, forming a laminated body having a laminated structure using a sheet-shaped positive electrode 150 and negative electrode 160, an electrolyte film, and a separator 170 as necessary, and housing the laminated body in a battery exterior 110.

It should be noted that the arrangement of the electrodes is designed such that there is a portion where the outer peripheral edge of the negative electrode active material layer and the outer peripheral edge of the positive electrode active material layer overlap, or there is a portion having too small width in the non-opposing portion of the negative electrode active material layer, electrode misalignment occurs during battery assembling. As a result, charging/discharging cycle characteristics of the non-aqueous secondary battery may deteriorate. Therefore, it is preferable to fix the position of the electrode body used for the non-aqueous secondary battery in advance with tapes such as a polyimide tape, a polyphenylene sulfide tape, a polypropylene (PP) tape, an adhesive and the like.

In a non-aqueous electrolyte solution containing acetonitrile, due to its high ionic conductivity of acetonitrile, lithium ions released from the positive electrode during the initial charging of the non-aqueous secondary battery may diffuse to the entire negative electrode. In the non-aqueous secondary battery, the area of the negative electrode active material layer is commonly larger than that of the positive electrode active material layer. However, if lithium ions are diffused and stored to the portion of the negative electrode active material layer which does not face the positive electrode active material layer, lithium ions are not released during initial discharging and remain in the negative electrode. Therefore, the contribution of the unreleased lithium ions becomes an irreversible capacity. For this reason, the non-aqueous secondary battery using a non-aqueous electrolyte solution containing acetonitrile may exhibit low initial charging/discharging efficiency.

Meanwhile, when the area of the positive electrode active material layer is larger than that of the negative electrode active material layer, or both are the same, current is likely to be concentrated at the edge portion of the negative electrode active material layer during charging, thus making it easier to form lithium dendrite.

A ratio of the area of the entire negative electrode active material layer to the area of the portion where the positive electrode active material layer and the negative electrode active material layer face each other is not particularly limited, but is preferably more than 1.0 and less than 1.1, more preferably more than 1.002 and less than 1.09, still more preferably more than 1.005 and less than 1.08, and particularly preferably more than 1.01 and less than 1.08 for the above reasons. In the non-aqueous secondary battery using a non-aqueous electrolyte solution containing acetonitrile, it is possible to improve the initial charging/discharging efficiency by decreasing the ratio of the area of the entire negative electrode active material layer to the area of the portion where the positive electrode active material layer and the negative electrode active material layer face each other.

Decreasing the ratio of the area of the entire negative electrode active material layer to the area of the portion where the positive electrode active material layer and the negative electrode active material layer face each other means limiting the proportion of the area of the position of the negative electrode active material layer which does not face the positive electrode active material layer. Thus, it becomes possible to minimize the amount of lithium ions stored in the position of the negative electrode active material layer which does not face the positive electrode active material layer (i.e., the amount of lithium ions which are not released from the negative electrode during the initial discharge and become the irreversible capacity) of lithium ions released from the positive electrode during initial charging. Therefore, by designing the ratio of the area of the entire negative electrode active material layer to the area of the position where the positive electrode active material layer and the negative electrode active material layer face each other within the

above range, it is possible to enhance initial charging/discharging efficiency of the battery and also to suppress the formation of lithium dendrite while intendedly improving load characteristics of the battery by using acetonitrile.

The non-aqueous secondary battery 100 in the present embodiment can function as a battery by initial charging, but is stabilized by partially decomposing the electrolyte solution at the time of initial charging. Although there is no particular limitation on the method of the initial charging, initial charging is preferably carried out at 0.001 to 0.3 C, more preferably 0.002 to 0.25 C, and still more preferably 0.003 to 0.2 C It is also possible to give preferable results by carrying out initial charging via constant voltage charging. By setting a long voltage range in which the lithium salt is involved in the electrochemical reaction, a stable and strong negative electrode SEI is formed on a surface of the electrode, which suppresses an increase in internal resistance, and exerts satisfactory effect on members other than the negative electrode 160, such as the positive electrode 150 and the separator 170 without causing firm fixation of the reaction product to only the negative electrode 160. Therefore, it is remarkably effective to carry out initial charging in consideration of the electrochemical reaction of the lithium salt dissolved in the non-aqueous electrolyte solution.

The non-aqueous secondary battery 100 in the present embodiment can also be used as a battery pack in which a plurality of non-aqueous secondary batteries 100 are connected in series or in parallel. From the viewpoint of controlling the charge/discharge state of the battery pack, when using the positive electrode active material represented by the above formula (Xba) used in the positive electrode, the working voltage range per one battery is preferably 1.5 to 4.0 V, and particularly preferably 2.0 to 3.8V.

When using the positive electrode active material represented by the general formula ($a^t$), the working voltage range per one battery is preferably 2 to 5 V, more preferably 2.5 to 5 V, and particularly preferably 2.75 V to 5 V.

[EXAMPLES]

Examples that specifically describe the present embodiment will be illustrated below. The present invention is not limited to the following Examples unless it departs from the scope thereof.

<I. Heat-Resistant Properties Test of Fluorine-Containing Cyclic Sulfonylimide Salt (Compound (1A)) Composition>

<Analytical Method>

Analytical methods, starting materials, reaction conditions and the like used in Examples and Comparative Examples were as follows.

(Nuclear Magnetic Resonance Analysis (NMR): Molecular Structural Analysis by $^{1}$H-NMR and $^{19}$F-NMR)

For the products obtained in Examples and Comparative Examples, molecular structural analysis was carried out using $^{1}$H-NMR (400 MHz) and $^{19}$F-NMR (337 MHz) under the following measurement conditions.

[Measurement conditions]

Measurement device: JNM-ECZ400S type nuclear magnetic resonance apparatus (manufactured by JEOL, Ltd.)
Observation nucleus: $^{1}$H, $^{19}$F
Solvent: Deuterated chloroform, Deuterated dimethyl sulfoxide
Reference material: Tetramethylsilane ($^{1}$H, 0.00 ppm), Trichlorofluoromethane ($^{19}$F, 0.00 ppm)
Concentration of reference material: 5% by mass
Concentration of measured sample: 20% by mass
Pulse width: 6.5 μsec
Delay time: 2 seconds
Number of scans: 8 times ($^{1}$H), 1,024 times ($^{19}$F)

<Content of Compound (2)>

From the measurement results of $^{19}$F-NMR, the content of the compound (2) was measured from the integrated value of the representative peaks of the compounds (1A) and (2).

In this Example, the content of the fluorine-containing cyclic sulfonimide lithium salt (compound (1-2)) represented by the following structural formula:

[Chemical Formula 25]

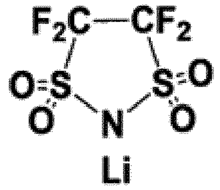

was calculated from the integrated value of the peak at -115.4 ppm (4F), and the content of $HCF_2$ $SO_2$ NHLi (compound (2-1)) was calculated from the integrated value of the peak at - 122.6 ppm (2F).

(ICP Atomic Emission Spectroscopy)

For the products obtained in Examples and Comparative Examples, molecular structural analysis was carried out under the following measurement conditions.

[Measurement Conditions]

Measurement device: SPS3520UV-DD (manufactured by Hitachi High-Tech Science Corporation)
Measurement atom: Li

Sample preparation conditions: A primary dilution water having a 1% by mass concentration was obtained by mixing 0.1 g of the product with 9.9 g of ultrapure water. Then, 1.5 g of the primary dilution water was mixed with 28.5 g of an aqueous 1% by mass nitric acid solution to prepare a measurement sample.

High-frequency power: 1.2 kW
Plasma gas (argon) flow rate: 16 L/min
Auxiliary gas (argon) flow rate: 0.5 L/min
Carrier gas (argon) pressure: 0.24 MPa
Carrier gas (argon) flow rate: 0.3 L/min
Photometric height: 12 mm
Chamber gas (argon) flow rate: 0.6 L/min

(Temperature at which Mass Reduction Rate reaches 1% by mass and 2% by mass)

For the products obtained in Examples and Comparative Examples, the mass reduction rate was measured under the following measurement conditions.

The sample (5 mg) was weighed in an aluminum pan ("Crimp cell (for autosampler) product number 346-66963-91" manufactured by Shimadzu Corporation, a cover was not used during measurement), the temperature was raised from 25°C to 100°C at a temperature rise rate of 10°C/min using a simultaneous thermogravimetric analyzer ("DTG-60A", manufactured by Shimadzu Corporation) under a nitrogen stream (flow rate of 50 mL/min) and, after temperature rising and holding at 100°C for 30 minutes, the sample was heated at a measurement temperature within a range of 100°C to 500°C at a temperature rise rate of 10°C/min, and the state of mass reduction was observed.

During the process of heating at a measurement temperature within range of 100°C to 500°C and a heating rate of 10°C/min, with the mass at the start of heating from 100°C after holding at 100°C for 30 minutes as a reference (100% by mass), the temperature (°C) at which the mass reduction rate of 1% by mass or 2% by mass was observed was measured.

<Starting Materials used>

Starting materials used in Examples and Comparative Examples are shown below. (Fluorosulfonyl group-containing carboxylic acid (compound (3)))
2,2-Difluoro-2-(fluorosulfonyl)acetic acid (manufactured by FUJIFILM Wako Pure Chemical Corporation) (compound (3-1))

Ammonia (manufactured by Sumitomo Seika Chemicals Co., Ltd.)

(Alkali Metal Salt (Compound (6))

Lithium hydroxide monohydrate (manufactured by FUJIFILM Wako Pure Chemical Corporation)

(Solvent)

Acetonitrile manufactured by FUJIFILM Wako Pure Chemical Corporation, wherein dried molecular sieve 3A 1/16 (manufactured by FUJIFILM Wako Pure Chemical Corporation) was added and dehydration was carried out, and then the molecular sieve 3A 1/16 was removed to adjust the water content.

Tetrahydrofuran manufactured by FUJIFILM Wako Pure Chemical Corporation, wherein dried molecular sieve 3A 1/16 (manufactured by FUJIFILM Wako Pure Chemical Corporation) was added and dehydration was carried out, and then the molecular sieve 3A 1/16 was removed to adjust the water content.

Activated carbon (manufactured by FUJIFILM Wako Pure Chemical Corporation)

<Reaction Temperature>

The reaction temperature is room temperature when it is room temperature without an external heating and cooling device. When using the external heating/cooling device such as a water bath or an oil bath, the reaction temperature is the temperature of the medium used in the external heating/cooling device.

[Example 1-1]

(Electrolytic Coupling Reaction Step)

In a 200 mL three-necked flask stirrer, a stirrer, acetonitrile (59 g) and water (75 g) were added in a nitrogen atmosphere and, after cooling to 0°C, $FO_2SCF_2CO_2H$ (compound (3-1), 25.7 g, 144.3 mmol) was added. As an anode and a cathode, platinum plate electrodes (25 mm × 50 mm) were placed at intervals of 6 mm and immersed in the solution. A current of 1.5 A was applied to the electrodes for 4 hours while stirring was maintained under cooling at 0°C. When the stirring was stopped after completion of the electrification, the reaction solution was separated into two phases. When the lower phase was fractionated, 7.9 g of liquid was obtained. The liquid thus obtained was sampled and, as a result of the measurement by $^{19}$F-NMR, it was confirmed that 90.3% by mass (yield of 37.1%) of $FO_2SCF_2CF_2SO_2F$ (compound (4-1)) and 1.2% by mass of $HCF_2SO_2F$ (compound (5-1)) are contained.

By purifying the crude $FO_2SCF_2CF_2SO_2F$ (compound (4-1)) obtained by the above operation by atmospheric simple distillation, 6.9 g of a liquid containing 95.6% by mass of $FO_2SCF_2CF_2SO_2F$ (compound (4-1)) and 0.042% by mass of $HCF_2SO_2F$ (compound (5-1)) was obtained.

$FO_2SCF_2CF_2SO_2F$ (compound (4-1))

$^{19}$F-NMR: δ (ppm) 46.1(2F), -108.7 (4F)

$HCF_2SO_2F$ (compound (5-1))

$^{1}$H-NMR: δ (ppm) 7.0 ppm (1H)
$^{19}$F-NMR: 36.0 (1F), -122.0 (2F)

(Cyclization Step)

After cooling a 3 L autoclave to -78°C and adding ammonia gas (250 g, 14,680 mmol) and tetrahydrofuran (222.3 g), a solution of $FO_2SCF_2CF_2SO_2F$ (compound (4-1), 208 g, purity of 95.6% by mass, 747.2 mmol) obtained in the electrolytic coupling reaction step in tetrahydrofuran (222.3 g) was added dropwise over 3 hours. After completion of the dropwise addition, the mixture was stirred at room temperature for 12 hours. After stirring, the reaction solution was filtered under pressure to remove insoluble solids, and the filtrate was concentrated under reduced pressure and then dried to obtain 193.0 g of light brown solids. A sample of the obtained solids was analyzed by $^{19}$F-NMR and, as a result, it was confirmed that 99.0% by mass (yield of 98.3%) of a fluorine-containing cyclic sulfonylimide ammonium salt (compound (1-N-2)) represented by the following structural formula:

[Chemical Formula 26]

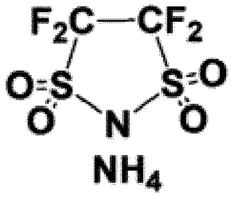

and 0.048% by mass of $HCF_2$ $SO_2$ $NHNH_4$ (compound (2-2)) are contained.
$^{19}$ F-NMR: δ (ppm) -115.4 (4F)

$HF_2$ $CSO_2$ $NHNH_4$ (compound (2-2))

$^{1}$ H-NMR: δ (ppm) 6.0 ppm (1H)
$^{19}$ F-NMR: 36.0 (1F), -122.6 (2F)

(Cation Exchange Step)

In a 1 L three-necked flask, a stirrer, tetrahydrofuran (400 g), the fluorine-containing cyclic sulfonylimide ammonium salt (compound (1-N-2), 192.7 g, 99.0% purity by weight, 733.2 mmol) obtained in Example I-1 and lithium hydroxide monohydrate (34.2 g, 815.1 mmol) were added in a nitrogen atmosphere, followed by stirring at 70°C for 4 hours. The reaction solution thus obtained was concentrated under reduced pressure, and then water (500 mL) and activated carbon (65.8 g) were added, followed by stirring at 105°C for 3 hours. The reaction solution thus obtained was filtered under pressure to remove insoluble solids, and then concentrated under reduced pressure to obtain light brown solids. To the light brown solids thus obtained, tetrahydrofuran (889 g) was added and, after stirring at 50°C for 30 minutes, the insoluble solids were removed by filtration under pressure, followed by concentration under reduced pressure to obtain 177.9 g of white solids. The solids thus obtained were sampled and analyzed by ICP atomic emission spectroscopy and, as a result, it was confirmed that the ammonium cations were exchanged with lithium cations. As a result of further analysis by $^{19}$F-NMR, it was confirmed that 99.9% by mass (yield of 97.3%) of a fluorine-containing cyclic sulfonimide lithium salt (compound (1-2)) represented by structural formula:

[Chemical Formula 27]

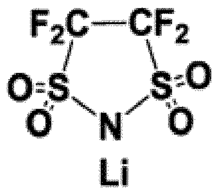

and 0.046% by mass of $HCF_2$ $SO_2$ NHLi (compound (2)) are contained.
Compound (1-2)
$^{19}$ F-NMR: δ (ppm) -115.4 (4F)

$HCF_2$ $SO_2$ NHLi (compound (2-1))

$^{1}$ H-NMR: δ (ppm) 6.0 ppm (1H)
$^{19}$ F-NMR: 36.0 (1F), -122.6 (2F)

[Comparative Example I-1]

(Electrolytic Coupling Reaction Step)

In the same manner as in Example I-1, except that purification by distillation was not carried out after the electrolytic reaction, $FO_2$ $SCF_2$ $CF_2$ $SO_2F$ (compound (4-1)) was produced to obtain 7.9 g (yield of 37.2%) of a liquid containing 90.3% by mass of $FO_2$ $SCF_2$ $CF_2$ $SO_2$ F (compound (4-1)) and 1.2% by mass of $HCF_2SO_2F$ (compound (5-1)).

(Cyclization Step)

In the same manner as in Example I-1, $FO_2$ $SCF_2$ $CF_2$ $SO_2F$ (compound (4-1)) obtained in the electrolytic coupling reaction step was used and a fluorine-containing cyclic sulfonylimide ammonium salt (compound (1-N-2)) was produced to obtain 188.0 g (yield of 98.3%) of a light brown solid containing 96.2% by mass of a fluorine-containing cyclic sulfonylimide ammonium salt (compound (1-N-2)) and 1.43% by mass of $HCF_2$ $SO_2$ $NHNH_4$ (compound (2-2)).

(Cation Exchange Step)

In the same manner as in Example I-1, the fluorine-containing cyclic sulfonylimide ammonium salt (compound (1-N-2)) obtained in the cyclization step was used and a fluorine-containing cyclic sulfonimide lithium salt (compound (1-2)) was produced to obtain 170.3 g (yield of 97.2%) of a white solid containing 98.3% by mass of a fluorine-containing cyclic sulfonimide lithium salt (compound (1-2)) and 1.22% by mass of $HCF_2$ $SO_2$ NHLi (compound (2-1)).

The temperature at which the mass reduction rate of the fluorine-containing cyclic sulfonimide lithium salt (compound (1-2)) obtained in Example I-1 and Comparative Example I-1 reaches 1% by mass and 2% by mass was measured according to the above method.

[Table 1]

| | Content of compound (2-2) [ppm] | Temperature at which mass reduction rate reaches 1% by mass [°C] | Temperature at which mass reduction rate reaches 2% by mass [°C] |
|---|---|---|---|
| Example I-1 | 462 | 250.4 | 395 |
| Comparative Example I-1 | 12,205 | 209.5 | 380.6 |

As is apparent from Table 1, in the fluorine-containing cyclic sulfonimide lithium salt (compound (1-2)) of Example 1-1 in which the content of $HF_2$ $CSO_2$ NHLi (compound (2-1)) was reduced, the temperature at which the mass reduction rate reaches 2% by mass increases, and the heat-resistant properties are improved compared to the fluorine-containing cyclic sulfonimide lithium salt (compound (1-2)) of Comparative Example I-1 having large content of $HCF_2$ $SO_2$ NHLi (compound (2-1). The reason is considered that the content of the thermally unstable compound (2) was reduced and thus the decomposition of the compound (1A) caused by the decomposition reaction of the compound (2) was suppressed, leading to an improvement in heat-resistant properties of the compound (1A).

<II. Non-Aqueous Electrolyte Solution>

(1) Preparation of Non-Aqueous Electrolyte Solution

In an inert atmosphere, various non-aqueous solvents were mixed so as to have a predetermined concentration. Further, various lithium salts were added so as to have a predetermined concentration to prepare non-aqueous electrolyte solutions (S01) to (S20). A non-aqueous electrolyte solution (S21) was prepared by using (S01) as a mother electrolyte solution and adding a compound represented by the formula (2-1) to a predetermined parts by mass. The compositions of these non-aqueous electrolyte solutions are shown in Table 2.

The compound (1-2) was synthesized by the method described below. Lot A is the compound after purification by the crystallization step, Lot B is the compound synthesized through the cyclization step and the cation exchange step after purification by distillation after the electrolytic coupling reaction step, and Lot C is the compound which underwent neither purification by crystallization nor purification by distillation after the electrolytic coupling reaction step.

In the electrolyte solutions (S02) and (S15), insoluble components, which are derived from the compound (1-2) (lot C) before purification and are different from the compound (2-1), were observed. Therefore, regarding the electrolyte solutions (S02) and (S15), a coin-type non-aqueous secondary battery was fabricated using the electrolyte solution from which the insoluble solids are removed by precipitating the insoluble components on the bottom of a container, followed by decantation.

The abbreviations of non-aqueous solvents and lithium salts in Table 2 have the following meanings.

(Non-Aqueous Solvent)

AN: Acetonitrile
PC: Propylene carbonate
EMC: Ethyl methyl carbonate
ES: Ethylene sulfite
VC: Vinylene carbonate
FEC: 4-Fluoro-1,3-dioxolan-2-one

(Lithium Salt)

(1-2): Compound represented by the above formula (1-2)
(1-6): Compound represented by the following formula (1-6)

[Chemical Formula 28]

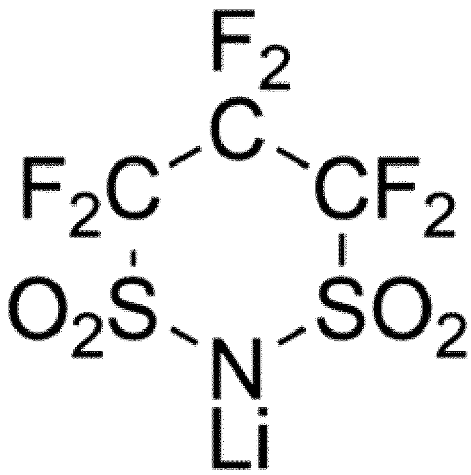

(1-6)

LiFSI: Lithium bis(fluorosulfonyl)imide ($LiN(SO_2F)_2$)
LiTFSI: Lithium bis(trifluoromethanesulfonul)imide ($LiN(SO_2CF_3)_2$)
(Fluorine-Containing Sulfonamide Compound)
(2-1): Fluorine-containing sulfonamide compound represented by the above formula (2-1): $HCF_2SO_2NHLi$

Fabrication of Compound (1-2)

<Analytical Method>

Analytical methods, starting materials, reaction conditions and the like used in the fabrication of the compound (1-2) were as follows.

(Nuclear Magnetic Resonance Analysis (NMR): Molecular Structural Analysis by $^1H$-NMR and $^{19}F$-NMR)

For the products obtained in the fabrication of the compound (1-2), molecular structural analysis was carried out using $^1H$-NMR (400 MHz) and $^{19}F$-NMR (337 MHz) under the following measurement conditions.

[Measurement Conditions]

Measurement device: JNM-ECZ400S type nuclear magnetic resonance apparatus (manufactured by JEOL, Ltd.)
Observation nucleus: $^1H$, $^{19}F$
Solvent: Deuterated chloroform, Deuterated dimethyl sulfoxide
Reference material: Tetramethylsilane ($^1H$, 0.00 ppm), Trichlorofluoromethane ($^{19}F$, 0.00 ppm)

Concentration of reference material: 5% by mass
Concentration of measured sample: 20% by mass
Pulse width: 6.5 μsec
Delay time: 2 seconds
Number of scans: 8 times ($^1H$), 1,024 times ($^{19}F$)

<Content of Compound (2-1)>

From the measurement results of $^{19}$F-NMR, the content of the compound (2-1) was measured from the integrated value of the representative peaks of the compounds (1-2) and (2-1).

In this Example, the content of the fluorine-containing cyclic sulfonimide lithium salt (compound (1-2)) represented by the following structural formula:

[Chemical Formula 29]

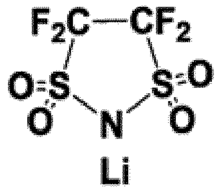

was calculated from the integrated value of the peak at -115.4 ppm (4F), and the content of $HCF_2SO_2NHLi$ (compound (2-1)) was calculated from the integrated value of the peak at -122.6 ppm (2F).

(ICP Atomic Emission Spectrometry)

For the compound (1-2) thus fabricated, molecular structural analysis was carried out under the following measurement conditions.

[Measurement Conditions]

Measurement device: SPS3520UV-DD (manufactured by Hitachi High-Tech Science Corporation)
Measurement atom: Li

Sample preparation conditions: A primary dilution water having a 1% by mass concentration was obtained by mixing 0.1 g of the product with 9.9 g of ultrapure water. Then, 1.5 g of the primary dilution water was mixed with 28.5 g of an aqueous 1% by mass nitric acid solution to prepare a measurement sample.

High-frequency power: 1.2 kW
Plasma gas (argon) flow rate: 16 L/min
Auxiliary gas (argon) flow rate: 0.5 L/min
Carrier gas (argon) pressure: 0.24 MPa
Carrier gas (argon) flow rate: 0.3 L/min
Photometric height: 12 mm
Chamber gas (argon) flow rate: 0.6 L/min

<Starting Materials used>

Starting materials used in the fabrication of the compound (1-2) are shown below.

2,2-Difluoro-2-(fluorosulfonyl)acetic acid (compound (3-1), manufactured by FUJIFILM Wako Pure Chemical Corporation)

Ammonia (manufactured by Sumitomo Seika Chemicals Co., Ltd.)

Lithium hydroxide monohydrate (manufactured by FUJIFILM Wako Pure Chemical Corporation)

Acetonitrile manufactured by FUJIFILM Wako Pure Chemical Corporation, wherein dried molecular sieve 3A 1/16 (manufactured by FUJIFILM Wako Pure Chemical Corporation) was added and dehydration was carried out, and then the molecular sieve 3A 1/16 was removed to adjust the water content.

Tetrahydrofuran manufactured by FUJIFILM Wako Pure Chemical Corporation, wherein dried molecular sieve 3A 1/16 (manufactured by FUJIFILM Wako Pure Chemical Corporation) was added and dehydration was carried out, and then the molecular sieve 3A 1/16 was removed to adjust the water content.

Activated carbon (manufactured by FUJIFILM Wako Pure Chemical Corporation)

<Reaction Temperature>

The reaction temperature is room temperature when it is room temperature without an external heating and cooling device. When using the external heating/cooling device such as a water bath or an oil bath, the reaction temperature is the temperature of the medium used in the external heating/cooling device.

<Synthesis of Compound (1-2) (Lots A and C)

(Electrolytic Coupling Reaction Step)

In a 200 mL three-necked flask stirrer, a stirrer, acetonitrile (59 g) and water (75 g) were added in a nitrogen atmosphere and, after cooling to 0°C, $FO_2SCF_2CO_2H$ (compound (3-1), 25.7 g, 144.3 mmol) was added. As an anode and a cathode, platinum plate electrodes (25 mm × 50 mm) were placed at intervals of 6 mm and immersed in a solution. A current of 1.5 A was applied to the electrodes for 4 hours while stirring was maintained under cooling at 0°C. When the stirring was stopped after completion of the electrification, the reaction solution was separated into two phases. When the lower phase was fractionated, 7.9 g of liquid was obtained. The liquid thus obtained was washed with water and then dried over molecular sieve to obtain 6.9 g of a liquid containing $FO_2SCF_2CF_2SO_2F$ (compound (4-1)).

$FO_2SCF_2CF_2SO_2F$ (compound (4-1))

$^{19}$F-NMR (deuterated chloroform): δ (ppm) 46.1(2F), -108.7 (4F)

(Cyclization Step)

After cooling a 3 L autoclave to -78°C and adding ammonia gas (250 g, 14.68 mmol) and tetrahydrofuran (250mL), a solution of $FO_2SCF_2CF_2SO_2F$ (compound (4-1), 208 g, 0.71 mmol) in tetrahydrofuran (250.0 mL) was added dropwise while maintaining the temperature in the autoclave at -55°C. After completion of the dropwise addition, the mixture was stirred overnight at room temperature. Thereafter, while maintaining stirring, the internal pressure was returned to normal pressure, and argon was blown into the autoclave at a rate of 0.5 L/min for 1.5 hours to discharge ammonia. White solids were removed by filtering the reaction solution, followed by washing several times with tetrahydrofuran. The filtrate thus obtained was transferred to a 3 L flask, concentrated under reduced pressure and then concentrated under reduced pressure at 40°C for 12 hours to obtain 188.0 g of a solid. The solid thus obtained was sampled and analyzed by $^{19}$F-NMR and, as a result, it was confirmed to be a fluorine-containing cyclic sulfonylimide ammonium salt (compound (1-N-2), 0.7 mol, yield of 98%) represented by the following structural formula:

[Chemical Formula 30]

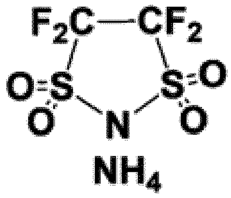

$^{19}$F-NMR (deuterated dimethyl sulfoxide): δ (ppm) -115.4 (4F)

(Cation Exchange Step)

In a 1 L three-necked flask, a stirrer, tetrahydrofuran (450 mL), the fluorine-containing cyclic sulfonylimide ammonium salt (compound (1-N-2), 187.0 g, 0.69 mol) and lithium hydroxide monohydrate (32.2 g, 0.77 mol) were added in a nitrogen atmosphere, followed by stirring at 70°C for 4 hours. The reaction solution thus obtained was concentrated under reduced pressure, and then ion-exchanged water (500 mL) and activated carbon (65.8 g) were added, followed by stirring at 105°C for 3 hours. The reaction solution thus obtained was filtered under pressure to remove insoluble solids, and then concentrated under reduced pressure to obtain solids (205.8 g). To the light brown solids thus obtained, tetrahydrofuran (1 L) was added and, after stirring at 50°C for 30 minutes, the insoluble solids were removed by filtration under pressure, followed by concentration under reduced pressure and further vacuum drying at 70°C for 16 hours, then at 90°C for 10 hours to obtain 170.3 g of slightly light brown solids. The solids thus obtained were sampled and analyzed by ICP atomic emission spectroscopy and, as a result, it was confirmed that the ammonium cations were exchanged with lithium cations. As a result of further analysis by $^{19}$F-NMR, it was confirmed to be a fluorine-containing cyclic sulfonimide lithium salt (compound (1-2), 0.68 mol, yield of 98%) represented by the structural formula:

[Chemical Formula 31]

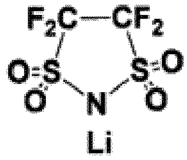

The crystals thus obtained were sampled and analyzed by $^{19}$F-NMR and, as a result, it was confirmed that 99.1% by mass of the compound (1-2) and 6,600 ppm by mass of $HCF_2SO_2NHLi$ (compound (2-1)) are contained (Lot C).

Compound (1-2)
$^{19}$F-NMR (deuterated dimethyl sulfoxide): δ (ppm) -115.4 (4F)

$HCF_2SO_2NHLi$ (compound (2-1))

$^{1}$H-NMR (deuterated dimethyl sulfoxide): δ (ppm) 6.0 (1H)
$^{19}$F-NMR (deuterated dimethyl sulfoxide): δ (ppm) -122.6 (2F)

(Crystallization Step)

In a 100 mL recovery flask equipped with a stirrer, 10 g of the synthesized compound (1-2) and 27 g of tert-butyl methyl ether were weighed, and a hermetic stopper was attached, followed by stirring for 30 minutes. The mixture thus obtained was filtered under reduced pressure and then cooled in a freezer at -20°C for 30 minutes to yield colorless crystals. After removing the liquid portion by decantation, tert-butyl methyl ether at -35°C was added and mixed with a spatula to wash the crystals, and the liquid portion was removed by decantation. The crystals thus obtained were dried under reduced pressure for 3 hours under the conditions of 60°C and 40 hPa without stirring. The crystals were pulverized using a mortar and a pestle, and 20 mL of Vertrel (manufactured by Chemours-Mitsui Fluoroproducts Co., Ltd, main component: 1,1,1,2,2,3,4,5,5,5-decafluoropentane) was added, and then an additional drying step in which the operation of drying under reduced pressure for 2 hours under the conditions of 100°C and 15 hPa without stirring was repeated four times to obtain 4.9 g (yield of 49.0%) of crystals. The crystals thus obtained were sampled and analyzed by $^{19}$F-NMR, and it was confirmed that 99.9% by mass of the compound (1-2) and 462 ppm by mass of $HCF_2SO_2NHLi$

(compound (2-1)) are contained (Lot A).
Compound (1-2)

$^{19}$F-NMR (deuterated dimethyl sulfoxide): δ (ppm) -115.4 (4F)

$HCF_2SO_2NHLi$ (compound (2-1))

$^{1}$H-NMR (deuterated dimethyl sulfoxide): δ (ppm) 6.0 (1H)
$^{19}$F-NMR (deuterated dimethyl sulfoxide): δ (ppm) -122.6 (2F)

<Synthesis of Compound (1-2) (Lot B)>

(Electrolytic Coupling Reaction Step)

In a Schlenk tube with external dimensions of a diameter of 30 mm and a height of 170 mm, and a capacity of 50 mL, a stirrer, acetonitrile (4.5 g) and water (22.6 g) were added in a nitrogen atmosphere and, after cooling to 0°C, $FO_2SCF_2CO_2H$ (compound (3-1), 30.0 g, 168.2 mmol) was added. As an anode and a cathode, platinum plate electrodes (13 mm × 50 mm) were placed at intervals of 3 mm and immersed in a solution. A current of 1.5 A (231 mA/cm$^2$) was applied to the electrodes for 3 hours while stirring was maintained under cooling at 0°C. When the stirring was stopped after completion of the electrification, the reaction solution was separated into two phases. When the lower phase was fractionated, 17.2 g of liquid was obtained. The liquid thus obtained was dried over molecular sieve and then purified by distillation to obtain 12.2 g of a liquid containing $FO_2SCF_2CF_2SO_2F$ (compound (4-1)).

$FO_2SCF_2CF_2SO_2F$ (compound (4-1))

$^{19}$F-NMR (deuterated chloroform): δ (ppm) 46.1 (2F), -108.7 (4F)

(Cyclization Step)

After cooling a 3 L autoclave to -78°C and adding ammonia gas (240 g, 14,09 mmol) and tetrahydrofuran (200 mL), a solution of $FO_2SCF_2CF_2SO_2F$ (compound (4-1), 173 g, 0.59 mol) in tetrahydrofuran (200.0 mL) was added dropwise while maintaining the temperature in the autoclave at -55°C. After completion of the dropwise addition, the mixture was stirred overnight at room temperature. Thereafter, while maintaining stirring, the internal pressure was returned to normal pressure, and argon was blown into the autoclave at a rate of 0.5 L/min for 1.5 hours to discharge ammonia. White solids were removed by filtering the reaction solution, followed by washing several times with tetrahydrofuran. The filtrate thus obtained was transferred to a 3 L flask, concentrated under reduced pressure and then concentrated under reduced pressure at 40°C for 12 hours to obtain 160.2. g of a solid. The solid thus obtained was sampled and analyzed by $^{19}$F-NMR and, as a result, it was confirmed to be a fluorine-containing cyclic sulfonylimide ammonium salt (compound (1-N-2), 0.56 mol, yield of 95%) represented by the following structural formula:

[Chemical Formula 32]

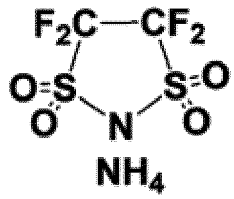

$^{19}$F-NMR (deuterated dimethyl sulfoxide): δ (ppm) -115.4 (4F).

(Cation Exchange Step)

In a 1 L three-necked flask, a stirrer, tetrahydrofuran (564 mL), a fluorine-containing cyclic sulfonylimide ammonium salt (compound (1-N-2), 242.0 g, 0.93 mol) and lithium hydroxide monohydrate (42.9 g, 1.02 mol) were added in a nitrogen atmosphere, followed by stirring at 75°C for 4 hours. The reaction solution thus obtained was concentrated under reduced pressure, and then ion-exchanged water (628 mL) and activated carbon (82.7 g) were added, followed

by stirring at 105°C for 3 hours. The reaction solution thus obtained was filtered under pressure to remove insoluble solids, and then concentrated under reduced pressure to obtain solids (245 g). To the light brown solids thus obtained, tetrahydrofuran (1.27 L) was added and, after stirring at 50°C for 30 minutes, the insoluble solids were removed by filtration under pressure, followed by concentration under reduced pressure and further vacuum drying at 70°C for 16 hours, then at 90°C for 6 hours to obtain 212 g of white solids. The solids thus obtained were sampled and analyzed by ICP atomic emission spectroscopy and, as a result, it was confirmed that the ammonium cations were exchanged with lithium cations. As a result of further analysis by $^{19}$F-NMR, it was confirmed to be a fluorine-containing cyclic sulfonimide lithium salt (compound (1-2), 0.85 mol, yield of 91%)represented by structural formula:

[Chemical Formula 33]

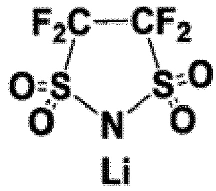

The crystals thus obtained were samples and analyze by $^{19}$F-NMR and, as a result, it was confirmed that 99.8% by mass of compound (1-2) and 761 ppm by mass of $HCF_2SO_2NHLi$ (compound (2-1)) are contained (Lot B).
Compound (1-2)

$^{19}$F-NMR (deuterated dimethyl sulfoxide): δ (ppm) -115.4 (4F)

$HCF_2SO_2NHLi$ (compound (2-1))

$^{1}$H-NMR (deuterated dimethyl sulfoxide): δ (ppm) 6.0 (1H)
$^{19}$F-NMR (deuterated dimethyl sulfoxide): δ (ppm) -122.6 (2F)

[Table 2]

| Electrolyte solution no. | Lithium salt [mol/L] | | | | | | | Composition of non-aqueous solvent [% by volume] | | | | | | | (2-1) Content in electrolyte solution [ppm by mass] |
|---|---|---|---|---|---|---|---|---|---|---|---|---|---|---|---|
| | (1-2) | | | (1-6) | LiFSI | LiTFSI | $LiPF_6$ | AN | PC | EMC | EC | ES | VC | FEC | |
| | Lot | (2-1) Content [ppm by mass] | Concentration | | | | | | | | | | | | |
| S01 | A | 462 | 0.94 | - | - | - | - | 93.6 | - | - | - | - | - | 6.4 | 102 |
| S02 | C | 6,600 | 0.94 | - | - | - | - | 93.6 | - | - | - | - | - | 6.4 | 1,451 |
| S03 | - | - | - | - | 0.94 | - | - | 93.9 | - | - | - | - | - | 6.1 | - |
| S04 | - | - | - | - | - | 0.93 | - | 93.4 | - | - | - | - | - | 6.6 | - |
| S05 | B | 761 | 0.94 | - | - | - | - | 93.6 | - | - | - | - | - | 6.4 | 137 |
| S06 | A | 462 | 0.1 | - | 0.9 | - | 0.3 | 93.6 | - | - | - | - | - | 6.4 | 9 |
| S07 | A | 462 | 0.7 | - | 0.3 | - | 0.3 | 93.6 | - | - | - | - | - | 6.4 | 62 |
| S08 | A | 462 | 0.9 | - | - | - | - | 20 | - | 73.6 | - | - | - | 6.4 | 80 |
| S09 | A | 462 | 0.9 | - | - | - | - | 40 | - | 53.6 | - | - | - | 6.4 | 80 |
| S10 | A | 462 | 0.9 | - | - | - | - | 60 | - | 33.6 | - | - | - | 6.4 | 80 |
| S11 | A | 462 | 1.3 | - | - | - | - | 20 | - | 73.6 | - | - | - | 6.4 | 115 |
| S12 | A | 462 | 1 | - | - | - | - | - | - | 69 | 29 | - | 2 | - | 89 |
| S13 | B | 761 | 1 | - | - | - | - | - | - | 69 | 29 | - | 2 | - | 146 |
| S14 | - | - | - | - | 1 | - | 0.3 | 93.6 | - | - | - | - | - | 6.4 | - |
| S15 | C | 6,600 | 1 | - | - | - | - | - | - | 69 | 29 | - | 2 | - | 1,265 |
| S16 | - | - | - | - | 1 | - | - | - | - | 69 | 29 | - | 2 | - | - |
| S17 | - | - | - | 0.94 | - | - | - | 93.6 | - | - | - | - | - | 6.4 | - |
| S18 | - | - | - | - | - | - | 1 | 93.6 | - | - | - | - | - | 6.4 | - |
| S19 | A | 462 | 1.25 | - | - | - | 0.05 | - | 10 | 73.5 | 10 | 4 | 2 | - | 111 |
| S20 | A | 462 | 1.3 | - | - | - | - | - | 10 | 73.5 | 10 | 4 | 2 | - | 115 |
| S21 | A | 462 | 0.94 | - | - | - | - | 93.6 | - | - | - | - | - | 6.4 | 118 |

(2) Fabrication of Coin-Type Non-Aqueous Secondary Battery

(2-1) Fabrication of Positive Electrode (P1)

A composite oxide of lithium, nickel, manganese and cobalt ($LiNi_{0.33}Mn_{0.33}Co_{0.33}O_2$) as a positive electrode active material, carbon black powder as a conductive aid, and polyvinylidene fluoride (PVDF) as a binder were mixed at a mass ratio of 94:3:3 to obtain a positive electrode mixture.

N-methyl-2-pyrrolidone as the solvent was added to the obtained positive electrode mixture, followed by further mixing to prepare a positive electrode mixture-containing slurry. While adjusting the basis weight of the positive electrode mixture-containing slurry to 11.4 mg/cm$^2$ , the positive electrode mixture-containing slurry was coated on one side of an aluminum foil having a thickness of 20 $\mu$m, which serves as a positive electrode current collector, and then the solvent was removed by drying. Then, rolling was carried out by a roll press so that the density of the positive electrode active material layer was 2.71 g/cm$^3$ to obtain a positive electrode (P1) composed of the positive electrode active material layer and the positive electrode current collector.

(2-2) Fabrication of Negative Electrode (N1)

A graphite powder as a negative electrode active material, a carbon black powder as a conductive aid, and polyvinylidene fluoride (PVDF) as a binder were mixed at a mass ratio of 90:3:7 to obtain a negative electrode mixture.

Water as the solvent was added to the negative electrode mixture thus obtained, followed by further mixing to prepare a negative electrode mixture-containing slurry. While adjusting the basis weight of the negative electrode mixture-containing slurry to 5.4 mg/cm$^2$ , the negative electrode mixture-containing slurry was coated on one side of a copper foil having a thickness of 8 $\mu$m, which serves as a negative electrode current collector, and then the solvent was removed by drying. Then, rolling was carried out by a roll press so that the density of the negative electrode active material layer was 1.31 g/cm$^3$ to obtain a negative electrode (N1) composed of the negative electrode active material layer and the negative electrode current collector.

(2-3) Assembling of Coin-Type Non-aqueous Secondary Battery

A polypropylene gasket was set in a CR2032 type battery casing (SUS304/Al-cladded), and the positive electrode (P1) obtained as described above punched in a disk shape having a diameter of 15.958 mm was set in the center of the gasket while the positive electrode active material layer faces upward. A glass fiber filter paper (GA-100, manufactured by Advantech Co., Ltd.) punched in a disk shape having a diameter of 16.156 mm was set therein, and 150 $\mu$L of each of non-aqueous electrolyte solutions (S01 to S21) was injected. Then, the negative electrode (N1) obtained as described above punched in a disk shape having a diameter of 16.156 mm was set therein while the negative electrode active material layer faces downward. Further, a spacer and a spring were set in a battery casing, and a battery cap was fitted and crimped with a caulking machine. The overflowing electrolyte solution was wiped off with a waste cloth. After maintaining a laminated body of a positive electrode, a glass fiber filter paper and a negative electrode, and an assembly of the non-aqueous electrolyte solution at a temperature of 25°C for 12 hours to fully adapt the electrolyte solution to the laminated body to obtain a coin-type non-aqueous secondary battery.

(3) Evaluation of Coin-Type Non-aqueous Secondary Battery

For the coin-type non-aqueous secondary batteries obtained as described above, first, an initial charging treatment and the initial charging/discharge capacity measurement were carried out according to the following procedure (3-1). Then, each coin-type non-aqueous secondary battery was evaluated according to the procedure (3-2). The charging/discharging was carried out using a charging/discharging apparatus ACD-M01A (trade name) manufactured by Aska Electronic Co., Ltd., and a program thermostatic bath IN804 (trade name) manufactured by Yamato Scientific Co., Ltd.

As used herein, "1 C" means the current value at which a fully charged battery is expected to be discharged in one hour with a constant current to terminate discharging. In the following evaluations (3-1) to (3-2), "1 C" means the current value at which a fully charged battery of 4.2 V is expected to be discharged to 3.0 V in one hour with a constant current to terminate discharging.

The portable non-aqueous secondary battery assembled according to the above procedure (2-3) is a 3 mAh class cell, and the battery voltage at which the battery is fully charged is defined as 4.2 V, and a current corresponding to 1 C is set at 3.0 mA. Hereinafter, unless otherwise specified, the notation of current value and the voltage is omitted for convenience.

(3-1) Initial Charging/Discharging Treatment

After setting the ambient temperature of the coin-type non-aqueous secondary battery at 25°C and charging with a constant current of 0.075 mA corresponding to 0.025 C to reach 3.1 V, the battery was discharged with a constant voltage of 4.2 V until the current attenuated to 0.025 mA. After resting for 3 hours, the battery was charged with a constant current of 0.15 mA corresponding to 0.05 C to reach 4.2 V, and then discharged with a constant voltage of 4.2 V until the current attenuated to 0.025 C. The charge capacity at this time (the sum of the charge capacity up to 3.1 V and the charge capacity up to 4.2 V) was defined as the initial charge capacity (X). Thereafter, the battery was discharged to 3.0 V with a constant current of 0.45 mA corresponding to 0.15C. The discharge capacity at this time was defined as the initial discharge capacity (Y). The initial charging/discharging efficiency was calculated based on the following formula.

$$\text{Initial charging/discharging efficiency} = (\text{initial discharge capacity (Y)/initial charge capacity (X)}) \times 100\ [\%]$$

After reaching 4.2 V with a constant current of 0.6 mA corresponding to 0.2 C, the battery was charged with a constant voltage of 4.2 V until the current attenuated to 0.025 C. Thereafter, the battery was discharged to 3 V with a current value of 0.6 mA corresponding to 0.2 C. Then, the same charging/discharging as above was carried out for 1 cycle.

(3-2) Cycle Test at 25°C

For the coin-type non-aqueous secondary battery subjected to the initial charging/discharging treatment by the method described in (3-1), the ambient temperature was set at 25°C and the battery was charged with a constant current of 3 mA corresponding to 1 C to reach 4.2 V, and then charged with a constant voltage of 4.2 V until the current attenuated to 0.025 C. Thereafter, the battery was discharged to 3.0 V with a constant current of 3 mA corresponding to 1 C. With this process of carrying out charging/discharging once each serving as one cycle, charging/discharging was carried out for 100 cycles. The discharge capacity at this time was defined as the 100th cycle discharge capacity (hereinafter sometimes referred to as (T)). The cycle capacity retention rate was calculated based on the following formula.

$$\text{Cycle capacity retention rate} = (\text{100th cycle discharge capacity in cycle test at 25°C (T)/initial discharge capacity in initial charging/discharging treatment (Y)}) \times 100\ [\%]$$

Note that, if the voltage is reduced 2 V or less before the cycle test at 25°C, the cycle test was not carried out as a voltage abnormality.

(4) ICP Atomic Emission Spectrometry of Negative Electrode

After completion of the cycle test at 25°C, the battery was discharged with a constant current corresponding to 0.1 C until the battery voltage reached 2.5 V in an environment at 25°C. Thereafter, the coin-type non-aqueous secondary battery was disassembled in an argon atmosphere, and the negative electrode was taken out. The negative electrode was washed with acetonitrile and then dried. Furthermore, the pretreatment shown below was carried out and then the negative electrode was subjected to ICP atomic emission spectrometry.

As the pretreatment, the sample was placed in a Teflon (registered trademark) decomposition container, and a mixed solvent of 3 mL of 68% nitric acid and 5 mL of 98% sulfuric acid was added. After thermal decomposition by microwave at 220°C for 45 minutes (first stage reaction), 2 mL of 68% nitric acid was added, followed by further heat decomposition by microwave at 230°C for 40 minutes (second stage reaction), the total amount was adjusted to about 100 g with ultrapure water. Since graphite remained as the residue, the filtered solution was used for ICP measurement. ETHOS one manufactured by Milestone General K.K. was used as a microwave pretreatment device, and the microwave output was 1,000 W.

The ICP atomic emission spectrometry was carried out under the following conditions, and the Al eluting amount of the negative electrode per 1 g of the negative electrode (including negative electrode current collector foil) was measured.

Apparatus: SPS3520UV-DD manufactured by SII Nano Technology Inc.
High frequency power: 1.2 (Kw)

Plasma gas (Ar) flow rate: 16 (L/min)
Auxiliary gas (Ar) flow rate: 0.5 (L/min)
Carrier gas (Ar) pressure: 0.24 (MPa)
Carrier gas (Ar) flow rate: 0.3 (L/min)
Chamber gas (Ar) flow rate: 0.6 (L/min)
Purge gas ($N_2$) flow rate: 5 (L/min)
Photometric height: 12 (mm)
Element: Al
Spectrometer: R
Wavelength: 167.079 (nm)
Purge gas: ON

The calibration curve was formed using 6-point calibration curve standard solutions of 0, 0.01, 0.05, 0.1, 0.5, 1 and 2 mg/L prepared by diluting 100 mg/L of an Al standard solution with an aqueous acid aqueous solution (prepared by adding 68% nitric acid and 98% sulfuric acid to ultrapure water so as to have the same acid concentration as that of a decomposition solution of the sample).

(5) Surface SEM Analysis of Positive Electrode Al Current Collector

After completion of the cycle test at 25°C, the battery was discharged with a constant current corresponding to 0.1 C until the battery voltage reached 2.5 V in an environment at 25°C. Thereafter, the coin-type non-aqueous secondary battery was disassembled in an argon atmosphere, and the positive electrode was taken out. The positive electrode active material on the positive electrode Al current collector was removed with a cotton swab soaked in N-methyl-2-pyrrolidone (NMP). The positive electrode Al current collector was washed with acetonitrile and then dried. Then, the positive electrode Al current collector was cut into an appropriate size in an argon atmosphere and fixed to a sample station for scanning electron microscope (SEM) observation to obtain a surface SEM observation sample. Using a dedicated transfer vessel, the sample was introduced into a SEM apparatus while shutting off the atmosphere.

SU8220 manufactured by Hitachi Ltd. was used for SEM observation. The measurement condition was an acceleration voltage of 1 kV.

Surface SEM photographs of the positive electrode Al current collector after the cycle test of the coin-type non-aqueous secondary battery fabricated with the non-aqueous electrolyte solution used in Example II-1 are shown in FIG. 3 (at a magnification of 40 times) and FIG. 4 (at a magnification of 1,000 times).

Surface SEM photographs of the positive electrode Al current collector after the cycle test of the coin-type non-aqueous secondary battery fabricated with the non-aqueous electrolyte solution used in Comparative Example II-1 are shown in FIG. 5 (at a magnification of 40 times) and FIG. 6 (at a magnification of 1,000 times).

Surface SEM photographs of the positive electrode Al current collector after the cycle test of the coin-type non-aqueous secondary battery fabricated with the non-aqueous electrolyte solution used in Comparative Example II-3 are shown in FIG. 7 (at a magnification of 40 times), FIG. 8 (at a magnification of 1,000 times) and FIG. 9 (at a magnification of 10,000 times).

Here, the interpretation of each test will be described.

The initial efficiency indicates the ratio of the initial discharge capacity to the initial charge capacity, and is commonly lower than the charge/discharge efficiency after the second time. This is because Li ions are used to form the negative electrode SEI during the initial charging, thus reducing Li ions which can be discharged. Here, there is no problem if the initial charge/discharge efficiency is 84% or more, and 85% or more is preferable.

The cycle capacity retention rate at 25°C is an index of battery deterioration due to repeated use. It is considered that the larger this value is, the less the reduction in capacity due to repeated use, thus enabling use for applications intended for long-term use.

Therefore, it is preferable that the cycle capacity retention rate at 25°C is 70% or more.

Also, if the voltage is reduced to 2 V or less before the cycle test at 25°C, it is considered that micro short circuits occur in the battery and it is difficult to use the battery for applications intended for long-term use.

The Al eluting amount of the negative electrode is an index of battery deterioration due to repeated use. It is considered that the larger this value is, the more the corrosion of the positive electrode Al current collector foil due to repeated use is suppressed and the deterioration of the negative electrode SEI due to reductive deposition of Al on the negative electrode is suppressed, thus enabling use for applications intended for long-term use.

Therefore, the Al eluting amount of the negative electrode is preferably 1,000 ppm by mass or less, and more preferably 500 ppm by mass or less.

The results of the initial charging/discharging treatment, cycle test at 25°C, and ICP atomic emission spectrometry of the negative electrode are shown in Table 3.

[Table 3]

| | Electrolyte solution No. | Positive electrode No. | Negatvive electrode No. | Initial charge capacity [mAh] | Initial discharge capacity [mAh] | Initial efficiency [%] | Cycle capcity retention rate [%] | Al eluting amount of negative electrode [ppm by mass] |
|---|---|---|---|---|---|---|---|---|
| Example II-1 | S01 | P1 | N1 | 3.78 | 3.24 | 85.9 | 79.7 | 180 |
| Comparative Example II-1 | S02 | P1 | N1 | 3.81 | 3.22 | 84.4 | 78.9 | 5,500 |
| Comparative Example II-2 | S03 | P1 | N1 | 4.23 | 2.92 | 69.1 | Voltage anomaly | - |
| Comparative Example II-3 | S04 | P1 | N1 | 4.08 | 3.92 | 71.4 | 0.4 | 20,000 |
| Example II-2 | S05 | P1 | N1 | 3.76 | 3.20 | 85.0 | 79.1 | 650 |
| Example II-3 | S06 | P1 | N1 | 3.71 | 3.21 | 86.5 | 81.7 | 100 |
| Example II-4 | S07 | P1 | N1 | 3.60 | 3.08 | 85.6 | 97.5 | 80 |
| Example II-5 | S08 | P1 | N1 | 3.68 | 3.18 | 86.4 | 96.8 | 250 |
| Example II-6 | S09 | P1 | N1 | 3.65 | 3.14 | 86.0 | 92.2 | 280 |
| Example 11-7 | S10 | P1 | N1 | 3.55 | 3.05 | 85.9 | 88.1 | 325 |
| Example II-8 | S11 | P1 | N1 | 3.54 | 3.05 | 86.2 | 97.5 | 230 |
| Example II-9 | S12 | P1 | N1 | 3.71 | 3.17 | 85.4 | 98.2 | 8 |
| Example II-10 | S13 | P1 | N1 | 3.61 | 3.12 | 86.4 | 96.9 | 11 |
| Comparative Example II-4 | S14 | P1 | N1 | 3.70 | 3.04 | 82.2 | 56.4 | 180 |
| Comparative Example II-5 | S15 | P1 | N1 | 4.67 | 3.38 | 72.4 | 99.8 | 53 |
| Comparative Example II-6 | S16 | P1 | N1 | 3.66 | 3.15 | 86.1 | 85.2 | 3,200 |
| Comparative Example II-7 | S17 | P1 | N1 | 3.73 | 2.93 | 78.6 | 16.8 | 160 |
| Comparative Example II-8 | S18 | P1 | N1 | 3.75 | 3.21 | 85.6 | 49.5 | 17 |

(continued)

| | Electrolyte solution No. | Positive electrode No. | Negatvive electrode No. | Initial charge capacity [mAh] | Initial discharge capacity [mAh] | Initial efficiency [%] | Cycle capcity retention rate [%] | Al eluting amount of negative electrode [ppm by mass] |
|---|---|---|---|---|---|---|---|---|
| Example II-11 | S19 | P1 | N1 | 3.57 | 3.12 | 87.4 | 97.1 | 12 |
| Example II-12 | S20 | P1 | N1 | 3.61 | 3.15 | 87.3 | 96.9 | 18 |
| Example II-13 | S21 | P1 | N1 | 3.75 | 3.21 | 85.6 | 79.4 | 250 |

As shown in Table 3, in Examples II-1 to 11-13, the initial efficiency, cycle capacity retention rate and Al eluting amount of the negative electrode all fell within preferred ranges. Meanwhile, in Comparative Examples II-2, II-3, II-4, II-5 and II-7, the initial efficiency exhibited the value of less than 84%. In Comparative Examples II-3, II-4, II-7 and II-8, the cycle capacity retention exhibited the value of less than 70%. In Comparative Examples II-1, II-3 and II-6, the Al eluting amount of the negative electrode exhibited the value of more than 1,000 ppm by mass.

When comparing Example II-1 with Comparative Examples II-1 to II-3, it was found that by using a fluorine-containing cyclic sulfonylimide salt as the lithium salt and adjusting the amount of the fluorine-containing sulfonamide compound within a preferred range, the initial efficiency is improved. It is considered that this is because the Al corrosion reaction at the positive electrode and the Al reductive deposition reaction at the negative electrode during the initial charge were suppressed, thus suppressing an increase in irreversible capacity. Regarding the initial charge capacity and the initial discharge capacity, although the initial charge capacity of Example II-1 decreased as compared to Comparative Examples II-1 to II-3, the initial discharge capacity of Example II-1 did not decrease as compared Comparative Examples II-1 to II-3, which suggests that the irreversible reaction caused by Al corrosion is suppressed.

When comparing Example II-1 with Comparative Examples II-1 and II-3, it was found that by using a fluorine-containing cyclic sulfonylimide salt as the lithium salt and adjusting the amount of the fluorine-containing sulfonamide compound within a preferred range, the cycle capacity retention rate is improved, and the Al eluting amount of the negative electrode is reduced. It is considered that this is because the continuous Al corrosion reaction at the positive electrode and the Al reductive deposition reaction at the negative electrode are suppressed during the cycle test, and the increase in irreversible capacity is suppressed, thus suppressing physical damage of the negative electrode SEI due to Al reductive-deposited at the negative electrode.

In Comparative Example II-2 using LiFSI as the lithium salt, the voltage decreased to 2 V or less before the start of the cycle test, thus causing voltage abnormality. It is considered that this is because severe Al corrosion reaction occurred at the positive electrode and an Al reduction deposition reaction occurred at the negative electrode during the initial charge/discharge, and micro short circuits occurred due to Al reductive-deposited at the negative electrode.

From FIG. 7, many pitting corrosion was observed on the Al current collector surface. One of pits is magnified in FIG. 8 and FIG. 9, and in FIG. 9, additional pits were observed inside the pit, in addition to the distortion of the outer edge inside the pit. It is considered that this is because the positive electrode Al current collector was severely corroded when the fluorine-containing cyclic sulfonylimide salt was not used as the lithium salt. Meanwhile, in FIG. 3 and FIG. 4, no pitting corrosion was observed on the surface of the Al current collector, and severe corrosion of the positive electrode Al current collector can be suppressed by using a fluorine-containing cyclic sulfonylimide salt as the lithium salt.

When comparing Example II-1 with Example II-2, when Lot A was used as the fluorine-containing cyclic sulfonylimide salt, the cycle capacity retention rate was improved compared to the case where Lot B was used, and the Al eluting amount of the negative electrode is reduced. It is considered that this is because the purification by crystallization has higher effect of reducing the fluorine-containing sulfonamide compound compared with the purification by distillation after the electrolytic coupling reaction step, and the fluorine-containing cyclic sulfonylimide salt and the fluorine-containing sulfonamide compound in the electrolyte solution are reduced, and the continuous Al corrosion reaction at the positive electrode and the Al reduction deposition reaction at the negative electrode during the cycle test are suppressed, and thus an increase in irreversible capacity is suppressed, and physical damage of the negative electrode SEI due to Al reductive-deposited at the negative electrode was suppressed.

(6) Discharge IR Drop Increase Rate after 100 Cycles in Cycle Test at 25°C

In each cycle, the value (unit: kΩ) obtained by dividing a difference (unit: V) between the voltage before the start of discharging and the voltage 10 seconds after the start of discharging by 3.0 (unit: mA) as the discharge current value was defined as the discharge IR drop in each cycle and used as an index of internal resistance.

The discharge IR drop in the 1st cycle was defined as the 1st cycle discharge IR drop, and the discharge IR drop in the 100th cycle was defined as the 100th cycle discharge IR drop. The discharge IR drop increase rate after 100 cycles was calculated based on the following formula.

IR drop increase rate after 100 cycles = (100th cycle discharge IR drop/1st cycle discharge IR drop in cycle test at 25°C) $\times$ 100 [%]

The discharge IR drop increase rate after 100 cycles is an index of battery deterioration due to repeated use. It is considered that the smaller this value is, the less the increase in internal resistance due to repeated use, thus enabling use for applications intended for long-term use. Therefore, in a non-aqueous secondary battery containing a non-aqueous electrolyte solution containing acetonitrile, the discharge IR drop increase rate after 100 cycles is preferably 400% or less, more preferably 200% or less. The evaluation results thus obtained are shown in Table 4.

[Table 4]

| | Electrolyte solution No. | Discharge IR drop increase rate after 100 cycles [%] |
|---|---|---|
| Example II-14 | S01 | 164 |
| Comparative Example II-9 | S04 | 825 |
| Example II-15 | S05 | 301 |

As shown in Table 4, in Examples 11-14 and II-15, the discharge IR drop increase rate after 100 cycles was within a preferable range. Meanwhile, in Comparative Example II-9, the discharge IR drop increase rate after 100 cycles exhibited the value of more than 400%. It is considered that this is because the continuous Al corrosion reaction in the positive electrode and the Al reductive deposition reaction in the negative electrode were suppressed during the cycle test, and thus the increase in internal resistance was suppressed.

Comparing Example 11-14 with Example 11-15, when Lot A was used as the fluorine-containing cyclic sulfonylimide salt, the discharge IR drop increase rate after 100 cycles was reduced compared with the case where Lot B was used. It is considered that this is because the crystallization purification has higher effect of reducing the fluorine-containing sulfonamide compound compared with the purification by distillation after the electrolytic coupling reaction step, and the fluorine-containing cyclic sulfonylimide salt and the fluorine-containing sulfonamide compound in the electrolyte solution are reduced. It is considered that this is because the continuous Al corrosion reaction in the positive electrode and the Al reductive deposition reaction in the negative electrode were suppressed during the cycle test, and thus the increase in internal resistance was suppressed.

(7) Fabrication of Portable Non-aqueous Secondary Battery

(7-1) Fabrication of Positive Electrode (P2)

A composite oxide of lithium, nickel, manganese and cobalt ($LiNi_{0.8}Mn_{0.1}Co_{0.1}O_2$) as a positive electrode active material, a carbon black powder as a conductive aid, and polyvinylidene fluoride (PVDF) as a binder were mixed at a mass ratio of 94:3:3 to obtain a positive electrode mixture.

N-methyl-2-pyrrolidone as the solvent was added to the positive electrode mixture thus obtained, followed by further mixing to prepare a positive electrode mixture-containing slurry. While adjusting the basis weight of the positive electrode mixture-containing slurry to 16.6 mg/$cm^2$ , the positive electrode mixture-containing slurry was coated on one side of an aluminum foil having a thickness of 20 $\mu$m, which serves as a positive electrode current collector, and then the solvent was removed by drying. Then, rolling was carried out by a roll press so that the density of the positive electrode active material layer became 2.91 g/$cm^3$ to obtain a positive electrode (P2) composed of the positive electrode active material layer and the positive electrode current collector.

(7-2) Fabrication of Negative Electrode (N2)

A graphite powder as the negative electrode active material (A), a carbon black powder as the conductive aid (B) and a polyvinylidene fluoride (PVDF) as the binder (C) were mixed at a solid component mass ratio of 90:3:7 to obtain a negative electrode mixture.

Water as the solvent was added to the negative electrode mixture thus obtained, followed by further mixing to prepare a negative electrode mixture-containing slurry. While adjusting the basis weight of the negative electrode mixture-containing slurry to 10.3 mg/$cm^2$ , the slurry was coated on one side of a copper foil having a thickness of 8 $\mu$m, which serves as a negative electrode current collector, and then the solvent was removed by drying. Then, rolling was carried out by a roll press so that the density of the negative electrode active material layer became 1.36 g/$cm^3$ to obtain a negative electrode (N2) composed of the negative electrode active material layer and the negative electrode current collector.

(7-3) Assembling of Portable Non-aqueous Secondary Battery

The positive electrode (P2) obtained as described above was punched into a disk shape having a diameter of 18 mm, and the negative electrode (N2) obtained as described above was punched into a disk shape having a diameter of 18 mm were overlapped on both sides of a separator-embedded insulating sleeve (ECC1-00-0210-W/X, manufactured by EL-Cell) and after injecting 120 $\mu$L of each of non-aqueous electrolyte solutions (S19 to S20), the positive electrode side was pressed by an aluminum plunger and the negative electrode side was pressed by a SUS plunger to obtain a

laminated body. After assembling the laminated body by inserting it into a battery case (PAT-Cell, manufactured by EL-Cell), the battery case was sealed and maintained at 25°C for 12 hours to fully adapt the non-aqueous electrolyte solution to the laminated body to obtain a portable non-aqueous secondary battery.

(8) Evaluation of Portable Non-aqueous Secondary Battery

For the portable non-aqueous secondary battery obtained as described above, first, the initial charging treatment and the initial charging/discharge capacity measurement were carried out according to the following procedure (8-1). Then, each portable non-aqueous secondary battery was evaluated according to the following procedure (8-2). The charging/discharging was carried out using a charging/discharging apparatus ACD-M01A (trade name) manufactured by Aska Electronic Co., Ltd., and a program thermostatic bath IN804 (trade name) manufactured by Yamato Scientific Co., Ltd.

As used herein, "1 C" means the current value at which a fully charged battery is expected to be discharged in one hour with a constant current to terminate discharging. In the following evaluations (8-1) to (8-2), "1 C" means the current value at which a fully charged battery of 4.2 V is expected to be discharged to 3.0 V in one hour with a constant current to terminate discharging.

The portable non-aqueous secondary battery assembled according to the above procedure (7-3) is a 7.4 mAh class cell, and the battery voltage at which the battery is fully charged is defined as 4.2 V, and a current corresponding to 1 C is set at 7.4 mA. Hereinafter, unless otherwise specified, the notation of current value and the voltage is omitted for convenience.

(8-1) Initial Charging/Discharging Treatment of Portable Non-aqueous Secondary Battery

The ambient temperature of the portable non-aqueous secondary battery was set at 25°C, and the battery was charged with a constant current of 0.185 mA corresponding to 0.025 C to reach 3.1 V, and then charged with a constant voltage of 3.1 V for 1.5 hours. After resting for 3 hours, the battery was charged with a constant current of 0.37 mA corresponding to 0.05 C to reach 4.2 V, and then charged with a constant voltage of 4.2 V for 1.5 hours. Thereafter, the battery was discharged to 3.0 V with a constant current of 1.11 mA corresponding to 0.15 C.

(8-2) Cycle Test at 50°C of Portable Non-aqueous Secondary Battery

For the portable non-aqueous secondary battery subjected to initial charging/discharging treatment by the method described in (8-1), the ambient temperature was set at 50°C and the battery was charged with a constant current of 3.7 mA corresponding to 0.5 C to reach 4.2 V, and then charged with a constant voltage of 4.2 V for 1.5 hours. Then, the battery was discharged to 3.0 V with a constant current of 3.7 mA corresponding to 0.5 C. With this process of carrying out charging/discharging once each serving as one cycle, charging/discharging was carried out for 100 cycles.

In each cycle, the value (unit: kΩ) obtained by dividing a difference (unit: V) between the voltage before the start of discharging and the voltage 10 seconds after the start of discharging by 3.7 (unit: mA) as the discharge current value was defined as the discharge IR drop in each cycle and used as an index of internal resistance.

The discharge IR drop in the 1st cycle was defined as the 1st cycle discharge IR drop, and the discharge IR drop in the 100th cycle was defined as the 100th cycle discharge IR drop. The discharge IR drop increase rate after 100 cycles was calculated based on the following formula.

IR drop increase rate after 100 cycles = (100th cycle discharge IR drop/1st cycle discharge IR drop in cycle test at 50°C) $\times$ 100 [%]

The discharge IR drop increase rate after 100 cycles is an index of battery deterioration due to repeated use. It is considered that the smaller this value is, the less the increase in internal resistance due to repeated use, thus enabling use for applications intended for long-term use. Therefore, in a non-aqueous secondary battery containing a non-aqueous electrolyte solution containing acetonitrile, the discharge IR drop increase rate after 100 cycles is preferably 200% or less, more preferably 130% or less. The evaluation results thus obtained are shown in Table 5.

[Table 5]

| | Electrolyte solution No. | Positive electrode No. | Negative electrode no. | $LiPF_6$/ (1-2) [Molar ratio] | $LiPF_6$/VC [Molar ratio] | (1-2)/ $LiPF_6$ [Molar ratio] | Discharge IR drop increase rate after 100 cycles [%] |
|---|---|---|---|---|---|---|---|
| Example II-16 | S19 | P2 | N2 | 0.04 | 0.75 | 25 | 129 |
| Example II-17 | S20 | P2 | N2 | 0 | 0.13 | - | 134 |

As shown in Table 5, in Example II-16, the discharge IR drop increase rate after 100 cycles exhibited the value of 130% or less. Meanwhile, in Example II-17, the discharge IR drop increase rate after 100 cycles exhibited the value of more than 130%.

When comparing Example II-16 with Example 11-17, when the non-aqueous electrolyte solution contains $LiPF_6$, the discharge IR drop increase rate after 100 cycles is improved. It is considered that this is because the inclusion of $LiPF_6$ satisfies the amount of hydrogen fluoride (HF) required for aluminum passivation and formation of the negative electrode SEI, and the decomposition product of the cyclic anion works as a negative electrode SEI component.

When comparing Example II-16 with Example II-17, when the content of $LiPF_6$ is within a range of 0.01 or more and 4 or less, in terms of molar ratio, relative to the content of vinylene carbonate, the discharge IR drop increase rate after 100 cycles is improved. It is considered that this is because, when the content of $LiPF_6$ is within a preferable range relative to the content of vinylene carbonate, the ratio of the organic component derived from the cyclic anion-containing lithium salt and cyclic carbonate in the negative electrode SEI to the inorganic component derived from $LiPF_6$ is appropriately controlled.

<III. Electrolytic Coupling Reaction (β/α Relationship and Relationship Between Reaction Vessel and Electrodes)>

<Analytical Method>

Analytical methods, starting materials, reaction conditions and the like used in Examples and Comparative Examples were as follows.

(Nuclear Magnetic Resonance Analysis (NMR): Molecular Structural Analysis by $^{1}$H-NMR and $^{19}$F-NMR)

For the products obtained in Examples and Comparative Examples, molecular structural analysis was carried out using $^{1}$H-NMR (400 MHz) and $^{19}$F-NMR (337 MHz) under the following measurement conditions.

[Measurement conditions]

Measurement device: JNM-ECZ400S type nuclear magnetic resonance apparatus

(manufactured by JEOL, Ltd.)

Observation nucleus: $^{1}$H, $^{19}$F
Solvent: Deuterated chloroform, Deuterated dimethyl sulfoxide
Reference material: Tetramethylsilane ($^{1}$H, 0.00 ppm), Trichlorofluoromethane ($^{19}$F, 0.00 ppm)
Concentration of reference material: 5% by mass
Concentration of measured sample: 20% by mass
Pulse width: 6.5 μsec
Delay time: 2 seconds
Number of scans: 8 times ($^{1}$H), 1,024 times ($^{19}$F)

<Starting Materials used>

Starting materials used in Examples and Comparative Examples are shown below. (Fluorosulfonyl group-containing carboxylic acid (compound (3))
2,2-Difluoro-2-(fluorosulfonyl)acetic acid (manufactured by FUJIFILM Wako Pure Chemical Corporation) (compound (3-1))
Ammonia (manufactured by Sumitomo Seika Chemicals Co., Ltd.) (Alkali Metal Salt (Compound (6))
Lithium hydroxide monohydrate (manufactured by FUJIFILM Wako Pure Chemical Corporation)

(Solvent)

Acetonitrile manufactured by FUJIFILM Wako Pure Chemical Corporation, wherein dried molecular sieve 3A 1/16 (manufactured by FUJIFILM Wako Pure Chemical Corporation) was added and dehydration was carried out, and then the molecular sieve 3A 1/16 was removed to adjust the water content.
Tetrahydrofuran manufactured by FUJIFILM Wako Pure Chemical Corporation, wherein dried molecular sieve 3A 1/16 (manufactured by FUJIFILM Wako Pure Chemical Corporation) was added and dehydration was carried out, and then the molecular sieve 3A 1/16 was removed to adjust the water content.
Activated carbon (manufactured by FUJIFILM Wako Pure Chemical Corporation)
Octafluorotoluene (manufactured by Tokyo Chemical Industry Co., Ltd.)

<Reaction Temperature>

The reaction temperature is room temperature when it is room temperature without an external heating and cooling device. When using the external heating/cooling device such as a water bath or an oil bath, the reaction temperature is the temperature of the medium used in the external heating/cooling device.

<Example regarding $\beta/\alpha$>

[Example III-1]

In a 100 mL three-necked flask stirrer, a stirrer, acetonitrile (12.9 g) and water (17.1 g) were added ($\beta/\alpha$ = 0.75) in a nitrogen atmosphere and, after cooling to 0°C, $FO_2SCF_2CO_2H$ (compound (3-1), 5.0 g, 28.1 mmol) was added. As an anode and a cathode, platinum plate electrodes (25 mm × 50 mm) were placed at intervals of 3 mm and immersed in a solution. A current of 1.5 A was applied to the electrodes for 0.5 hour while stirring was maintained under cooling at 0°C. After completion of the electrification, the liquid thus obtained was sampled and, as a result of the measurement by $^{19}$F-NMR, it was confirmed that the production ratio of $FO_2SCF_2CF_2SO_2F$ (compound (4-1)) to $HCF_2SO_2F$ (compound (5-1)) was 100:0.9.

$FO_2SCF_2CF_2SO_2F$ (compound (4-1))

$^{19}$F-NMR: $\delta$ (ppm) 46.1 (2F), -108.7 (4F)

$HCF_2SO_2F$ (compound (5-1))

1 H-NMR: $\delta$ (ppm) 7.7ppm (1H)
1 9 F-NMR: 37.0 (1F), -120.3 (2F)

[Example III-2]

In the same manner as in Example III-1, except that acetonitrile (10.0 g) and water (20.0 g) were used, electrification was carried out ($\beta/\alpha$ = 0.50). After completion of the electrification, the liquid thus obtained was sampled and, as a result of the measurement by $^{19}$F-NMR, it was confirmed that the production ratio of $FO_2SCF_2CF_2SO_2F$ (compound (4-1)) to $HCF_2SO_2F$ (compound (5-1)) was 100:0.8.

[Example III-3]

In the same manner as in Example III-1, except that acetonitrile (5.0 g) and water (25.0 g) were used, electrification was carried out (β/α = 0.20). After completion of the electrification, the liquid thus obtained was sampled and, as a result of the measurement by $^{19}$F-NMR, it was confirmed that the production ratio of $FO_2SCF_2CF_2SO_2F$ (compound (4-1)) to $HCF_2SO_2F$ (compound (5-1)) was 100:0.4.

[Example III-4]

(Electrolytic Coupling Reaction)

In the same manner as in Example III-1, except that acetonitrile (2.5 g) and water (27.5 g) were used, electrification was carried out (β/α = 0.09). After completion of the electrification, the liquid thus obtained was sampled and, as a result of the measurement by $^{19}$F-NMR, it was confirmed that the production ratio of $FO_2SCF_2CF_2SO_2F$ (compound (4-1)) to $HCF_2SO_2F$ (compound (5-1)) was 100:0.3.

(Cyclization Step)

A 1 L autoclave was cooled to -78°C and ammonia gas (60.0 g, 3523.2 mmol) and tetrahydrofuran (110.0 g) were added, and then a solution of $FO_2SCF_2CF_2SO_2F$ (compound (3-1), 50 g, 187.5 mmol) obtained in the electrolytic coupling reaction in tetrahydrofuran (50.0 g) was added dropwise over 3 hours. After completion of the dropwise addition, the mixture was stirred at room temperature for 12 hours. After completion of the stirring, the reaction solution was subjected to filtered under pressure to remove insoluble solids, and the filtrate was concentrated under reduced pressure and then dried to obtain 49.1 g of light brown solids. The solids thus obtained was analyzed by $^{19}$F-NMR and, as a result, it was confirmed to be a fluorine-containing cyclic sulfonylimide ammonium salt (compound (1-N-2), yield of 99.1%) represented by the following structural formula:

[Chemical Formula 34]

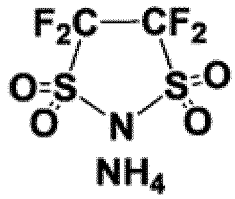

$^{19}$F-NMR: δ (ppm) -115.4 (4F)

(Cation Exchange Step)

In a 1 L three-necked flask, a stirrer, tetrahydrofuran (100 g), the fluorine-containing cyclic sulfonylimide ammonium salt (compound (1-N-2), 45.0 g, 170.4 mmol) obtained in the cyclization step and lithium hydroxide monohydrate (8.1 g, 193.0 mmol) were added in a nitrogen atmosphere, followed by stirring at 70°C for 4 hours. The reaction solution thus obtained was concentrated under reduced pressure, and then water (120 mL) and activated carbon (16.5 g) were added, followed by stirring at 105°C for 3 hours. The reaction solution thus obtained was filtered under pressure to remove insoluble solids, and then concentrated under reduced pressure to obtain light brown solids. To the light brown solids thus obtained, tetrahydrofuran (220 g) was added and, after stirring at 50°C for 30 minutes, the insoluble solids were removed by filtration under pressure, followed by concentration under reduced pressure to obtain 40.9 g of white solids. The solids thus obtained were sampled and analyzed by ICP atomic emission spectroscopy and, as a result, it was confirmed that the ammonium cations were exchanged with lithium cations. As a result of further analysis by $^{19}$F-NMR, it was confirmed to be a fluorine-containing cyclic sulfonimide lithium salt (compound (1-2), yield of 95.9%) represented by the following structural formula:

[Chemical Formula 35]

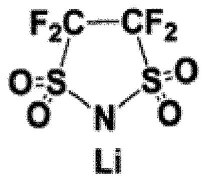

$^{19}$F-NMR: δ (ppm) -115.4 (4F)

[Example III-5]

In the same manner as in Example III-1, except that acetonitrile (13.0 g) and water (17.0 g) were used, electrification was carried out (β/α = 0.76). After completion of the electrification, the liquid thus obtained was sampled and, as a result of the measurement by $^{19}$F-NMR, it was confirmed that the production ratio of $FO_2SCF_2CF_2SO_2F$ (compound (4-1)) to $HCF_2SO_2F$ (compound (5-1)) was 100:1.6.

[Example III-6]

In the same manner as in Example III-1, except that acetonitrile (15.0 g) and water (15.0 g) were used, electrification was carried out (β/α = 1.00). After completion of the electrification, the liquid thus obtained was sampled and, as a result of the measurement by $^{19}$F-NMR, it was confirmed that the production ratio of $FO_2SCF_2CF_2SO_2F$ (compound (4-1)) to $HCF_2SO_2F$ (compound (5-1)) was 100:4.4.

[Example III-7]

In the same manner as in Example III-1, except that acetonitrile (20.0 g) and water (10.0 g) were used, electrification was carried out (β/α = 2.00). After completion of the electrification, the liquid thus obtained was sampled and, as a result of the measurement by $^{19}$F-NMR, it was confirmed that the production ratio of $FO_2SCF_2CF_2SO_2F$ (compound (4-1)) to $HCF_2SO_2F$ (compound (5-1)) was 100:8.4.

[Example III-8]

In a Schlenk tube with external dimensions of a diameter of 30 mm and a height of 170 mm, and a capacity of 50 mL, a stirrer, acetonitrile (5 g) and water (25 g) were added (β/α = 0.2) in a nitrogen atmosphere and, after cooling to 0°C, $FO_2SCF_2CO_2H$ (compound (3-1), 5.2 g, 29.4 mmol) was added. As an anode and a cathode, platinum plate electrodes (13 mm × 50 mm) were placed at intervals of 3 mm and immersed in a solution. A current of 1.5 A (231 $mA/cm^2$) was applied to the electrodes for 0.5 hour (1 F/mol) while stirring was maintained under cooling at 0°C. When the stirring was stopped after completion of the electrification, the reaction solution was separated into two phases. Analysis of the upper phase by $^{19}$F-NMR revealed that $HCF_2SO_2F$ (compound (5-1)) was produced in a yield of 0.4%, $HO_2CCF_2SO_3H$ was produced in a yield of 0.2%, and 23% of $FO_2SCF_2CO_2H$ remained. Analysis of the lower phase by $^{19}$F-NMR revealed that $FO_2SCF_2CF_2SO_2F$ (compound (4-1)) was produced in a yield of 49% and $HCF_2SO_2F$ (compound (5-1)) was produced in a trace amount. The current efficiency was 49%. The production ratio of $FO_2SCF_2CF_2SO_2F$ (compound (4-1)) to $HCF_2SO_2F$ (compound (5-1)) was 100:0.8.

[Example III-9]

In a Schlenk tube with external dimensions of a diameter of 30 mm and a height of 170 mm, and a capacity of 50 mL, a stirrer, acetonitrile (10 g) and water (20 g) were added (β/α = 0.5) in a nitrogen atmosphere and, after cooling to 0°C, $FO_2SCF_2CO_2H$ (compound (3-1), 5.1 g, 28.7 mmol) was added. As an anode and a cathode, platinum plate electrodes (13 mm × 50 mm) were placed at intervals of 3 mm and immersed in a solution. A current of 1.5 A (231 $mA/cm^2$) was applied to the electrodes for 0.5 hour (1 F/mol) while stirring was maintained under cooling at 0°C. When the stirring was stopped after completion of the electrification, the reaction solution was separated into two phases. Analysis of the upper phase by $^{19}$F-NMR revealed that $FO_2SCF_2CF_2SO_2F$ (compound (4-1)) was produced in a yield of 0.5%, $FO_2SCF_2CF_2SO_3H$ was produced in a yield of 0.8%, $HCF_2SO_2F$ (compound (5-1)) was produced in a yield of 1%, $HO_2CCF_2SO_3H$ was produced in a yield of 0.2%, and 16% of $FO_2SCF_2CO_2H$ (compound (3-1)) remained.

Analysis of the lower phase by $^{19}F$-NMR revealed that $FO_2SCF_2CF_2SO_2F$ (compound (4-1)) was produced in a yield of 53% and $HCF_2SO_2F$ was produced in a trace amount. The current efficiency was 53%. The production ratio of $FO_2SCF_2CF_2SO_2F$ (compound (4-1)) to $HCF_2SO_2F$ (compound (5-1)) was 100:1.9.

[Example III-10]

In a Schlenk tube with external dimensions of a diameter of 30 mm and a height of 170 mm, and a capacity of 50 mL, a stirrer, acetonitrile (4.17 g) and water (20.85 g) were added ($\beta/\alpha$ = 0.2) in a nitrogen atmosphere and, after cooling to 0°C, $FO_2SCF_2CO_2H$ (compound (3-1), 10.1 g, 56.9 mmol) was added. As an anode and a cathode, platinum plate electrodes (13 mm × 50 mm) were placed at intervals of 3 mm and immersed in a solution. A current of 1.5 A (231 mA/cm$^2$) was applied to the electrodes for 1 hour (1 F/mol) while stirring was maintained under cooling at 0°C. When the stirring was stopped after completion of the electrification, the reaction solution was separated into two phases. Analysis of the upper phase by $^{19}F$-NMR revealed that $HCF_2SO_2F$ (compound (5-1)) was produced in a yield of 0.3%, $HO_2CCF_2SO_3H$ was produced in a yield of 0.6% and 17% of $FO_2SCF_2CO_2H$ (compound (3-1)) remained. Analysis of the lower phase by $^{19}F$-NMR revealed that $FO_2SCF_2CF_2SO_2F$ (compound (4-1)) was produced in a yield of 57% and $HCF_2SO_2F$ (compound (5-1)) was produced in a trace amount. The current efficiency was 57%. The production ratio of $FO_2SCF_2CF_2SO_2F$ (compound (4-1)) to $HCF_2SO_2F$ (compound (5-1)) was 100:0.5.

[Example III-11]

In a Schlenk tube with external dimensions of a diameter of 30 mm and a height of 170 mm, and a capacity of 50 mL, a stirrer, acetonitrile (3.3 g) and water (16.7 g) were added ($\beta/\alpha$ = 0.2) in a nitrogen atmosphere and, after cooling to 0°C, $FO_2SCF_2CO_2H$ (compound (3-1), 15.2 g, 85.2 mmol) was added. As an anode and a cathode, platinum plate electrodes (13 mm × 50 mm) were placed at intervals of 3 mm and immersed in a solution. A current of 1.5 A (231 mA/cm$^2$) was applied to the electrodes for 1.5 hours (1 F/mol) while stirring was maintained under cooling at 0°C. When the stirring was stopped after completion of the electrification, the reaction solution was separated into two phases. Analysis of the upper phase by $^{19}F$-NMR revealed that $HCF_2SO_2F$ (compound (5-1)) was produced in a yield of 0.1%, $HO_2CCF_2SO_3H$ was produced in a yield of 0.6% and 13% of $FO_2SCF_2CO_2H$ (compound (3-1)) remained. Analysis of the lower phase by $^{19}F$-NMR revealed that $FO_2SCF_2CF_2SO_2F$ (compound (4-1)) was produced in a yield of 62% and $HCF_2SO_2F$ (compound (5-1)) was produced in a trace amount. The current efficiency was 62%. The production ratio of $FO_2SCF_2CF_2SO_2F$ (compound (4-1)) to $HCF_2SO_2F$ (compound (5-1)) was 100:0.2.

[Example III-12]

In a Schlenk tube with external dimensions of a diameter of 30 mm and a height of 170 mm, and a capacity of 50 mL, a stirrer, acetonitrile (4.5 g) and water (22.5 g) were added ($\beta/\alpha$ = 0.2) in a nitrogen atmosphere and, after cooling to 0°C, $FO_2SCF_2CO_2H$ (compound (3-1), 30.0 g, 168.5 mmol) was added. As an anode and a cathode, platinum plate electrodes (13 mm × 50 mm) were placed at intervals of 3 mm and immersed in a solution. A current of 1.5 A (231 mA/cm$^2$) was applied to the electrodes for 3 hours (1 F/mol) while stirring was maintained under cooling at 0°C. When the stirring was stopped after completion of the electrification, the reaction solution was separated into two phases. Analysis of the upper phase by $^{19}F$-NMR revealed that $HCF_2SO_2F$ (compound (5-1)) was produced in a yield of 0.1%, $HO_2CCF_2SO_3H$ was produced in a yield of 0.7% and 9% of $FO_2SCF_2CO_2H$ (compound (3-1)) remained. Analysis of the lower phase by $^{19}F$-NMR revealed that $FO_2SCF_2CF_2SO_2F$ (compound (4-1)) was produced in a yield of 72% and $HCF_2SO_2F$ (compound (5-1)) was produced in a trace amount. The current efficiency was 72%. The production ratio of $FO_2SCF_2CF_2SO_2F$ (compound (4-1)) to $HCF_2SO_2F$ (compound (5-1)) was 100:0.1.

<Relationship Between Reaction Vessel and Electrodes>

[Example III-13]

In a Schlenk tube with external dimensions of a diameter of 30 mm and a height of 170 mm, and a capacity of 50 mL, a stirrer, acetonitrile (15 g) and water (15 g) were added in a nitrogen atmosphere and, after cooling to 0°C, $FO_2SCF_2CO_2H$ (compound (3-1), 5.0 g, 28.3 mmol)) was added. As an anode and a cathode, platinum plate electrodes (13 mm × 50 mm) were placed at intervals of 3 mm and immersed in a solution. A current of 1.5 A (231 mA/cm$^2$) was applied to the electrodes for 0.25 hour (0.5 F/mol) while stirring was maintained under cooling at 0°C. When the stirring was stopped after completion of the electrification, the reaction solution was separated into two phases. Analysis of the upper phase by $^{19}F$-NMR revealed that $FO_2SCF_2CF_2SO_2F$ (compound (4-1)) was produced in a yield of 8%, $FO_2SCF_2CF_2SO_3H$ was produced in a yield of 1%, $HCF_2SO_2F$ (compound (5-1)) was produced in a yield of 3%, $HO_2$

$CCF_2SO_3H$ was produced in a yield of 0.3% and 41% of $FO_2SCF_2CO_2H$ (compound (3-1)) remained. Analysis of the lower phase by $^{19}$F-NMR revealed that $FO_2SCF_2CF_2SO_2F$ (compound (4-1)) was produced in a yield of 22% and $HCF_2SO_2F$ (compound (5-1)) was produced in a trace amount. The current efficiency was 60%. Regarding the relationship between the reaction vessel and the electrodes, the height TL was 75 mm, the highest level TE was 65 mm, the height TS was 8 mm, and the lowest level TE' was 15 mm, so that TL > TE and TE' >TS were satisfied. During the reaction, the voltage did not rise drastically, and hunting did not occur.

$FO_2SCF_2CF_2SO_3H$

$^{19}$F-NMR: δ (ppm) 44.3 (1F), -106.2 (2F), -113.7 (2F)

$HCF_2SO_2F$ (compound (5-1))

$^{19}$F-NMR: δ (ppm) 37.0 (1F), -120.3 (2F)
$^{1}$H-NMR: δ (ppm) 6.5 (1H)

$HO_2CCF_2SO_3H$

$^{19}$F-NMR: δ (ppm) -111.5 (2F)

$FO_2SCF_2CO_2H$ (compound (3-1))

$^{19}$F-NMR: δ (ppm) 37.1 (1F), -102.0 (2F)

[Example III-14]

In a Schlenk tube with external dimensions of a diameter of 30 mm and a height of 170 mm, and a capacity of 50 mL, a stirrer, acetonitrile (15 g) and water (15 g) were added in a nitrogen atmosphere and, after cooling to 0°C, $FO_2SCF_2CO_2H$ (compound (3-1), 5.1 g, 28.4 mmol) was added. As an anode and a cathode, platinum plate electrodes (13 mm × 50 mm) were placed at intervals of 3 mm and immersed in a solution. A current of 1.5 A (231 mA/cm$^2$) was applied to the electrodes for 0.5 hour (1 F/mol) while stirring was maintained under cooling at 0°C. When the stirring was stopped after completion of the electrification, the reaction solution was separated into two phases. Analysis of the upper phase revealed that $FO_2SCF_2CF_2SO_2F$ (compound (4-1)) was produced in a yield of 8%, $FO_2SCF_2CF_2SO_3H$ was produced in a yield of 2%, $HCF_2SO_2F$ (compound (5-1)) was produced in a yield of 2% and 11% of $FO_2SCF_2CO_2H$ remained. Analysis of the lower phase revealed that $FO_2SCF_2CF_2SO_2F$ (compound (4-1)) was produced in a yield of 41% and $HCF_2SO_2F$ (compound (5-1)) was produced in a trace amount. The current efficiency was 49%. Regarding the relationship between the reaction vessel and the electrodes, the height TL was 75 mm, the highest level TE was 65 mm, the height TS was 8 mm, and the lowest level TE' was 15 mm, so that TL > TE and TE' >TS were satisfied. During the reaction, the voltage did not rise drastically, and hunting did not occur.

[Example III-15]

In a Schlenk tube with external dimensions of a diameter of 30 mm and a height of 170 mm, and a capacity of 50 mL, a stirrer, acetonitrile (15 g) and water (15 g) were added in a nitrogen atmosphere and, after cooling to 0°C, $FO_2SCF_2CO_2H$ (compound (3-1), 5.1 g, 28.5 mmol) was added. As an anode and a cathode, platinum plate electrodes (13 mm × 50 mm) were placed at intervals of 3 mm and immersed in a solution. A current of 1.5 A (231 mA/cm$^2$) was applied to the electrodes for 0.75 hour (1.5 F/mol) while stirring was maintained under cooling at 0°C. When the stirring was stopped after completion of the electrification, the reaction solution was separated into two phases. Analysis of the upper phase revealed that $FO_2SCF_2CF_2SO_2F$ (compound (4-1)) was produced in a yield of 7%, $FO_2SCF_2CF_2SO_3H$ was produced in a yield of 3%, $HCF_2SO_2F$ (compound (5-1)) was produced in a yield of 1%, $HO_2CCF_2SO_3H$ was produced in a trace amount, and 5% of $FO_2SCF_2CO_2H$ (compound (3-1)) remained. Analysis of the lower phase revealed that $FO_2SCF_2CF_2SO_2F$ (compound (4-1)) was produced in a yield of 38% and $HCF_2SO_2F$ (compound (5-1)) was produced. The current efficiency was 30%. Regarding the relationship between the reaction vessel and the electrodes, the height TL was 75 mm, the highest level TE was 65 mm, the height TS was 5 mm, and the lowest level TE' was 15 mm, so that TL > TE and TE' >TS were satisfied. During the reaction, the voltage did not rise drastically, and hunting did not occur.

[Example III-16]

In a Schlenk tube with external dimensions of a diameter of 30 mm and a height of 170 mm, and a capacity of 50 mL, a stirrer, acetonitrile (15 g) and water (15 g) were added in a nitrogen atmosphere and, after cooling to 0°C, $FO_2$ $SCF_2$ $CO_2$ H (compound (3-1), 5.0 g, 28.3 mmol) was added. As an anode and a cathode, platinum plate electrodes (13 mm × 50 mm) were placed at intervals of 3 mm and immersed in a solution. A current of 1.5 A (231 $mA/cm^2$) was applied to the electrodes for 1 hour (2 F/mol) while stirring was maintained under cooling at 0°C. When the stirring was stopped after completion of the electrification, the reaction solution was separated into two phases. Analysis of the upper phase by $^{19}F$-NMR revealed that $FO_2$ $SCF_2$ $CF_2$ $SO_2$ F (compound (4-1)) was produced in a yield of 7%, $FO_2$ $SCF_2$ $CF_2$ $SO_3$ H was produced in a yield of 3%, $HCF_2$ $SO_2F$ (compound (5-1)) was produced in a yield of 1%, $HO_2CCF_2SO_3H$ was produced in a trace amount, and 2% of $FO_2$ $SCF_2$ $CO_2H$ (compound (3-1)) remained. Analysis of the lower phase by $^{19}F$-NMR revealed that $FO_2$ $SCF_2$ $CF_2$ $SO_2$ F (compound (4-1)) was produced in a yield of 44% and $HCF_2$ $SO_2F$ (compound (5-1)) was produced in a trace amount. The current efficiency was 26%. Regarding the relationship between the reaction vessel and the electrodes, the height TL was 75 mm, the highest level TE was 65 mm, the height TS was 8 mm, and the lowest level TE' was 15 mm, so that TL > TE and TE' >TS were satisfied. During the reaction, the voltage did not rise drastically, and hunting did not occur.

The storage stability of $FO_2$ $SCF_2$ $CO_2$ H (compound (3-1)) was measured according to the following method.

[Evaluation of Storage Stability]

In a 30 mL screw tube, $FO_2$ $SCF_2$ $CO_2$ H (compound (3-1), 10.0 g), acetonitrile (1.5 g), water (7.5 g), and octafluorotoluene (0.3 g) as an internal standard substance for $^{19}F$-NMR were charged and, after shaking, $^{19}F$-NMR was measured. As a result of calculating the integrated value of the doublet peak at -102.0 ppm of the compound (3-1) when the integrated value of the triplet peak at -57.4 ppm of octafluorotoluene was 3, it was found to be 88.72. This value was defined as the standard content of the compound (3-1) at an elapsed time of 0 hour. Subsequently, the above solution was divided into three 10 mL screw tubes and each screw tube was left to stand at -20°C, -3°C and 23°C and, after a predetermined period of time has elapsed, $^{19}F$-NMR was measured. After calculating the integrated value of the doublet peak at -102.0 ppm of the compound (3-1) when the integrated value of the triplet peak at -57.4 ppm of octafluorotoluene was 3, and the residual rate of the compound (3-1) was calculated.

$$\text{Residual rate of compound (3-1)} = (\text{integrated value of doublet peak at -102.0 ppm})/88.72 \times 100$$

[Table 6]

| Standing temperature | Standing time | Integrated value at -102.0 ppm | Residual rate of compound (3-1) |
|---|---|---|---|
| -20°C | 19 hours | 87.24 | 98.30% |
| -20°C | 94 hours | 84.08 | 94.80% |
| 3°C | 19 hours | 85.44 | 96.30% |
| 3°C | 94 hours | 72.56 | 81.80% |
| 23°C | 19 hours | 57.42 | 64.70% |
| 23°C | 94 hours | 45.47 | 51.30% |

As is apparent from Table 6, the stability of the mixture of $FO_2$ $SCF_2$ $CO_2$ H (compound (3-1)), acetonitrile and water is dramatically improved by storage at 3°C or lower. Therefore, it is considered that the electrolytic coupling reaction is carried out at a low temperature (for example, within a range of -35°C to 10°C) to ensure the stability of the starting material, leading to suppression of the formation of by-products.

<IV. Electrolytic Coupling Reaction (δ/γ Relationship and Relationship Between Reaction Vessel and Electrodes)>

[Example IV-1]

In a 100 mL three-necked flask stirrer, a stirrer, acetonitrile (4.0 g) and water (18.5 g) were added in a nitrogen

atmosphere and, after cooling to 0°C, $FO_2SCF_2CO_2H$ (compound (3-1), 15.0 g, 84.2 mmol) was added (δ/γ = 1.5). As an anode and a cathode, platinum plate electrodes (25 mm × 50 mm) were placed at intervals of 3 mm and immersed in a solution. A current of 1.5 A was applied to the electrodes for 1.5 hours while stirring was maintained under cooling at 0°C. When the stirring was stopped after completion of the electrification, the reaction solution was separated into two phases. The lower phase was fractionated to obtain 9.0 g of liquid. The liquid thus obtained was sampled and, as a result of the measurement by $^{19}F$-NMR, it was found to be $FO_2SCF_2CF_2SO_2F$ (compound (4-1), purity of 90.5%, yield of 72.7%).

$FO_2SCF_2CF_2SO_2F$ (compound (4-1))

$^{19}F$-NMR: δ (ppm) 46.1 (2F), -108.7 (4F)

[Example IV-2]

In the same manner as in Example IV-1, except that acetonitrile (3.3 g), water (16.7 g) and $FO_2SCF_2CO_2H$ (compound (3-1), 15.0 g, 84.2 mmol) were used, electrification was carried out (δ/γ = 1.3). When the stirring was stopped after completion of the electrification, the reaction solution was separated into two phases. The lower phase was fractionated to obtain 9.4 g of liquid. The liquid thus obtained was sampled and, as a result of the measurement by $^{19}F$-NMR, it was found to be $FO_2SCF_2CF_2SO_2F$ (compound (4-1), purity of 92.4%, yield of 77.5%).

[Example IV-3]

(Electrolytic Coupling Reaction)

In the same manner as in Example IV-1, except that acetonitrile (4.5 g), water (22.5 g) and $FO_2SCF_2CO_2H$ (compound (3-1), 30.0 g, 168.5 mmol) were used, and the electrification time was set at 3 hours, electrification was carried out (δ/γ = 0.9). When the stirring was stopped after completion of the electrification, the reaction solution was separated into two phases. The lower phase was fractionated to obtain 20.6 g of liquid. The liquid thus obtained was sampled and, as a result of the measurement by $^{19}F$-NMR, it was found to be $FO_2SCF_2CF_2SO_2F$ (compound (4-1), purity of 92.4%, yield of 84.9%).

(Cyclization Step)

After cooling a 1 L autoclave to -78°C and adding ammonia gas (60.0 g, 3523.2 mmol) and tetrahydrofuran (110.0 g), a solution of $FO_2SCF_2CF_2SO_2F$ (compound (4-1), 50 g, purity of 92.4%, 173.6 mmol) obtained in the electrolytic coupling reaction step in tetrahydrofuran (50.0 g) was added dropwise over 3 hours. After completion of the dropwise addition, the mixture was stirred at room temperature for 12 hours. After stirring, the reaction solution was filtered under pressure to remove insoluble solids, and the filtrate was concentrated under reduced pressure and then dried to obtain 45.4 g of light brown solids. The solids thus obtained was samples and analyzed by $^{19}F$-NMR and, as a result, it was confirmed to be a fluorine-containing cyclic sulfonylimide ammonium salt (compound (1-N-2), purity of 97.7%, yield of 98.2%) represented by the following structural formula:

[Chemical Formula 36]

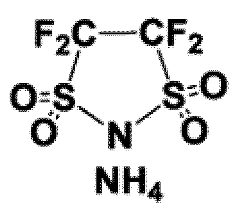

Compound (1-N-2)
$^{19}F$-NMR: δ (ppm) -115.4 (4F)

(Cation Exchange Step)

In a 1 L three-necked flask, a stirrer, tetrahydrofuran (100 g), the fluorine-containing cyclic sulfonylimide ammonium salt (compound (1-N-2), 45.0 g, 169.0 mmol) obtained in the cyclization step and lithium hydroxide monohydrate

(8.0 g, 190.7 mmol) were added in a nitrogen atmosphere, followed by stirring at 70°C for 4 hours. The reaction solution thus obtained was concentrated under reduced pressure, and then water (120 mL) and activated carbon (16.5 g) were added, followed by stirring at 105°C for 3 hours. The reaction solution thus obtained was filtered under pressure to remove insoluble solids, and then concentrated under reduced pressure to obtain light brown solids. To the light brown solids thus obtained, tetrahydrofuran (220 g) was added and, after stirring at 50°C for 30 minutes, the insoluble solids were removed by filtration under pressure, followed by concentration under reduced pressure to obtain 41.7 g of white solids. The solids thus obtained were sampled and analyzed by ICP atomic emission spectroscopy and, as a result, it was confirmed that the ammonium cations were exchanged with lithium cations. As a result of further analysis by $^{19}$F-NMR, it was confirmed to be a fluorine-containing cyclic sulfonimide lithium salt (compound (1-2), purity of 99.1%, yield of 98.2%) represented by the following structural formula:

[Chemical Formula 37]

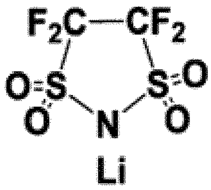

Compound (1-2)
$^{19}$F-NMR: $\delta$ (ppm) -115.4 (4F)

[Example IV-4]

In the same manner as in Example IV-1, except that acetonitrile (4.1 g), water (20.9 g) and $FO_2SCF_2CO_2H$ (compound (3-1), 10.0 g, 56.2 mmol) were used ($\delta/y$ = 2.5), and the electrification time was set at 1 hour, electrification was carried out. When the stirring was stopped after completion of the electrification, the reaction solution was separated into two phases. The lower phase was fractionated to obtain 4.8 g of liquid. The liquid thus obtained was sampled and, as a result of the measurement by $^{19}$F-NMR, it was found to be $FO_2SCF_2CF_2SO_2F$ (compound (4-1), purity of 91.0%, yield of 58.5%).

[Example IV-52]

In the same manner as in Example IV-1, except that acetonitrile (5.0 g), water (25.0 g) and $FO_2SCF_2CO_2H$ (compound (3-1), 5.0 g, 28.1 mmol) were used ($\delta/\gamma$ = 6.0), and the electrification time was set at 0.5 hour, electrification was carried out. When the stirring was stopped after completion of the electrification, the reaction solution was separated into two phases. The lower phase was fractionated to obtain 2.2 g of liquid. The liquid thus obtained was sampled and, as a result of the measurement by $^{19}$F-NMR, it was found to be $FO_2SCF_2CF_2SO_2F$ (compound (4-1), purity of 89.1%, yield of 51.5%).

[Example IV-6]

In the same manner as in Example IV-1, except that acetonitrile (4.0 g), water (20.0 g) and $FO_2SCF_2CO_2H$ (compound (3-1), 15.0 g, 84.2 mmol) were used ($\delta/\gamma$ = 1.6), electrification was carried out. When the stirring was stopped after completion of the electrification, the reaction solution was separated into two phases. The lower phase was fractionated to obtain 8.3g of liquid. The liquid thus obtained was sampled and, as a result of the measurement by $^{19}$F-NMR, it was found to be $FO_2SCF_2CF_2SO_2F$ (compound (4-1), purity of 91.1%, yield of 67.5%).

[Example IV-7]

In a Schlenk tube with external dimensions of a diameter of 30 mm and a height of 170 mm, and a capacity of 50 mL, a stirrer, acetonitrile (4.5 g) and water (22.5 g) were added in a nitrogen atmosphere and, after cooling to 0°C, $FO_2SCF_2CO_2H$ (compound (4-1), 30.0 g, 168.5 mmol) was added. As an anode and a cathode, platinum plate electrodes (13 mm × 50 mm) were placed at intervals of 3 mm and immersed in a solution. A current of 1.5 A (231 mA/$cm^2$) was applied to the electrodes for 3 hours (1 F/mol) while stirring was maintained under cooling at 0°C. When the stirring was

stopped after completion of the electrification, the reaction solution was separated into two phases. Analysis of the upper phase revealed that $HCF_2 SO_2 F$ (compound (5-1)) was produced in a yield of 0.1%, $HO_2 CCF_2 SO_3 H$ was produced in a yield of 0.7% and 9% of $FO_2 SCF_2 CO_2 H$ (compound (3-1)) remained. Analysis of the lower phase revealed that $FO_2 SCF_2 CF_2 SO_2 F$ (compound (4-1)) was produced in a yield of 72% and $HCF_2 SO_2F$ (compound (5-1)) was produced in a trace amount. The current efficiency was 72%.

[Example IV-8]

(Cyclization Step)

After cooling a 3 L autoclave to -78°C and adding ammonia gas (250 g, 14.68 mol) and tetrahydrofuran (250 mL), a solution of $FO_2 SCF_2 CF_2 SO_2F$ (compound (4-1), 208 g, 0.71 mmol) obtained in the electrolytic coupling reaction step in Example IV-3 in tetrahydrofuran (250.0 mL) was added dropwise while maintaining the temperature in the autoclave at -55°C. After completion of the dropwise addition, the mixture was stirred overnight at room temperature. Thereafter, while maintaining stirring, the internal pressure was returned to normal pressure, and argon was blown into the autoclave at a rate of 0.5 L/min for 1.5 hours to discharge ammonia. White solids were removed by filtering the reaction solution, followed by washing several times with tetrahydrofuran. The filtrate thus obtained was transferred to a 3 L flask, concentrated under reduced pressure and then vacuum-dried at 40°C for 12 hours to obtain 188.0 g of solids. The solids thus obtained were samples and, as a result of analysis by $^{19}F$-NMR, it was confirmed to be a fluorine-containing cyclic sulfonylimide ammonium salt (compound (1-N-2), 0.7 mol, yield of 98%) represented by the following structural formula.

[Chemical Formula 38]

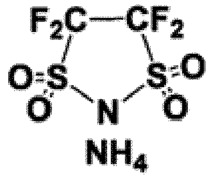

(Cation Exchange Step)

In a 1 L three-necked flask, a stirrer, tetrahydrofuran (450 mL), the fluorine-containing cyclic sulfonylimide ammonium salt (compound (1-N-2), 187.0 g, 0.69 mol) obtained in the cyclization step and lithium hydroxide monohydrate (32.2 g, 0.77 mol) were added in a nitrogen atmosphere, followed by stirring at 70°C for 4 hours. The reaction solution thus obtained was concentrated under reduced pressure, and then ion-exchanged water (500 mL) and activated carbon (65.8 g) were added, followed by stirring at 105°C for 3 hours. The reaction solution thus obtained was filtered under pressure to remove insoluble solids, and then concentrated under reduced pressure to obtain solids (205.8 g). To the light brown solids thus obtained, tetrahydrofuran (1 L) was added and, after stirring at 50°C for 30 minutes, the insoluble solids were removed by filtration under pressure, followed by concentration under reduced pressure at 70°C for 16 hours, then at 90°C for 10 hours to obtain 170.3 g of light brown solids. The solids thus obtained were sampled and analyzed by ICP atomic emission spectroscopy and, as a result, it was confirmed that the ammonium cations were exchanged with lithium cations. As a result of further analysis by $^{19}F$-NMR, it was confirmed to be a fluorine-containing cyclic sulfonimide lithium salt (compound (1-2), 0.68 mol, yield of 98%) represented by the following structural formula.

[Chemical Formula 39]

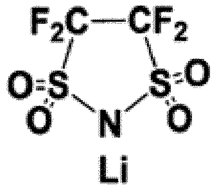

<V. Crystallization>

<Analytical Method>

Analytical methods, starting materials, reaction conditions and the like used in Examples and Comparative Examples were as follows.

(Nuclear Magnetic Resonance Analysis (NMR): Molecular Structural Analysis by $^{1}$H-NMR and $^{19}$F-NMR)

For the products obtained in Examples and Comparative Examples, molecular structural analysis was carried out using $^{1}$H-NMR (400 MHz) and $^{19}$F-NMR (376 MHz) under the following measurement conditions.

[Measurement conditions]

Measurement device: JNM-ECZ400S type nuclear magnetic resonance apparatus (manufactured by JEOL, Ltd.)
Observation nucleus: $^{1}$H, $^{19}$F
Solvent: Deuterated chloroform, Deuterated dimethyl sulfoxide
Reference material: Tetramethylsilane ($^{1}$H, 0.00 ppm), Trichlorofluoromethane ($^{19}$F, 0.00 ppm)
Concentration of reference material: 5% by mass
Concentration of measured sample: 20% by mass
Pulse width: 6.5 μsec
Delay time: 2 seconds
Number of scans: 8 times ($^{1}$H), 1,024 times ($^{19}$F)

In the following Examples, in spectra obtained by $^{19}$F-NMR measurement, the sum of the integrated values of the peaks of the fluorine-containing cyclic sulfonylimide salt is designated as T1, the sum of the integrated values of the peaks of impurities characterized by appearing as single or multiple doublet peaks within a range of -115 ppm to -125 ppm is designated as T2, and the ratio of T1 to T2 is designated as T1/T2, which are used as an indicator of the purity of the fluorine-containing cyclic sulfonylimide. The peaks of the fluorine-containing cyclic sulfonylimide salt and impurity in $^{19}$F-NMR peaks sometimes vary in chemical shift value by about ±5 ppm depending on the purity, amount of residual solvent, measurement conditions and the like. The fluorine-containing cyclic sulfonylimide salt according to the present invention is not limited by such attribution errors due to variations in chemical shift values.

[Example V-1]

According to the method described in [Example 2] and [Example 4] to [Example 6] of the example section of PTL 2 (WO 2006/106960 A), a fluorine-containing cyclic sulfonimide lithium salt (compound (1-2)) represented by the following structural formula:

[Chemical Formula 40]

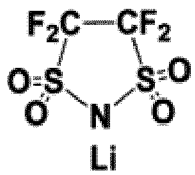

was synthesized using commercially available 2,2-difluoro-2-fluorosulfonylacetic acid (manufactured by FUJIFILM Wako Pure Chemical Corporation) as a starting material, followed by subjecting to an electrolytic coupling reaction step, a cyclization step with ammonia, and a cation exchange step with lithium hydroxide. In a 100 mL recovery flask equipped with a stirrer, 10 g of the synthesized compound (1-2) and 27 g of tert-butyl methyl ether were weighed, and a hermetic stopper was attached, followed by stirring for 30 minutes. The mixed solution thus obtained was filtered under reduced pressure and then cooled in a freezer at -20°C for 30 minutes to yield colorless crystals. After removing the liquid portion by decantation, tert-butyl methyl ether at -35°C was added and mixed with a spatula to wash the crystals, and the liquid portion was removed by decantation. The crystals thus obtained were dried under reduced pressure for 3 hours under

the conditions of 60°C and 40 hPa without stirring. The mass of the dried crystals was 4.9 g and the yield based on the amount used was 49%. In $^{19}$F-NMR, regarding the peaks (δ (ppm) -110.0 (4F)) of the compound (1-2) and the peaks of impurities seen as a single doublet at -117.2 ppm, T/T2 was 3,170. From $^{1}$H-NMR measurement using benzotrifluoride as an internal standard, the residual amount of tert-butyl methyl ether was 0.72% by mass, and from these results, the purity of the compound (1-2) was 99.2% by mass. It was possible to easily take out the entire amount of the obtained crystals after drying from the recovery flask by crushing them with a spatula.

[Comparative Example V-1]

The compound (1-2) was synthesized in the same manner as in Example V-1, but was not purified by crystallization thereafter. In $^{19}$F-NMR, regarding the sum of the integrated values of the peaks (δ (ppm) -115.4 (4F)) of the compound (1-2) and the peak of impurities appearing as multiple doublets within a range of -122 ppm to -125 ppm, T1/T2 was 215, indicating low purity, and the powder obtained after crushing turned brown.

[Comparative Example V-2]

The compound (1-2) was synthesized in the same manner as in Example V-1. In a 20 mL recovery flask, 1 g of the synthesized compound (1-2) and 2 g of acetone (boiling point of 56°C) were weighed, and a hermetic stopper was attached, followed by stirring for 30 minutes. The mixed solution was cooled overnight at -50°C, but no crystals were formed, thus failing to perform high purification. As a result of drying under reduced pressure under the conditions of 60°C and 10 hPa for 3 hours for recovery, only a portion of the wall surface of the contents solidified, and a highly viscous liquid remained at the bottom, and it was impossible to recover as solids with a spatula. The reason why the large amount of the solvent remained even when acetone having a low boiling point was used is considered that distillation of acetone was suppressed due to high affinity between acetone and lithium.

[Comparative Example V-3]

The compound (1-2) was synthesized in the same manner as in Example V-1. In a 50 mL recovery flask, 1 g of the synthesized compound (1-2) and 2 g of dimethyl carbonate were weighed, and a hermetic stopper was attached, followed by stirring for 30 minutes. After the mixture was filtered, 20 g of cyclohexane was added to the liquid thus obtained and, as a result, the content liquid was separated into two phases, and no crystals were formed. The phase-separated content liquid was cooled overnight at -20°C, but the compound (1-2) was not crystallized and could not be highly purified. This is probably because crystallization was suppressed due to high affinity between dimethyl carbonate and lithium.

[Example V-2]

The compound (1-2) was synthesized in the same manner as in Example V-1. In a 200 mL recovery flask, 10 g of the synthesized compound (1-2) and 20 g of dimethyl carbonate were weighed, and a hermetic stopper was attached, followed by stirring for 30 minutes. After the mixture was filtered, the liquid was cooled overnight at -50°C, but no crystals were formed. When the mixture was dried under reduced pressure at 100°C and 5 hPa for 10 hours for recovery, the mixture as a whole turned into a white turbid gel and then turned into white solid by continuously drying. After adding and dissolving 27 g of tert-butyl methyl ether, the mixture was cooled to -50°C overnight to yield colorless crystals. After removing the liquid portion by decantation, tert-butyl methyl ether at -35°C was added and mixed with a spatula to wash the crystals, and the liquid portion was removed by decantation. The crystals thus obtained were dried under reduced pressure for 3 hours under the conditions of 60°C and 40 hPa without stirring. Attempts were made to crush the obtained crystals with a spatula in order to recover them. However, the crystals obtained by crushing with a spatula amounted to only 2.7 g (yield of 27%) due to significant adhesion to the bottom of the recovery flask. The loss due to adhesion was 2.0 g. It is considered that the crystals partially dissolved during drying due to the residual dimethyl carbonate having a high affinity for lithium, leading to adhesion to the wall surface of the recovery flask. Since the non-uniformity of the sample due to adhesion of the crystals was expected, the collected crystals were further dried under the conditions at 100°C and 20 hPa for 3 hours and then pulverized with a pestle to finally obtain 2.4 g of crystals. T1/T2 by $^{19}$F-NMR was 1,550. From 1H-NMR measurements using benzotrifluoride as an internal standard, the amounts of residual tert-butyl methyl ether and residual dimethyl carbonate were 0.29% by weight and 0.13% by mass, respectively, and from these results, the purity of compound (1-2) was 99.5% by mass.

[Comparative Example V-4]

The compound (1-2) was synthesized in the same manner as in Example V-1. In a 20 mL recovery flask, 1.0 g of the synthesized compound (1-2) and 1.3 g of tetrahydrofuran (boiling point of 65°C) were weighed, and a hermetic stopper was attached, followed by stirring for 30 minutes. The mixed solution was cooled overnight at -50°C, but no crystals were formed, thus failing to perform high purification. As a result of drying under reduced pressure for 3 hours under the conditions of 60°C and 10 hPa for recovery, only a portion of the wall surface of the contents solidified, and a highly viscous liquid remained at the bottom, and it was impossible to recover as solids with a spatula. The reason why the large amount of the solvent remained even when tetrahydrofuran having a low boiling point was used is considered that distillation of tetrahydrofuran was suppressed due to strong coordinating power to lithium associated with the cyclic structure of tetrahydrofuran.

[Example V-5]

The compound (1-2) was synthesized, crystallized and dried in the same manner as in Example V-1 to obtain 4.9 g of crystals. Next, the crystals are washed by adding 30 mL of 1,1,1,2,2,3,4,5,5,5-decafluoropentane and mixing with a spatula, and then dried under the conditions of 100°C and 15 hPa for 7 hours. This washing and drying operation was repeated five times in total to finally obtain 4.6 g of crystals. T1/T2 by $^{19}$F-NMR was 3,355. From $^{1}$H-NMR measurement using benzotrifluoride as an internal standard, the residual amount of tert-butyl methyl ether was 0.072% by mass, and from these results, the purity of the compound (1-2) was 99.9% by mass. It was possible to easily take out the entire amount of the obtained crystals after drying from the recovery flask by crushing them with a spatula.

INDUSTRIAL APPLICABILITY

Since the non-aqueous electrolyte solution within the scope of structural elements of the present invention can be used in batteries that require an improvement in high-temperature durability and a reduction in corrosiveness to metal, in particular, non-aqueous secondary batteries using the non-aqueous electrolyte solution of the present invention are expected to be used, for example, as rechargeable batteries for mobile devices such as mobile phones, portable audio devices, personal computers and integrated circuit (IC) tags; rechargeable batteries for vehicles such as hybrid vehicles, plug-in hybrid vehicles and electric vehicles; low-voltage power sources such as12 V-class power sources, 24 V-class power sources and 48 V-class power sources; residential power storage systems, IoT devices and the like. The non-aqueous secondary batteries using the non-aqueous electrolyte solution of the present invention can also be applied to applications in cold regions, outdoor applications in summer and the like.

The fluorine-containing cyclic sulfonylimide salt (1A) according to the present invention has high heat-resistant properties and is usable in a wide temperature range, so that the fluorine-containing cyclic sulfonylimide salt can be suitably used as antistatic agents, ion conductive materials such as organic electrolytes, and flame retardants.

According to the production method according to the present invention, it is possible to produce a bis(fluorosulfonyl) compound, which is a starting material for surfactants, antistatic agents, electrolytes, ionic liquids, catalysts and the like, while reducing the content of fluorosulfonyl group-containing compounds as by-products.

According to the production method according to the present invention, it is possible to stably produce bis(fluorosulfonyl) compounds, which are starting materials for surfactants, antistatic agents, electrolytes, ionic liquids, catalysts and the like, in a high yield.

According to the production method of the present invention, it is possible to produce a fluorine-containing cyclic sulfonylimide salt with a small amount of residual solvent and a small amount of fluorine-based impurities with high efficiency. Reduction of the amount of residual solvent enables production with simpler equipment, low cost and heat history. It is also possible to suppress coloring by reducing the amount of impurities. Therefore, the fluorine-containing cyclic sulfonylimide salt obtained by the production method according to the present invention can be suitably used as ion conductive materials, antistatic agents, flame retardants and the like.

REFERENCE SIGNS LIST

1: Reaction vessel
2: Pair of electrodes
3: Aqueous phase (upper phase)
4: Organic phase (lower phase)
TL: Height (distance) from the bottom of the reaction vessel to the liquid surface of the reaction solution

TE: Height (distance) from the bottom of the reaction vessel to the top of the electrode (highest level)
TE': Height (distance) from the bottom of the reaction vessel to the bottom of the electrode (lowest level)
TS: Height (distance) from the bottom of the reaction vessel to the interface between the organic phase and the aqueous phase

100: Non-aqueous secondary battery
110: Battery exterior
120: Battery exterior space
130: Positive electrode lead body
140: Negative electrode lead body
150: Positive electrode
160: Negative electrode
170: Separator

**Claims**

**1.** A non-aqueous electrolyte solution comprising a non-aqueous solvent and a lithium salt, wherein

the non-aqueous solvent contains a carbonate solvent,
the lithium salt contains a fluorine-containing cyclic sulfonylimide salt represented by the following general formula (1):

[Chemical Formula 1]

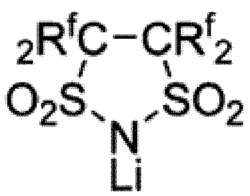

(1)

wherein $R^f$ s may be the same or different and $R^f$ each represents a fluorine atom or a perfluoro group having 4 or less carbon atoms, and contains a fluorine-containing sulfonamide compound represented by the following general formula (2):

$H(CR_2)_m-SO_2NHM^2$ (2)

wherein Rs may be the same or different and R each is a fluorine atom or a trifluoromethyl group, $M^2$ is Li, Na, K, H or $NH_4$, and m is 1 or 2, in an amount of 1,000 ppm by mass or less relative to the total amount of the non-aqueous electrolyte solution.

**2.** The non-aqueous electrolyte solution according to claim 1, wherein the fluorine-containing cyclic sulfonylimide salt represented by the general formula (1) contains any one of the following formulas (1-1) to (1-5).

[Chemical Formula 2]

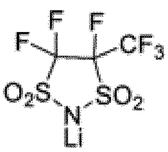

(1-1)

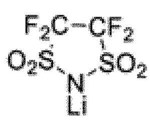

(1-2)

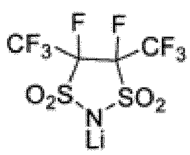

(1-3)

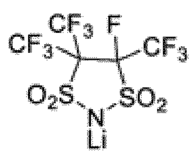

(1-4)

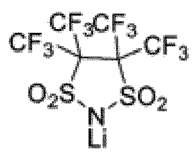

(1-5)

**3.** The non-aqueous electrolyte solution according to claim 1 or 2, wherein the fluorine-containing sulfonamide compound represented by the general formula (2) is contained in an amount of 0.1 ppm by weight or more relative to the total amount of the non-aqueous electrolyte solution.

**4.** The non-aqueous electrolyte solution according to any one of claims 1 to 3, wherein the non-aqueous solvent contains acetonitrile in an amount of 3% by volume or more and 97% by volume or less relative to the total amount of the non-aqueous solvent.

**5.** The non-aqueous electrolyte solution according to claim 4, wherein the content of the acetonitrile is 20% by volume or more and 95% by volume or less relative to the total amount of the non-aqueous solvent.

**6.** The non-aqueous electrolyte solution according to any one of claims 1 to 5, wherein the fluorine-containing sulfonamide compound represented by the general formula (2) is contained in an amount of 1 ppm by mass or more and 200 ppm by mass or less relative to the total amount of the non-aqueous electrolyte solution.

**7.** The non-aqueous electrolyte solution according to any one of claims 1 to 6, wherein the content of the fluorine-containing cyclic sulfonylimide salt represented by the general formula (1) is 0.8 mol or more and 1.5 mol or less relative to 1 L of the non-aqueous solvent.

**8.** The non-aqueous electrolyte solution according to any one of claims 1 to 7, wherein the fluorine-containing sulfonamide compound represented by the general formula (2) is contained in an amount of 80 ppm by mass or more and 120 ppm by mass or less relative to the total amount of the non-aqueous electrolyte solution.

**9.** The non-aqueous electrolyte solution according to any one of claims 1 to 8, wherein the lithium salt contains the fluorine-containing cyclic sulfonylimide salt represented by the general formula (1) and $LiPF_6$ ,

the non-aqueous solvent contains vinylene carbonate and/or fluoroethylene carbonate,
the content of the fluorine-containing cyclic sulfonylimide salt represented by the general formula (1) is 2.5 or more, in terms of a molar ratio, relative to the content of $LiPF_6$, and
the content of $LiPF_6$ is 0.01 or more and 4 or less relative to the contents of vinylene carbonate and fluoroethylene carbonate, in terms of a molar ratio.

**10.** The non-aqueous electrolyte solution according to any one of claims 1 to 9, wherein the content of the fluorine-containing cyclic sulfonylimide salt represented by the general formula (1) is more than 10 relative to the content of $LiPF_6$, in terms of a molar ratio.

**11.** A non-aqueous secondary battery comprising the non-aqueous electrolyte solution according to any one of claims 1 to 10.

**12.** The non-aqueous secondary battery according to claim 11, wherein the non-aqueous secondary battery comprises a battery exterior, and the battery exterior contains aluminum in at least a part of a liquid contact layer with the non-aqueous electrolyte solution on the positive electrode side.

**13.** A method for producing the non-aqueous electrolyte solution according to any one of claims 1 to 10, wherein the fluorine-containing cyclic sulfonylimide salt represented by the general formula (1) is produced by subjecting a fluorosulfonyl group-containing carboxylic acid compound represented by the following general formula (3):

$$MO_2C\text{-}(CR_2)_m\text{-}SO_2F \qquad (3)$$

wherein Rs may be the same or different and R each is a fluorine atom or a trifluoromethyl group, m is 1 or 2, and M is H, Li, Na, K or $NH_4$, as a starting material, to an electrolytic coupling reaction step, a cyclization step and a cation exchange step, the method comprising:

a mixing step of mixing the fluorine-containing cyclic sulfonylimide salt represented by the general formula (1) with a non-aqueous solvent, and
a purification step of reducing a content of a fluorosulfonyl group-containing compound represented by the following general formula (5):

$H\text{-}(CR_2)_m\text{-}SO_2F$ (5)

wherein Rs may be the same or different and R each is a fluorine atom or a trifluoromethyl group, and m is 1 or 2, in the reaction solution, to 0.1% by mass or less after the electrolytic coupling reaction step.

**14.** A method for producing the non-aqueous electrolyte solution according to any one of claims 1 to 10, wherein the fluorine-containing cyclic sulfonylimide salt represented by the general formula (1) is produced by subjecting a fluorosulfonyl group-containing carboxylic acid compound represented by the following general formula (3):

$MO_2C\text{-}(CR_2)_m\text{-}SO_2F$ (3)

wherein Rs may be the same or different and R each is a fluorine atom or a trifluoromethyl group, m is 1 or 2, and M is H, Li, Na, K or $NH_4$, as a starting material, to an electrolytic coupling reaction step, a cyclization step and a cation exchange step, the method comprising:

a crystallization step of dissolving the fluorine-containing cyclic sulfonylimide salt represented by the general formula (1) obtained after the cation exchange step in a monodentate chain ether solvent, followed by crystallization to obtain a purified fluorine-containing cyclic sulfonylimide salt, and
a mixing step of mixing the fluorine-containing cyclic sulfonylimide salt represented by the general formula (1) obtained in the crystallization step with a non-aqueous solvent.

**15.** A fluorine-containing cyclic sulfonylimide salt composition comprising a fluorine-containing cyclic sulfonylimide salt represented by the following general formula (1A):

[Chemical Formula 3]

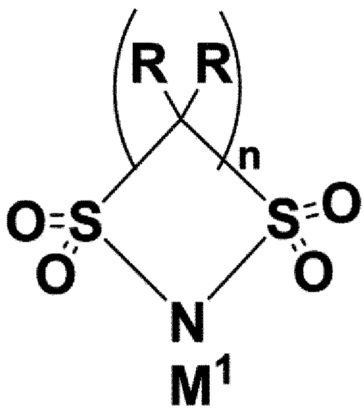

(1A)

wherein Rs may be the same or different and R each is a fluorine atom or a trifluoromethyl group, n is an integer of 1 to 4, and $M^1$ is Li, Na, K or $NH_4$, wherein
the temperature at which a mass reduction rate reaches 2% by mass when heated at a temperature rise rate of 10°C/min from 100°C under nitrogen stream is 385°C or higher.

**16.** The fluorine-containing cyclic sulfonylimide salt composition according to claim 15, wherein a fluorine-containing sulfonamide compound represented by the following general formula (2):

$H(CR_2)_m\text{-}SO_2NHM^2$ (2)

wherein Rs may be the same or different and R each is a fluorine atom or a trifluoromethyl group, $M^2$ is Li, Na, K, H or $NH_4$, and m is 1 or 2, is contained in an amount of 5,000 ppm by mass or less.

**17.** The fluorine-containing cyclic sulfonylimide salt composition according to claim 16, wherein n is 2 in the general formula (1A).

**18.** The fluorine-containing cyclic sulfonylimide salt composition according to claim 16 or 17, wherein R is a fluorine

atom in the general formulas (1A) and (2).

**19.** The fluorine-containing cyclic sulfonylimide salt composition according to any one of claims 16 to 18, wherein $M^1$ is Li in the general formula (1A), and $M^2$ is Li in the general formula (2).

**20.** The fluorine-containing cyclic sulfonylimide salt composition according to any one of claims 15 to 19, wherein the fluorine-containing cyclic sulfonylimide salt is represented by the following formula.

[Chemical Formula 4]

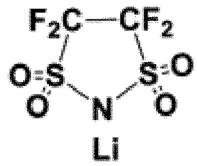

**21.** A method for producing a fluorine-containing cyclic sulfonylimide salt represented by the following general formula (1A):

[Chemical Formula 5]

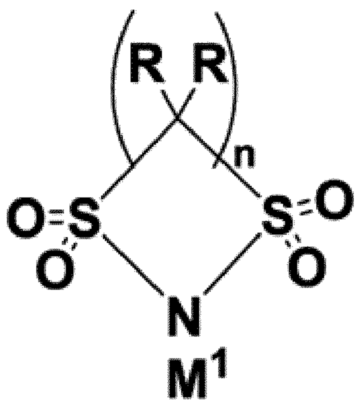

(1A)

wherein Rs may be the same or different and R each is a fluorine atom or a trifluoromethyl group, n is an integer of 1 to 4, and $M^1$ is Li, Na or K, wherein the fluorine-containing cyclic sulfonylimide salt is produced by subjecting a fluorosulfonyl group-containing carboxylic acid compound represented by the following general formula (3):

$MO_2C-(CR_2)_m-SO_2F$ (3)

wherein Rs may be the same or different and R each is a fluorine atom or a trifluoromethyl group, m is 1 or 2, and M is H, Li, Na, K or $NH_4$, as a starting material, to an electrolytic coupling reaction step, a cyclization step and a cation exchange step, the method comprising a purification step of reducing a content of a fluorosulfonyl group-containing compound represented by the following general formula (5):

$H-(CR_2)m -SO_2F$ (5)

wherein Rs may be the same or different and R each is a fluorine atom or a trifluoromethyl group, and m is 1 or 2, in the reaction solution, to 0.1% by mass or less after the electrolytic coupling reaction step.

**22.** A method for producing a fluorine-containing cyclic sulfonylimide salt represented by the following general formula (1A):

[Chemical Formula 6]

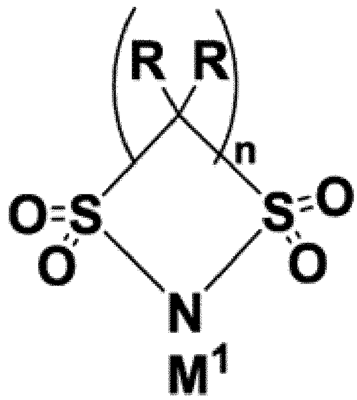

(1A)

wherein Rs may be the same or different and R each is a fluorine atom or a trifluoromethyl group, n is an integer of 1 to 4, and $M^1$ is Li, Na or K, wherein the fluorine-containing cyclic sulfonylimide salt is produced by subjecting a fluorosulfonyl group-containing carboxylic acid compound represented by the following general formula (3):

$$MO_2C\text{-}(CR_2)_m\text{-}SO_2F \qquad (3)$$

wherein Rs may be the same or different and R each is a fluorine atom or a trifluoromethyl group, m is 1 or 2, and M is H, Li, Na, K or $NH_4$, as a starting material, to an electrolytic coupling reaction step, a cyclization step and a cation exchange step, the method comprising a crystallization step of dissolving the fluorine-containing cyclic sulfonylimide salt obtained after the cation exchange step in a monodentate chain ether solvent, followed by crystallization to obtain a purified fluorine-containing cyclic sulfonylimide salt.

**23.** The method according to claim 22, wherein the monodentate chain ether solvent used for crystallization is at least one solvent selected from the group consisting of ethyl ether, propyl methyl ether, propyl ethyl ether, propyl ether, isopropyl methyl ether, isopropyl ethyl ether, isopropyl ether, butyl methyl ether, butyl ethyl ether, isobutyl methyl ether, isobutyl ethyl ether, sec-butyl methyl ether, sec-butyl ethyl ether, tert-butyl methyl ether, tert-butyl ethyl ether, pentyl methyl ether, isopentyl methyl ether, sec-pentyl methyl ether, tert-pentyl methyl ether, neopentyl methyl ether and cyclopentyl methyl ether.

**24.** The method according to claim 22 or 23, wherein n is 2 in the general formula (1A).

**25.** The method according to any one of claims 22 to 24, wherein R is F in the general formula (1A).

**26.** The method according to any one of claims 22 to 25, wherein $M^1$ is Li in the general formula (1A).

**27.** The method according to any one of claims 21 to 26, wherein, in the electrolytic coupling reaction step, the fluorosulfonyl group-containing carboxylic acid compound represented by the general formula (3) is subjected to an electrolytic coupling reaction in the presence of a mixed solvent of water and a nitrile group-containing solvent in which a ratio ($\beta/\alpha$) of the mass ($\alpha$) of water to the mass ($\beta$) of the nitrile group-containing solvent is 0.80 or less.

**28.** The method according to claim 27, wherein the ratio ($\beta/\alpha$) of the mass ($\alpha$) of water to the mass ($\beta$) of the nitrile group-containing solvent is 0.75 or less.

**29.** The method according to any one of claims 21 to 28, wherein, in the electrolytic coupling reaction step, the fluorosulfonyl group-containing carboxylic acid compound represented by the general formula (3) is subjected to an electrolytic coupling reaction in a reaction vessel in the presence of a mixed solvent of water and a nitrile group-containing solvent, and
wherein, during the reaction, the height (distance) TL from the bottom of the reaction vessel to the liquid surface of the reaction solution and the height (distance) TE from the bottom of the reaction vessel to the top of the electrode (highest level) satisfy the relationship TL > TE, and after the reaction, the height (distance) TS from the bottom of the reaction vessel to the interface between the organic phase and the aqueous phase and the height (distance) TE' from the bottom of the reaction vessel to the bottom of the electrode (lowest level) satisfy the relationship TE' > TS.

**30.** The method according to claim 29, wherein the TE' and TS satisfy the relationship: TE' $\times$ 0.6 > TS.

**31.** The method according to any one of claims 27 to 30, wherein a content of an alkali metal in the reaction solution during the electrolytic coupling reaction is 2,000 ppm by mass or less.

**32.** The method according to any one of claims 27 to 31, wherein, in the electrolytic coupling reaction step, a molar ratio of the compound represented by the general formula (5) produced is set at 0.01 or less relative to 1 mol of the formation amount of a bis(fluorosulfonyl) compound (4) represented by the following general formula (4):

$$FO_2S\text{-}(CR_2)_m\text{-}SO_2F \qquad (4)$$

wherein Rs may be the same or different and R each is a fluorine atom or a trifluoromethyl group, and m is 1 or 2.

**33.** The method according to any one of claims 27 to 32, wherein, in the electrolytic coupling reaction step, the fluorosulfonyl group-containing carboxylic acid compound represented by the general formula (3) is subjected to an electrolytic coupling reaction in the presence of a mixed solvent of water and a nitrile group-containing solvent, and a ratio ($\delta/\gamma$) of the mass ($\delta$) of the mixed solvent to the mass ($\gamma$) of the fluorosulfonyl group-containing carboxylic acid compound is 1.5 or less.

**34.** The method according to any one of claims 27 to 33, wherein, in the electrolytic coupling reaction, a plate spacing between the anode and cathode is 0.001 mm to 9.9 mm.

**35.** The method according to claim 33 or 34, wherein, in the electrolytic coupling reaction, the yield of the compound (4) is set at 70% or more.

**36.** The method according to any one of claims 27 to 35, wherein the nitrile group-containing solvent is at least one selected from the group consisting of acetonitrile, propionitrile, butyronitrile and benzonitrile.

**37.** The method according to claim 36, wherein the nitrile group-containing solvent is acetonitrile.

**38.** The method according to any one of claims 27 to 37, wherein the electrolytic coupling reaction is carried out at a reaction temperature within a range of -35°C or higher and 10°C or lower.

FIG. 1

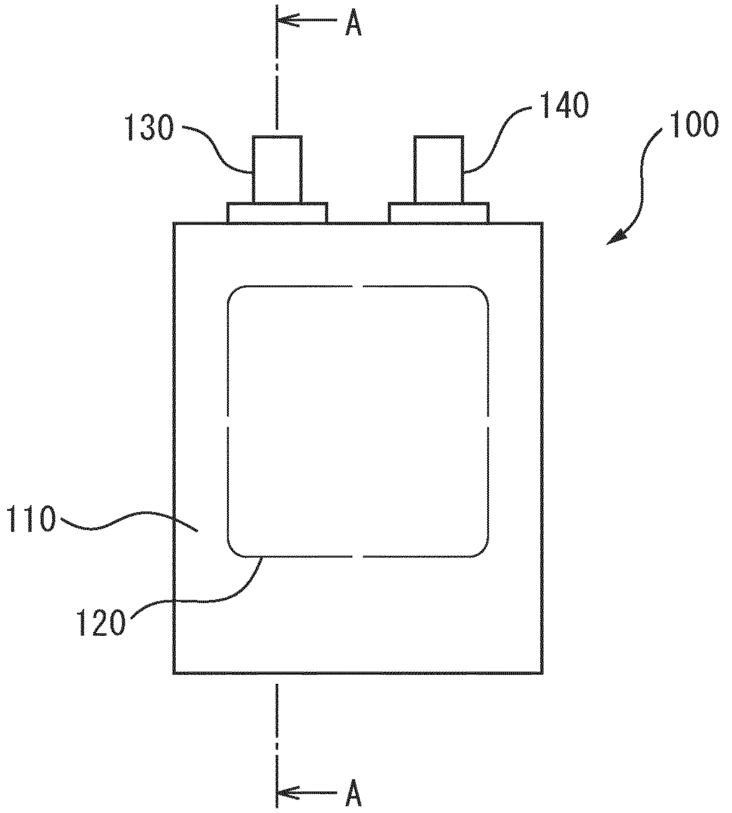


FIG. 2

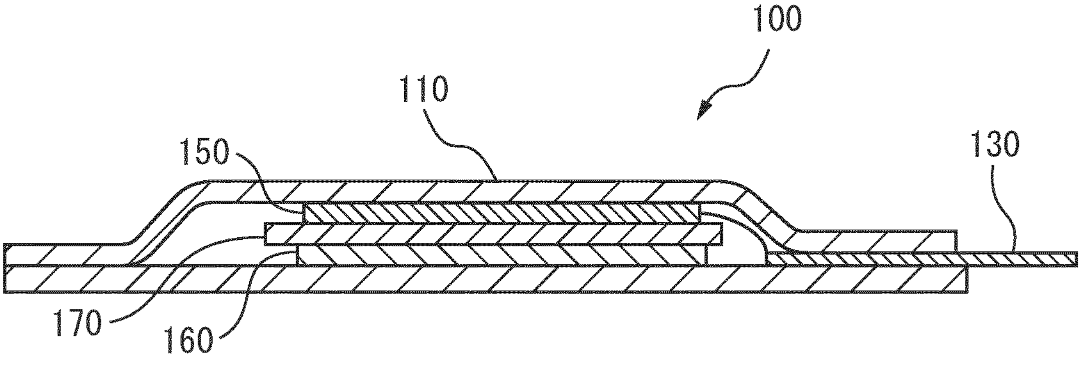

FIG. 3

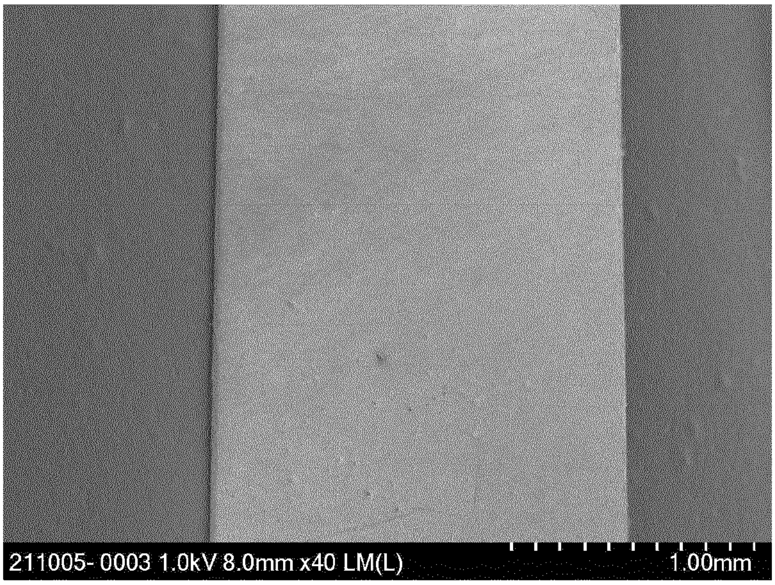


(Example II-1, 40 times magnification)

FIG. 4

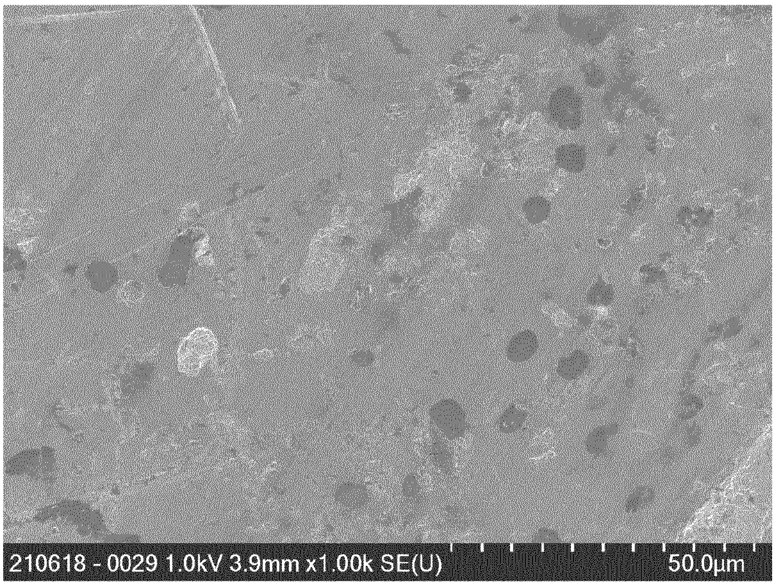


(Example II-1, 1,000 times magnification)

FIG. 5

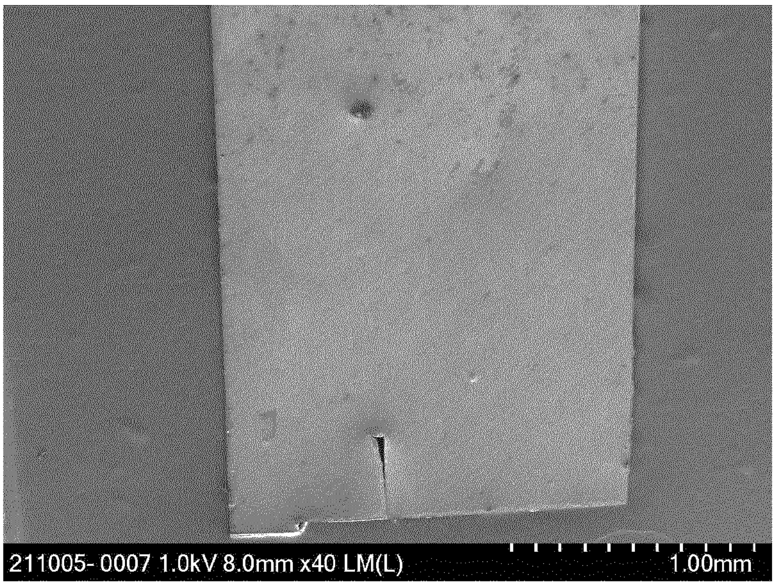


(Comparative Example II-1, 40 times magnification)

FIG. 6

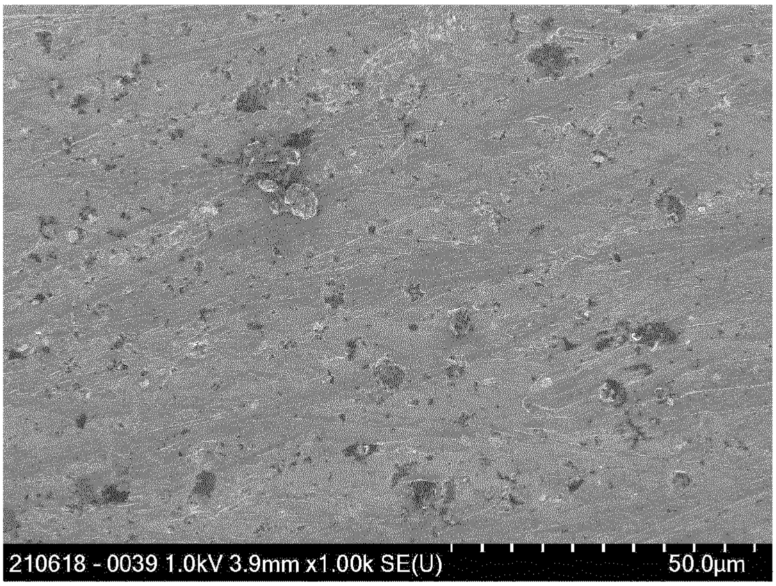


(Comparative Example II-1, 1,000 times magnification)

FIG. 7

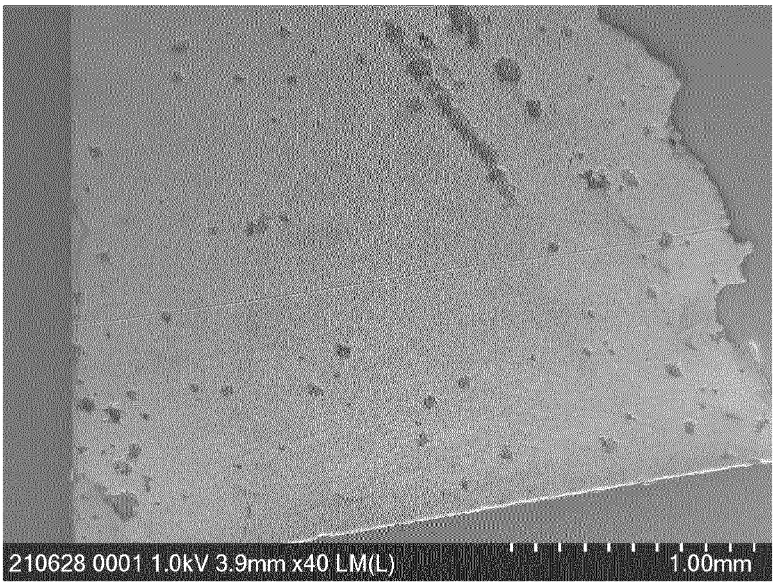


(Comparative Example II-3, 40 times magnification)

FIG. 8

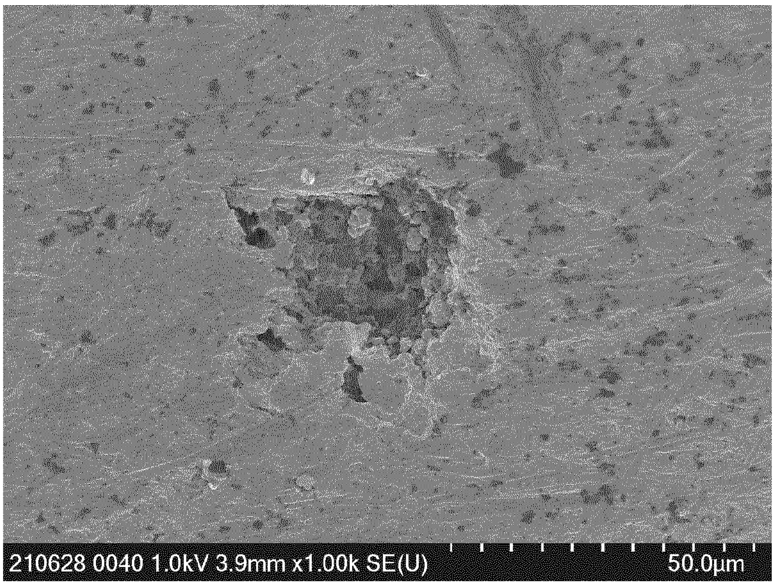


(Comparative Example II-3, 1,000 times magnification)

FIG. 9

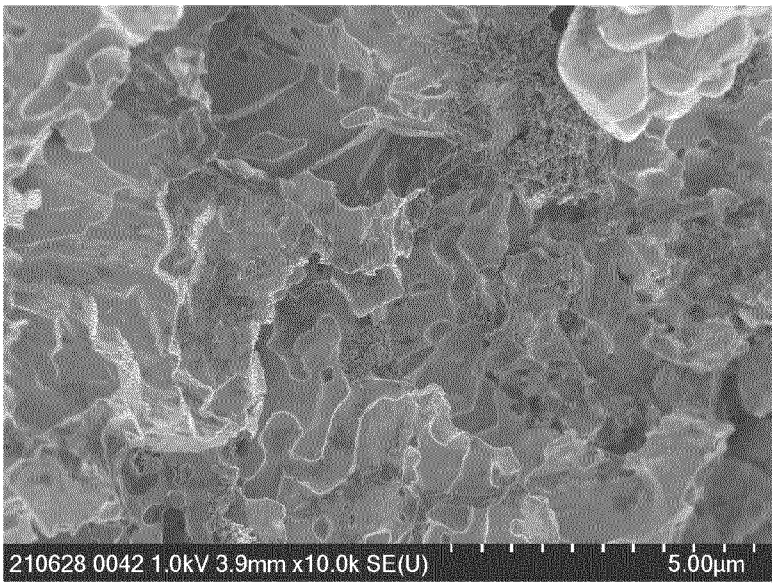


(Comparative Example II-3, 10,000 times magnification)

FIG. 10

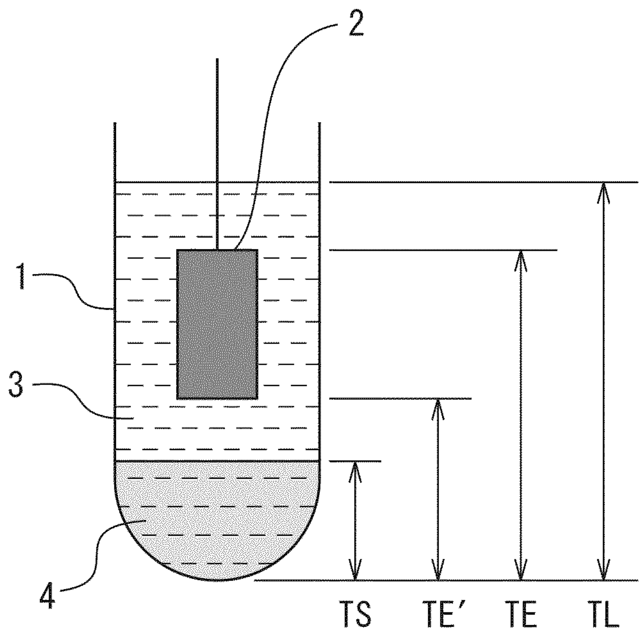

## INTERNATIONAL SEARCH REPORT

International application No.
**PCT/JP2022/014660**

**A. CLASSIFICATION OF SUBJECT MATTER**

***C07C 303/22***(2006.01)i; ***C07C 309/80***(2006.01)i; ***C07C 309/82***(2006.01)i; ***C07C 313/02***(2006.01)i; ***C07D 285/00***(2006.01)i; ***C07D 285/01***(2006.01)i; ***H01M 10/052***(2010.01)i; ***H01M 10/0567***(2010.01)i; ***H01M 10/0568***(2010.01)i; ***H01M 10/0569***(2010.01)i
FI: H01M10/0569; C07C303/22; C07C309/80; C07C309/82; C07C313/02; C07D285/00; C07D285/01 CSP; H01M10/052; H01M10/0567; H01M10/0568

According to International Patent Classification (IPC) or to both national classification and IPC

**B. FIELDS SEARCHED**

Minimum documentation searched (classification system followed by classification symbols)

C07C303/22; C07C309/80; C07C309/82; C07C313/02; C07D285/00; C07D285/01; H01M10/052; H01M10/0567; H01M10/0568; H01M10/0569

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2010/110388 A1 (ASAHI GLASS COMPANY, LIMITED) 30 September 2010 (2010-09-30) claims, paragraphs [0028]-[0031] | 1-5, 7, 9-12, 15-20 |
| Y | WO 2009/025246 A1 (ASAHI GLASS COMPANY, LIMITED) 26 February 2009 (2009-02-26) claims, paragraphs [0020]-[0022], [0035], [0042] | 1-5, 7, 9-12, 15-20 |
| Y | WO 2020/262670 A1 (ASAHI KASEI KABUSHIKI KAISHA) 30 December 2020 (2020-12-30) paragraph [0005] | 4-5 |
| A | WO 2006/106960 A1 (ASAHI GLASS COMPANY, LIMITED) 12 October 2006 (2006-10-12) claims | 1-38 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 May 2022** | **07 June 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/014660**

| Patent document cited in search report | Publication date (day/month/year) | Patent family member(s) | Publication date (day/month/year) |
|---|---|---|---|
| WO 2010/110388 A1 | 30 September 2010 | US 2011/0300439 A1<br>claims, paragraphs [0022]-[0026]<br>EP 2413418 A1<br>CN 102365781 A<br>KR 10-2012-0002521 A | |
| WO 2009/025246 A1 | 26 February 2009 | US 2010/0137609 A1<br>claims, paragraphs [0041]-[0042], [0067], [0075]<br>EP 2179995 A1<br>CN 101778835 A<br>KR 10-2010-0042264 A | |
| WO 2020/262670 A1 | 30 December 2020 | US 2021/0344045 A1<br>paragraph [0005]<br>EP 3831780 A1<br>KR 10-2021-0011441 A<br>CN 112640180 A | |
| WO 2006/106960 A1 | 12 October 2006 | US 2008/0091009 A1<br>claims<br>EP 1864970 A1<br>CN 101155777 A<br>KR 10-2007-0116241 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010110388 A
- JP 2008021517 A
- WO 2006106960 A
- WO 2009025246 A


### Non-patent literature cited in the description


- Research report. Asahi Glass Co., Ltd, 2010, vol. 60, 13-21